(19) Europäisches Patentamt
European Patent Office
Office européen des brevets

(11) Veröffentlichungsnummer: **0 374 849**
**A2**

(12) # EUROPÄISCHE PATENTANMELDUNG

(21) Anmeldenummer: 89123481.7

(22) Anmeldetag: 19.12.89

(51) Int. Cl.5: C07D 215/04, C09K 19/34, C07D 215/14, C07D 215/227, C07D 215/48, C07D 239/26, C07D 409/04, C07C 255/54, C07C 255/50, C07D 319/06, C07D 213/30

(30) Priorität: 19.12.88 SU 4617047
19.12.88 SU 4617049
19.12.88 SU 4617050
19.12.88 SU 4617051
19.12.88 SU 4617052
19.12.88 SU 4617054

(43) Veröffentlichungstag der Anmeldung:
27.06.90 Patentblatt 90/26

(84) Benannte Vertragsstaaten:
CH DE FR GB IT LI NL

(71) Anmelder: PAVLJUCHENKO, Assya Iosifovna
pr. Marshala Zhukova, 62-38
Moscow, 123448(SU)

Anmelder: PETROV, Vladimir Fedorovich
Khoroshevskoe shosse, 66, kv. 87
Moskva(SU)

Anmelder: GREBENKIN, Mikhail Fedorovich
ul. Linia Oktyabrskoi zheleznoi dorogi, 10-79
Moscow, 127238(SU)

Anmelder: IVASCHENKO, Alexandr Vasilievich
ul. Pervomaiskaya, 44a-57 Moskovskaya obl.
Dolgoprudny, 141700(SU)

Anmelder: KOROTKOVA, Natalya Ivanovna
Likhachevskoe shosse, 20-3-16 Moskovskaya obl.
Dolgoprudny, 141700(SU)

Anmelder: SMIRNOVA, Natalya Ivanovna
Likhachevskoe shosse, 13-1-89 Moskovskaya obl.
Dolgoprudny, 142700(SU)

Anmelder: Krjuchkova, Ljudmila Vladimirovna
ulitsa Linia Oktyabrskoi Zheleznoi dorogi, 10, kv. 18
Moskva(SU)

Anmelder: Karamysheva, Ljudmila Alexeevna
ulitsa Poltavskaya, 2, kv. 126
Moskva(SU)

Anmelder: Agafonova, Irina Fedorovna
Dmitrovskoe shosse, 105, korpus 2, kv. 39
Moskva(SU)

Anmelder: Dakhnov, Petr Pavlovich
Moskovskaya oblast proezd Patsaeva, 18, kv. 169
Dolgoprudny(SU)

Anmelder: Geivandov, Ruben Khristoforovich
Moskovskaya oblast Moskovskoe shosse, 55, korpus 1 kv. 107
Dolgoprudny(SU)

Anmelder: Pozhidaev, Evgeny Pavlovich
ulitsa Yablochkova, 21, korpus 2, kv. 43
Moskva(SU)

Anmelder: Bolotin, Boris Markovich
ulitsa Chernenko, 22, korpus 2, kv. 270
Moskva(SU)

(72) Erfinder: PAVLJUCHENKO, Assya Iosifovna
pr. Marshala Zhukova, 62-38
Moscow, 123448(SU)
Erfinder: PETROV, Vladimir Fedorovich
Khoroshevskoe shosse, 66, kv. 87
Moskva(SU)
Erfinder: GREBENKIN, Mikhail Fedorovich
ul. Linia Oktyabrskoi zheleznoi dorogi, 10-79
Moscow, 127238(SU)
Erfinder: IVASCHENKO, Alexandr Vasilievich
ul. Pervomaiskaya, 44a-57 Moskovskaya obl.
Dolgoprudny, 141700(SU)

EP 0 374 849 A2

Erfinder: **KOROTKOVA, Natalya Ivanovna**
**Likhachevskoe shosse, 20-3-16 Moskovskaya obl.**
**Dolgoprudny, 141700(SU)**
Erfinder: **SMIRNOVA, Natalya Ivanovna**
**Likhachevskoe shosse, 13-1-89 Moskovskaya obl.**
**Dolgoprudny, 142700(SU)**
Erfinder: **Krjuchkova, Ljudmila Vladimirovna**
**ulitsa Linia Oktyabrskoi Zheleznoi dorogi, 10, kv. 18**
**Moskva(SU)**
Erfinder: **Karamysheva, Ljudmila Alexeevna**
**ulitsa Poltavskaya, 2, kv. 126**
**Moskva(SU)**
Erfinder: **Agafonova, Irina Fedorovna**
**Dmitrovskoe shosse, 105, korpus 2, kv. 39**
**Moskva(SU)**
Erfinder: **Dakhnov, Petr Pavlovich**
**Moskovskaya oblast proezd Patsaeva, 18, kv. 169**
**Dolgoprudny(SU)**
Erfinder: **Geivandov, Ruben Khristoforovich**
**Moskovskaya oblast Moskovskoe shosse, 55, korpus 1 kv. 107**
**Dolgoprudny(SU)**
Erfinder: **Pozhidaev, Evgeny Pavlovich**
**ulitsa Yablochkova, 21, korpus 2, kv. 43**
**Moskva(SU)**
Erfinder: **Bolotin, Boris Markovich**
**ulitsa Chernenko, 22, korpus 2, kv. 270**
**Moskva(SU)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **Flüssigkristalline Derivate von stickstoffhaltigen heterocyclischen Systemen als Komponenten eines flüssigkristallinen Materials und ein flüssigkristallines Material.**

(57) Vorgeschlagen werden neue flüssigkristalline Derivate von stickstoffhaltigen heterocyclischen Systemen der allgemeinen Formel $R^1$-$A^1$-Z-$A^2$-$R^2$ (1) worin $R^1$ und $R^2$ gleichzeitig oder unabhängig $C_nH_{2n+1}$, $-C_mH_{2m+1}$ , $C_nH_{2n+1}O$-, $C_mH_{2m+1}O$- , $CH_2=CH(CH_2)_k$-,-CN, -N=C=S,-Hal,

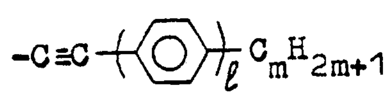

bei k = 0-3, 1 = 0-2: m, n = 1-15 sind:
Z = $-CH_2-CH_2-$, -CH=CH-, -C≡C, -COO-, Einfachbindung ist;
wobei falls

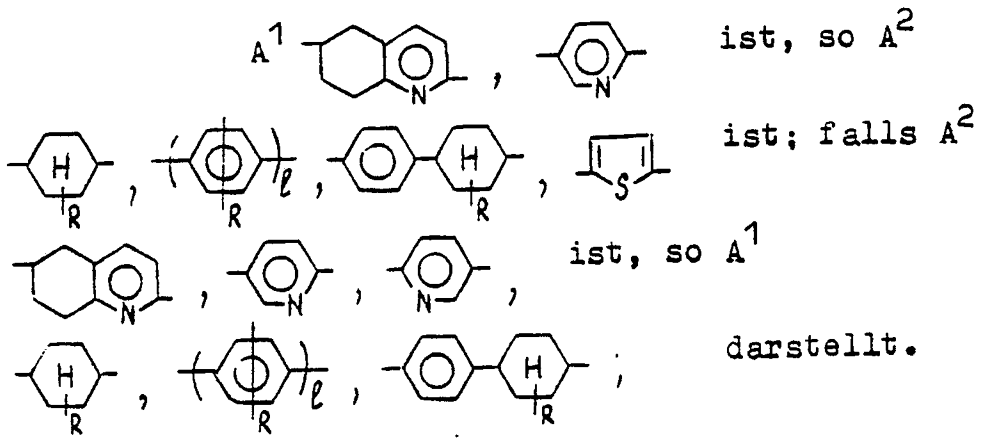
$A^1$
ist, so $A^2$
ist; falls $A^2$
ist, so $A^1$
darstellt.

R - $CH_3$,-CN, -Cl, -F ist, 1 0 bis 2 beträgt, als Komponenten eines flüssigkristallinen Materials. Das erfindungsgemäße flüssigkristalline Material enthält wenigstens zwei Komponenten, von denen wenigstens die eine ein flüssigkristallines Derivat des stickstoffhaltigen heterozyklischen Systems der allgemeinen Formel 1 in einer Menge von 1 bis 50 Masse-% darstellt.

# FLÜSSIGKRISTALLINE DERIVATE VON STICKSTOFFHALTIGEN HETEROCYCLISCHEN SYSTEMEN ALS KOMPONENTEN EINES FLÜSSIGKRISTALLINEN MATERIALS UND EIN FLÜSSIGKRISTALLINES MATERIAL

## Gebiet der Technik

Die vorliegende Erfindung bezieht sich auf neue organische Verbindungen, welche flüssigkristalline Eigenschaften aufweisen, und insbesondere betrifft sie flüssigkristalline Derivate von stickstoffhaltigen heterocyclischen Systemen als Komponenten eines flüssigkristallinen Materials und ein flüssigkristallines Material, welches für elektrooptische Einrichtungen verwendet werden kann.

## Zugrundeliegender Stand der Technik

Zur Zeit sind 5-Alkyl-2-(5-cyanophenyl)-pyridine der Formel

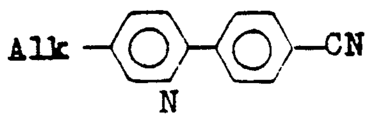

worin Alk 2 bis 7 Kohlenstoffatome enthält, als Komponenten von flüssigkristallinen Materialien (SU, A, 675800) bekannt.

Bekannt sind weiterhin 5-Alkyl-2-(4-alkyl- oder 4-alkoxyphenyl)-pyridine der Formel

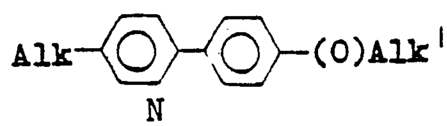

worin Alk, Alk′ 1 bis 12 Kohlenstoffatome enthalten, als Komponenten von flüssigkristallinen Materialien (GB, A, 261808).

Bekannt sind 5-Alkyl′-2-[4-trans-4-alkylcyclohexyl)-phenyl]-pyridine der Formel

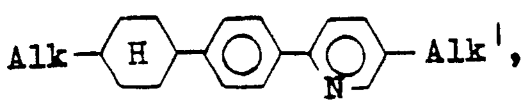

worin Alk, Alk′ 2 bis 6 Kohlenstoffatome haben (SU, A, 1302684), als Komponenten von flüssigkristallinen Materialien.

Die oben angegebenen Verbindungen zeichnen sich durch hohe Anisotropiewerte der Dielektrizitätskonstante bei geringen Klärpunkten und engen Bereichen der nematischen Phase aus. Diese Eigenschaften behindern die Verwendung von diese Verbindungen enthaltenden flüssigkristallinen Materialien in einem weiten Arbeitstemperaturbereich.

## Offenbarung der Erfindung

Der Erfindung liegt die Aufgabe zugrunde, neue flüssigkristalline Derivate von stickstoffhaltigen heterozyklischen Systemen als Komponenten eines flüssigkristallinen Materials, welche hohe Klärpunkte, Anisotropie des optischen Verhaltens und weite Bereiche der nematischen Phase aufweisen, sowie ein flüssigkristallines Material auf der Basis derselben zu entwickeln, welches sich für den Einsatz in einem weiten

Arbeitstemperaturbereich eignet.

Die Aufgabe wird dadurch gelöst, daß neue flüssigkristalline Derivate von stickstoffhaltigen heterozyklischen Systemen der allgemeinen Formel

$R^1 - A^1 - Z - A^2 - R^2$ (I)

worin $R^1$ und $R^2$ gleichzeitig oder unabhängig $-C_nH_{2n+1}$, $-C_mH_{2m+1}$, $C_nH_{2n+1}O-$, $C_mH_{2m+1}O-$, $CH_2=CH(CH_2)_k-$, -CN, -N=C=S, -Hal,

$-C\equiv C$ 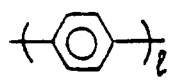 $C_mH_{2m+1}$,

k = 0-3, l = 0-2, m,n = 1-15 sind;

Z $-CH_2-CH_2-$, -CH=CH-, $-C\equiv C-$, -COO-, Einfachbindung ist:

wobei falls

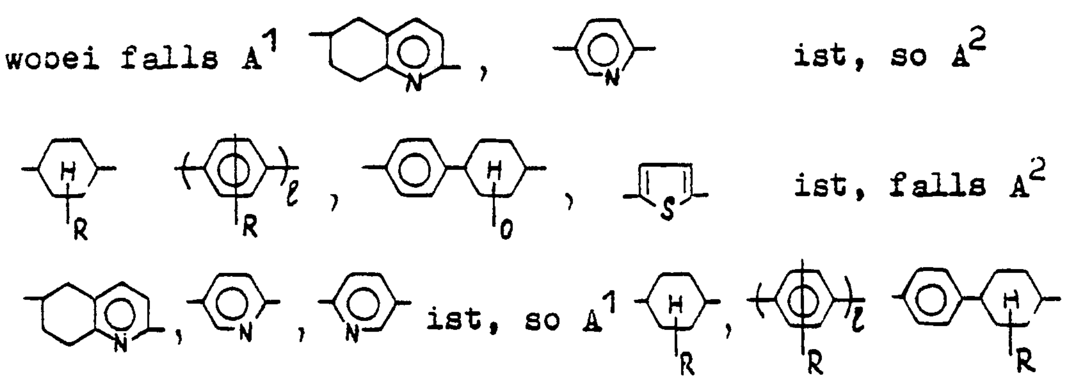
wobei falls $A^1$ , ist, so $A^2$
, , ist, falls $A^2$
, , ist, so $A^1$ ,

darstellt; R $-CH_3$, -Cl, -CN, -F ist, l 0 bis 2 beträgt, als Komponenten eines flüssigkristalline Materials vorgeschlagen werden.

Nachstehend werden flüssigkristalline Derivate von stickstoffhaltigen heterozyklischen Systemen der allgemeinen Formel $R^1 - A^1 - Z - A^2 - R_2$ (I) angeführt, die erfindungsgemäß folgende Verbindungen darstellen:

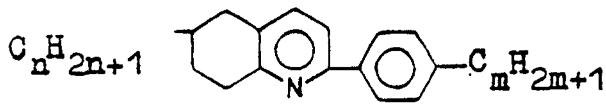

(II)

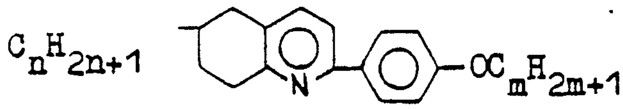

(III)

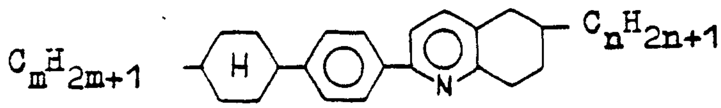

(IV)

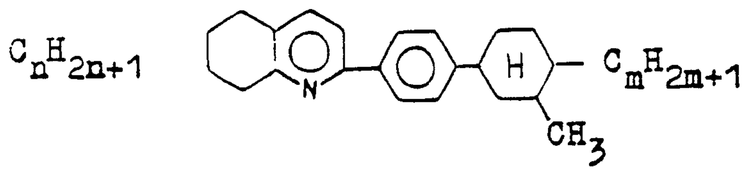

(V)

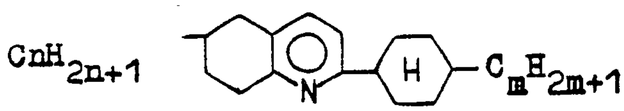

(VI)

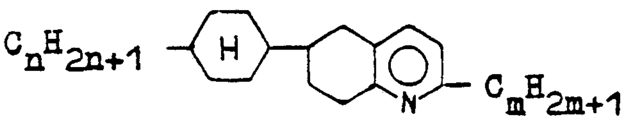

(VII)

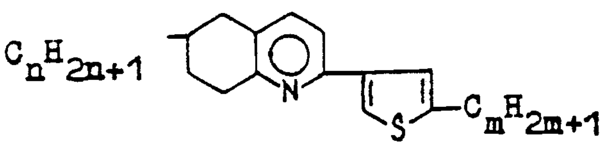

(VIII)

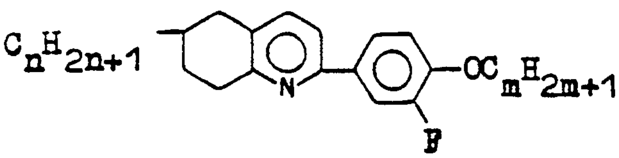

(IX)

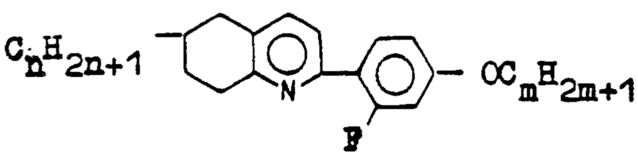

(X)

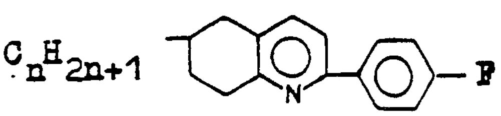

(XI)

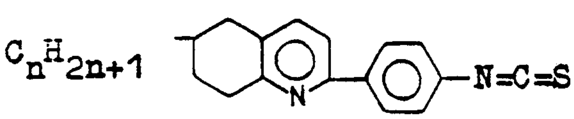

(XII)

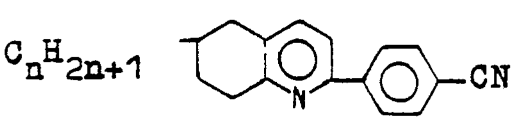

(XIII)

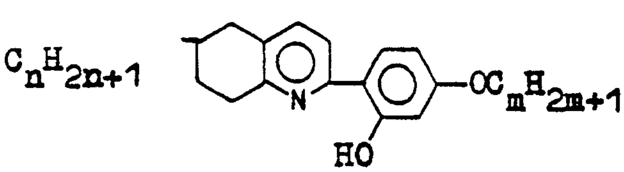

(XIV)

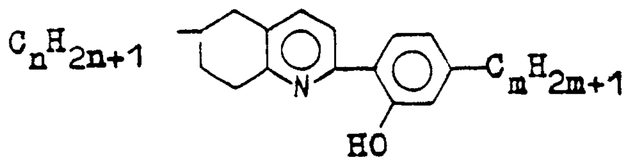

(XV)

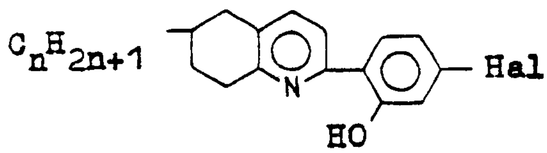

(XVI)

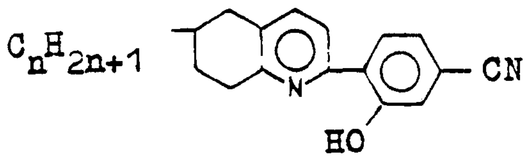

(XVII)

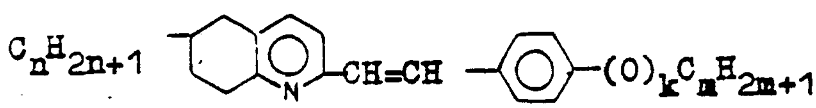

(XVIII)

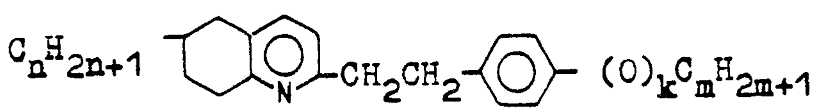

(XIX)

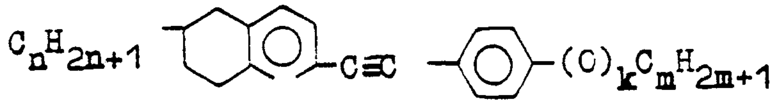

(XX)

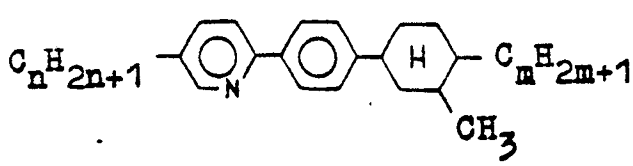

(XXI)

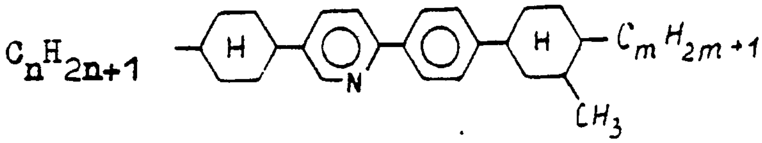

(XXII)

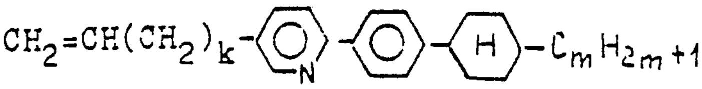

/XXIII/

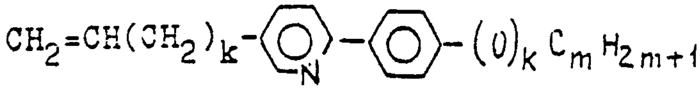

/XXIV/

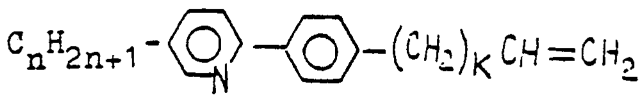

/XXV/

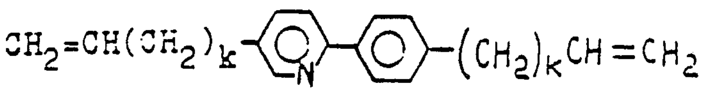

/XXVI/

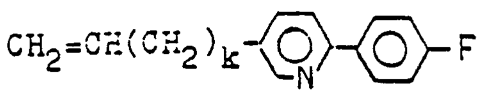

/XXVII/

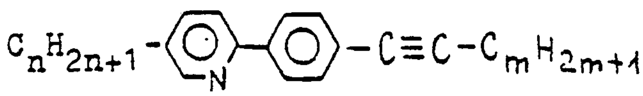

/XXVIII/

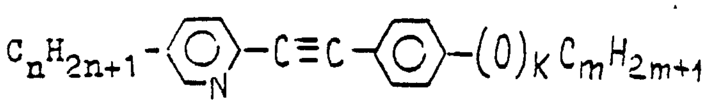

/XXIX/

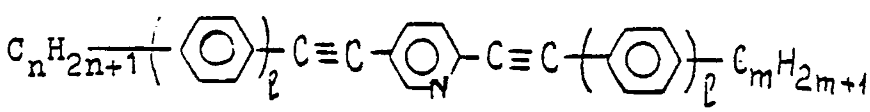

/XXX/

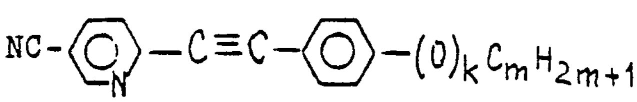

/XXXI/

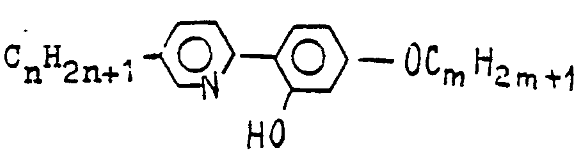

/XXXII/

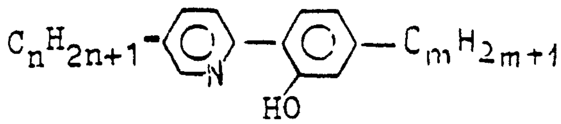

/XXXIII/

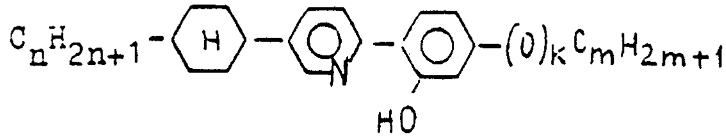

/XXXIV/

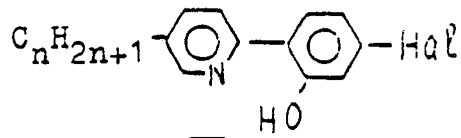

/XXXV/

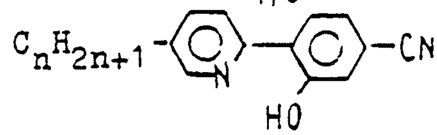

/XXXVI/

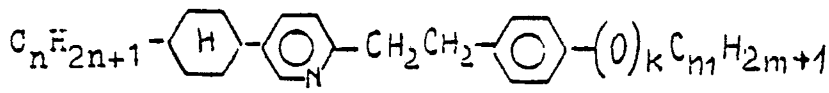

/XXXVII/

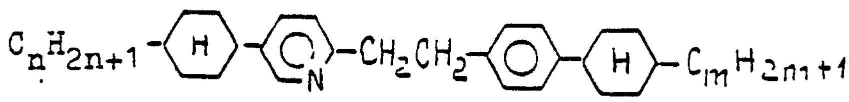

/XXXVIII/

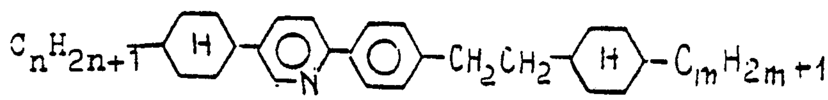

/XXXIX/

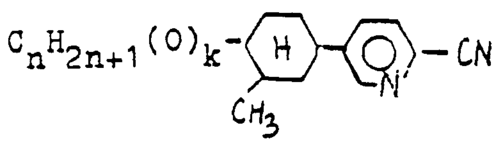

/XL/

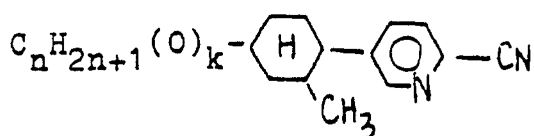

/XLI/

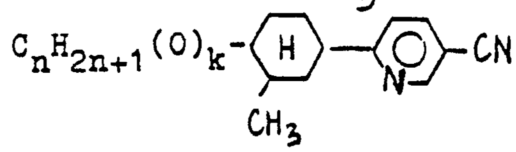

/XLII/

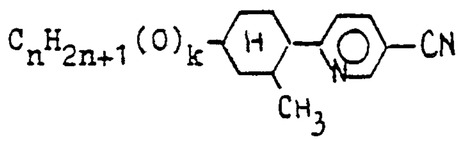
/XLIII/

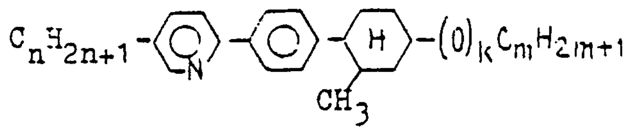
/XLIV/

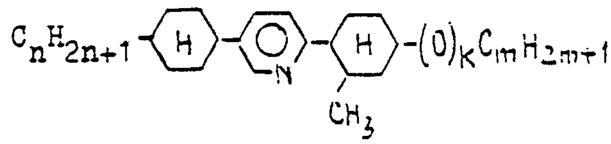
/XLV/

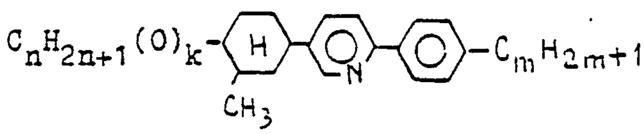
/XLVI/

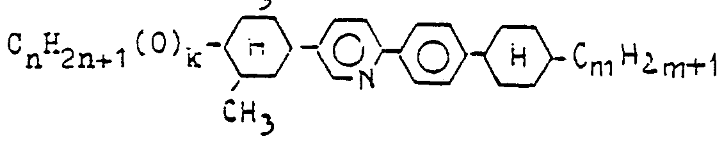
/XLVII/

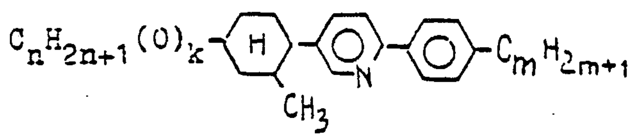
/XLVIII/

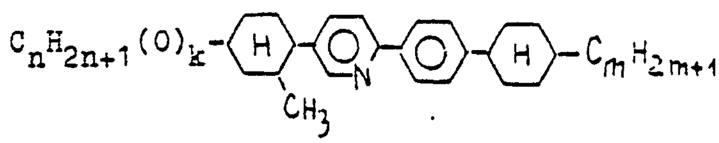
/XLIX/

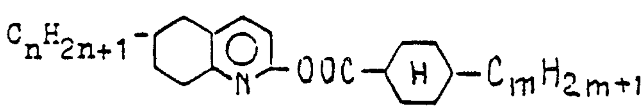
/L/

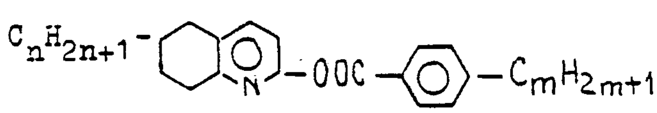
/LI/

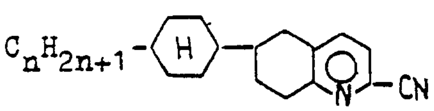
/LII)

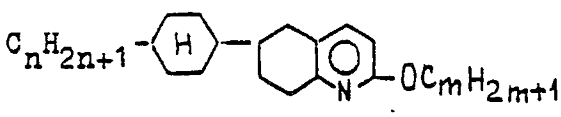
/LIII/

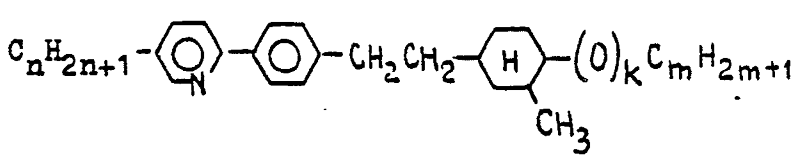
/LIV/

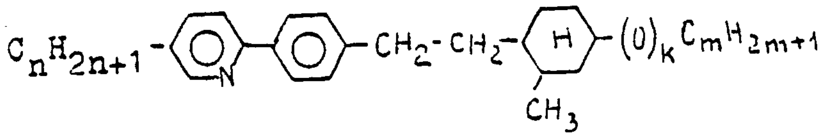

/LV/

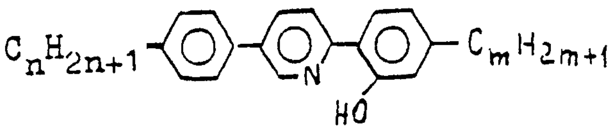

/LVI/

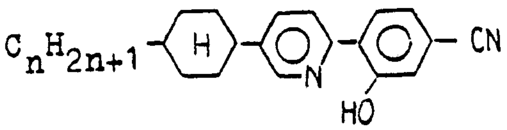

/LVII/

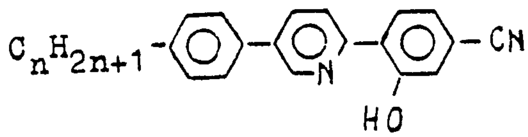

/LVIII/

Die erfindungsgemäßen Verbindungen zeichnen sich durch hohe Klärpunkte, Anisotropie des optischen Verhaltens und weite Bereiche der nematischen Phase aus, d.h. durch das Spektrum der Eigenschaften, die beim Einsatz der erfindungsgemäßen Verbindungen als Komponenten von flüssigkristallinen Materialien erforderlich sind.

Vorgeschlagen wird ebenfalls ein wenigstens zwei Komponenten enthaltendes flüssigkristallines Material, bei dem erfindungsgemäß wenigstens eine der genannten Komponenten als flüssigkristallines Derivat des stickstoffhaltigen heterozyklischen Systems der Formel I bis LVIII dient.

Es ist zweckmäßig, daß das flüssigkristalline Material 1 bis 50 Masse% flüssigkristallines Derivat des stickstoffhaltigen heterozyklischen Systems der Formel I enthält.

Das flüssigkristalline Material mit solchem Mischungsverhältnis weist die optimalen Eigenschaften auf.

Die angegebenen flüssigkristallinen Materialien werden bei Mikrorechenmaschinen, Uhren, elektrooptischen Anzeigeelementen Verwendung finden.

Die beste Ausführungsform der Erfindung

Der Verbindungen II, III, IV, V, VI, VIII, IX, X, XI, XIV, XV, XVI UND XVII sind durch Kondensation von Man nich-Salzen mit 4-Alkylcyclohexanon und anschließende Zyklisierung der 1,5-Dicarbonylverbindung mit Hydroxylammoniumchlorid zu einem entsprechenden Derivat von Tetrahydrochinolin synthetisiert. Die Verbindung XII wird durch Folgereaktionen aus 2-(4-Nitrophenyl)-6-alkyl-5,6,7,8-tetrahydrochinolin erhalten. Die Verbindung XIII wird durch Umsetzen von 2-(4-Bromphenyl-6-alkyl-5,6,7,8-tetrahydrochinolin mit Kupfer yanid gewonnen.

Die Verbindung VII wurde durch Umsetzen des entsprechenden Mannich-Salzes mit 4-(trans-4-Alkylcyclohexyl)-cyclohexanon und anschließende Entschwefelung des Thiophenderivats hergestellt.

Die Verbindung XVIII wurde durch Kondensation von 2-Methyl-6-alkyl-5,6,7,8-tetrahydrochinolin mit n-Alkyl-oder Alkoxybenzaldehyd hergestellt.

Die Verbindung XIX wurde durch Hydrierung der Verbindung XVIII hergestellt.

Die Verbindung XX wurde durch Bromierung an der Doppelbindung der Verbindung XVIII und anschließende Alkalibehandlung hergestellt.

Die Verbindungen XXI und XXII wurden durch Kondensation von 1-acryloyl-4-substituierten Benzolen mit dem betreffenden Enamin zu 3,6-disubstituierten 3,4-Dihydro-2-N-piperidino-2H-pyranen synthetisiert, wobei die letztgenannten durch Umsetzen mit Hydroxylammoniumchlorid zu Pyridinderivaten erhalten wurden.

Die Verbindungen XXIII, XXIV, XXVII wurden durch Umsetzen von n-substituiertem Phenyllithium mit Pyridin und anschließender Alkylierung durch entsprechendes Alkenylbromid hergestellt.

Die Verbindungen XXV und XXVI wurde durch Umsetzen des entsprechenden magnesiumorganischen Derivats mit 2-(4-jodphenyl)-5-substituiertem Pyridin hergestellt.

Die Verbindungen XXVIII wurde durch Umsetzen von Alkylacetylenen mit 2-(4-Jodphenyl)-5-alkylpyridin in Gegenwart eines Katalysators hergestellt. Ähnlich wurden aus 2-Jod-5-brompyridin durch Folgereaktionen die Verbindungen XXIX, XXX, XXXI synthetisiert.

Die Verbindungen XXXII, XXXIII, XXXIV, XXXV, XXXVI wurden durch Kondensation von 1-acryloyl-2,4-disubstituierten Benzolen mit Enamin und anschließender Behandlung des entsprechenden Pyrans mit Hydroxylammoniumchlorid und Hydrolyse der 2-Alkoxygruppe zur 2,5-disubstituiertem Pyridinderivat synthetisiert.

Die Verbindungen XXXVII und XXXVIII wurden aus dem entsprechenden Derivat von 2-Methyl-pyridin und substituierten Benzaldehyden unter anschließender Hydrierung an der Doppelbindung synthetisiert.

Die Verbindung XL und XLI wurden aus entsprechenden Pyridinderivaten mit der Kohlenstoff-Kohlenstoff-Doppelbindung in 2-Stellung durch Oxydation derselben zu 5-substituierter Pikolinsäure und Folgeumsetzung der Karbonylgruppe zu Nitrilgruppe hergestellt.

Die Verbindungen XLII und XLIII wurden durch Kondensation von Acetylderivaten mit Cyanacetamid, Substitution der 2-Hydroxygruppe durch Chlor und Reduzierung desselben mit 5% Pd/C gewonnen.

Die Verbindungen XLIV bis XLIX, LIV, LV, LVI, LVII und LVIII wurden durch Kondensation entsprechender Enamine und Vinylketone und anschließende Behandlung des Reaktionsprodukts mit Hydroxylammoniumchlorid hergestellt.

Die Verbindungen L, LI wurden durch Acylierung von 6-Alkyl-5,6,7,8-tetrahydrochinol-2-on hergestellt.

Die Verbindungen LIII wurden durch Substitution der 2-Hydroxygruppe durch Brom und anschließender Behandlung des letztgenannten durch Alkoxygruppe oder Cyanogruppe von LII hergestellt.

Das erfindungsgemäße flüssigkristalline Material erhält man durch Vermischen von wenigstens zwei flüssigkristallinen Komponenten, wobei wenigstens eine von ihnen erfindungsgemäß ein Derivat des stickstoffhaltigen heterocyclischen Systems der Formel I ist, welches in einer Menge von 1 bis 50 Masse% genommen wird. Das Vermischen erfolgt unter Erhitzen bis zur Entstehung der isotropen Phase und anschließender Abkühlung.

Als zweite Komponente des flüssigkristallinen Materials kann eine beliebige Verbindung dienen, die aus der Gruppe von Derivaten von Biphenyl, Cyclohexan, Dioxanen, Pyrimidin, Alkyl(alkoxy)phenylestern der Alkyl(alkoxy)benzoesäuren und trans-Cyclohexancarbonsäuren, Alkylphenylestern der 4-(trans-4-Alkylcyclohexyl)benzoesäure, 4-(trans-4-Alkyl-cyclohexyl)phenylestern der trans-4-Alkyl-cyclohexancarbonsaure, 1,2-Diphenylethyn, 1-Cyclohexyl-2-phenylethan ausgewählt wird.

Man wählt die Menge der Komponenten und das Mischungsverhältnis abhängig von den gegebenen Eigenschaften des flüssigkristallinen Materials aus.

Nachstehend werden Beispiele zur konkreten Ausführung der vorliegenden Erfindung angeführt, in denen man das Herstellungsverfahren und die Eigenschaften von Derivaten der stickstoffhaltigen heterozyklischen Systeme der Formel I bis LVIII sowie das Herstellungsverfahren und die Eigenschaften des flüssigkristallinen Materials angibt.

Beispiel 1

Herstellung von 2-(4-Heptylphenyl)-6-pentyl-5,6,7,8-tetrahydrochinolin (II a)

Das Gemisch von 0.1 Mol 1-(3-Dimethylaminopropionyl)-4-heptylbenzolhydrochlorid und 0,2 Mol 4-Amyl y lohexanon erhitzt man bei einer zwischen 160 und 170 °C liegenden Temperatur innerhalb von 1 Stunde, kühlt ab und gießt in 100 ml $H_2O$ aus, säuert mit 5%iger Salzsäure an, extrahiert mit Benzol, trocknet Benzol über $Na_2SO_4$, filtriert durch $SiO_2$ bei L = 5/40 und h = 2 cm und destilliert im Vakuum ab. Dem Rückstand werden 0,4 Mol $NH_2CH \cdot HCl$, 100 ml Ethanol und 50 ml Wasser zugesetzt. Das Reaktionsgut wird innerhalb von 8 Stunden gesiedet, bis zum Erreichen eines pH-Werts von 9 angesäuert und mit Benzol extrahiert. Benzol wird mit Wasser (2x50 ml) gewaschen, über $Na_2SO_4$ getrocknet und durch $SiO_2$ bei L = 5/40 und h = 2 cm filtriert. Man destilliert Benzol ab, kristallisiert den Rückstand aus Alkohol um und isoliert 0,68 g (21 %) 2- [4-Heptylphenyl] -6-pentyl-5,6,7,8-tetrahydrochinolin.

Analog werden andere Verbindungen von 2-(4-Alkylphenyl)-6-alkyl-5,6,7,8-tetrahydrochinolin der Formel II b-i hergestellt, deren Eigenschaften, Bruttoformel und Elementaranalysenbefund in der Tabelle 1 angegeben sind. Die Phasenübergangstemperaturen der Verbindung II a-i und der bekannten Pyridinderivate zeigt vergleichsweise die Tabelle 2.

Die Gesamtausbeute bei Verbindungen der Formel II beträgt 20 bis 25%. Alle neuen Stoffe der Formel II stellen bei Raumtemperatur ein weißes kristallines Pulver dar.

Tabelle 1

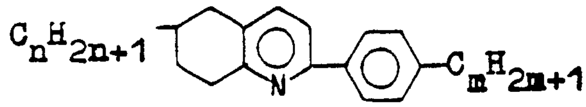

(II)

| II | n | m | $T_{C-N}$, C-S °C | $T_{S-N}$, S-I °C | $T_{N-I}$, °C |
|---|---|---|---|---|---|
| a | 5 | 7 | 37,0 | 76,0 | - |
| b | 3 | 3 | 63,0 | - | 59,0$^x$ |
| c | 3 | 5 | 55,4 | - | 66,0 |
| d | 3 | 2 | 70,0 | - | 41,0$^x$ |
| e | 5 | 5 | 43,0 | 72,7 | 73,6 |
| f | 7 | 7 | 22,0 | 84,0 | - |
| g | 5 | 9 | 21,0 | 73,5 | - |
| h | 7 | 9 | 33,0 | 83,0 | - |
| i | 7 | 5 | 24,0 | 82,0 | - |

$^x$der monotrope Übergang

Fortsetzung der Tabelle 1

| II | Gefunden in % | | Bruttoformel | Berechnet in % | |
|---|---|---|---|---|---|
| | C | H | | C | H |
| a | 85,79 | 10,50 | $C_{27}H_{39}N$ | 85,86 | 10,41 |
| b | 85,90 | 9,30 | $C_{21}H_{27}N$ | 85,96 | 9,27 |
| c | 85,87 | 9,80 | $C_{23}H_{31}N$ | 85,92 | 9,72 |
| d | 85,91 | 9,16 | $C_{20}H_{25}N$ | 85,97 | 9,02 |
| e | 85,85 | 10,04 | $C_{25}H_{35}N$ | 85,90 | 10,09 |
| f | 85,90 | 10,56 | $C_{29}H_{43}N$ | 85,86 | 10,68 |
| g | 85,79 | 10,71 | $C_{29}H_{43}N$ | 85,86 | 10,68 |
| h | 85,83 | 10,87 | $C_{31}H_{47}N$ | 85,85 | 10,92 |
| i | 85,81 | 10,43 | $C_{27}H_{39}N$ | 85,86 | 10,41 |

Die Struktur von Verbindungen der Formel II mittels paramagnetischer Resonanzspektroskopie nachgewiesen

Tabelle 2

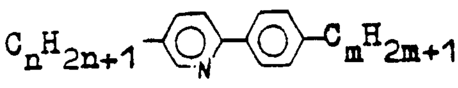 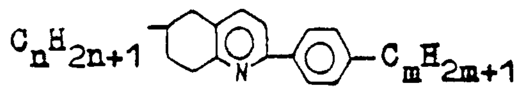 (II)

| n | m | $T_{C-N}$, $_{C-S}$ °C | $T_{S-N}$, $_{S-I}$ °C | $T_{N-I}$ °C | II | n | m | $T_{C-N}$, $_{C-S}$ °C | $T_{S-N}$, $_{S-I}$ °C | $T_{N-I}$ °C |
|---|---|---|---|---|---|---|---|---|---|---|
| | | | | | a | 5 | 7 | 37,0 | 76,0 | - |
| | | | | | b | 3 | 3 | 63,0 | - | 59,0$^x$ |
| | | | | | c | 3 | 5 | 55,4 | - | 66,0 |
| | | | | | d | 3 | 2 | 70,0 | - | 41,0$^x$ |
| 5 | 5 | 33,0 | 30,7$^x$ | - | e | 5 | 5 | 43,0 | 72,7 | 73,6 |
| 5 | 7 | 10,0 | 38,5 | - | | | | | | |
| 7 | 5 | 31,0 | 46,5 | - | f | 7 | 5 | 24,0 | 82,0 | - |
| | | | | | g | 7 | 7 | 22,0 | 84,0 | - |
| | | | | | h | 7 | 9 | 33,0 | 83,0 | - |
| | | | | | i | 5 | 9 | 21,0 | 73,5 | - |

$^x$der monotrope Übergang

Die Tabelle 2 zeigt, daß die Klärpunkte von Verbindungen der Formel II a-i die der bekannten Analoga der Pyridinderivate übersteigen.

Tabelle 3

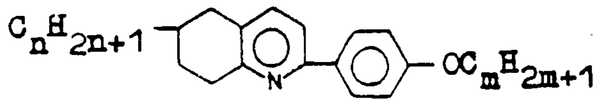 (III)

| III | n | m | $T_{C-N, C-S}$ °C | $T_{S-N, S-I}$ °C | $T_{N-I}$ °C |
|---|---|---|---|---|---|
| a | 1 | 10 | 73,0 | - | - |
| b | 3 | 5 | 59,0 | - | 90,0 |
| c | 3 | 6 | 82,0 | - | 95,0 |
| d | 5 | 5 | 87,2 | - | 99,0 |
| e | 5 | 6 | 66,0 | 84,0 | 102,4 |
| f | 7 | 10 | 85,0 | 102,0 | - |
| g | 7 | 3 | 93,0 | - | 92[x] |
| h | 6 | 5 | 85,0 | 87,0 | 99,0 |

[x] der monotrope Übergang

Fortsetzung der Tabelle 3

| III | Gefunden in % | | Brutto-formel | Berechnet in % | |
|---|---|---|---|---|---|
| | C | H | | C | H |
| a | 82,31 | 9,76 | $C_{26}H_{37}NO$ | 82,27 | 9,83 |
| b | 81,79 | 9,30 | $C_{23}H_{31}NO$ | 81,85 | 9,26 |
| c | 82,03 | 9,39 | $C_{24}H_{33}NO$ | 82,00 | 9,46 |
| d | 82,20 | 9,56 | $C_{25}H_{35}NO$ | 82,14 | 9,65 |
| e | 82,30 | 9,77 | $C_{26}H_{37}NO$ | 82,27 | 9,82 |
| f | 82,79 | 10,66 | $C_{32}H_{49}NO$ | 82,88 | 10,65 |
| g | 82,16 | 9,70 | $C_{25}H_{35}NO$ | 82,14 | 9,65 |
| h | 82,17 | 9,90 | $C_{26}H_{37}NO$ | 82,27 | 9,83 |

x der monotrope Übergang

Beispiel 2

Herstellung von 2-(4-Alkoxyphenyl)-6-alkyl-5,6,7,8-tetrahydrochinolin (III)

Die Verbindungen der Formel III werden analog zu den Verbindungen der Formel II durch Reaktion von 4-Alkylcyclohexanonen mit 1-(3-Dimethylaminopropyionyl)-4-alkoxybenzolhydrochlorid und anschließende Kondensation des 1,5-Dicarbonylderivates mit Hydroxylammoniumchlorid in einer Gesamtausbeute von 20 bis 25 % hergestellt.

Die Eigenschaften der Verbindungen III a-h und die Elementaranalysenangaben sind in der Tabelle 3 dargestellt.

Die Phasenübergangstemperaturen der Verbindungen III a-h und die Elementaranalysenangaben sind in der Tabelle 3 dargestellt.

Phasenübergangstemperaturen der Verbindungen III a-h und der Pyridinanaloge zeigt vergleichsweise die Tabelle 4.

Tabelle 4

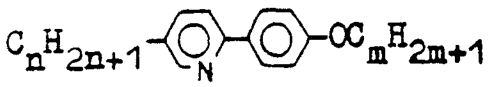 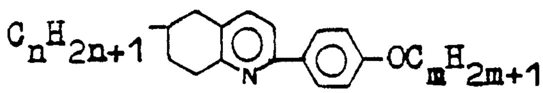 (III)

| n | m | $T_{C-N}$ / $T_{C-S}$ °C | $T_{S-N}$ / $T_{S-I}$ °C | $T_{N-I}$ °C | III | n | m | $T_{C-N}$ / $T_{C-S}$ °C | $T_{S-N}$ / $T_{S-I}$ °C | $T_{N-I}$ °C |
|---|---|---|---|---|---|---|---|---|---|---|
| | | | | | a | 1 | 10 | 73,0 | - | - |
| | | | | | b | 3 | 5 | 59,0 | - | 90,0 |
| 3 | 6 | 50,0 | 72,0 | - | c | 3 | 6 | 82,0 | - | 95,0 |
| 5 | 5 | 55,5 | - | 62,0 | d | 5 | 5 | 87,2 | - | 99,0 |
| 6 | 5 | 21,0 | 58,0 | 60,5 | e | 6 | 5 | 85,0 | 87,0 | 99,0 |
| | | | | | f | 5 | 6 | 66,0 | 84,0 | 102,4 |
| | | | | | g | 7 | 3 | 93,0 | - | $92,0^{x}$ |
| | | | | | h | 7 | 10 | 85,0 | 102,0 | - |

Nachstehende Beispiele (Tabelle 5) erläutern die Verwendung der erfindungsgemäßen Verbindungen II und III als Komponenten der nach der oben beschriebenen Methodik hergestellten flüssigkristallinen Materialien.

Die Zusammensetzungen in den Beispielen sind in Masse% angegeben.

Tabelle 5

| Nr. | Zusammensetzung | Masse% | Bereich der nematischen Phase in °C |
|---|---|---|---|
| 1 | 2 | 3 | 4 |
| 3. | 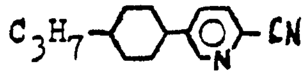 | 20 | -15 + 60,1 |
| | 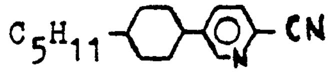 | 32 | |
| | 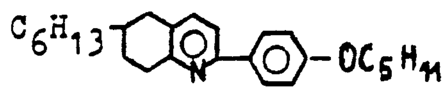 (IIIh) | 5 | |
| | 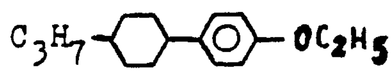 | 16 | |
| | 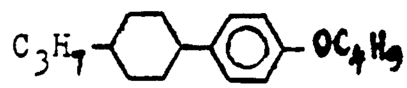 | 12 | |
| | 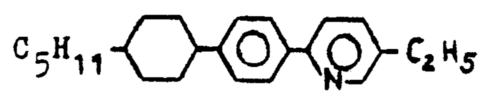 | 10 | |
| | 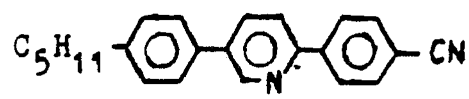 | 5 | |
| 4. | 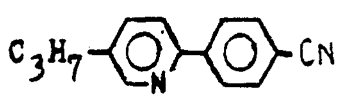 | 20 | -17 + 64,8 |
| | 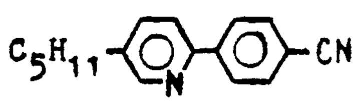 | 30 | |
| | 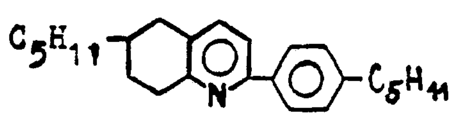 (IIe) | 5 | |

Fortsetzung der Tabelle 5

| 1 | 2 | 3 | 4 |
|---|---|---|---|
| | 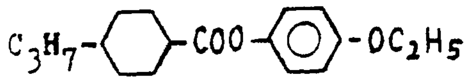 | 17 | |
| | 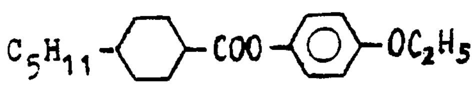 | 18 | |
| | 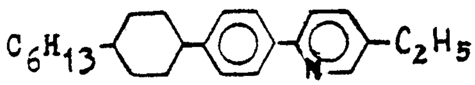 | 10 | |
| 5. | 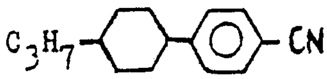 | 13 | -15 + 82,2 |
| | 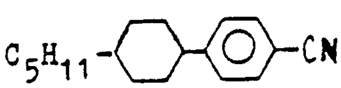 | 17 | |
| | 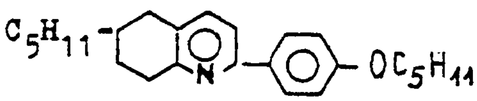 (IIId) | 7 | |
| | 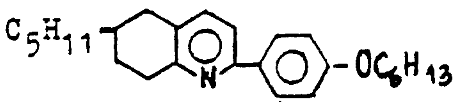 (IIIe) | 8 | |
| | 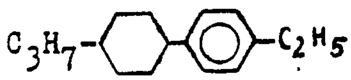 | 10 | |
| | 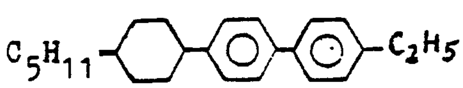 | 15 | |
| | 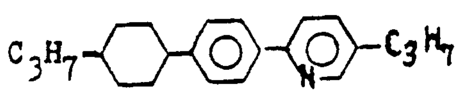 | 20 | |
| | 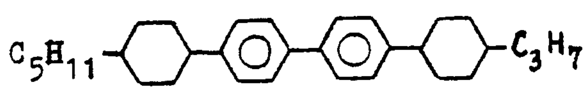 | 10 | |
| 6. | 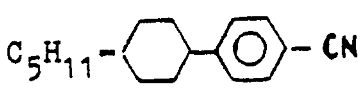 | 12 | -18 + 80,1 |
| | 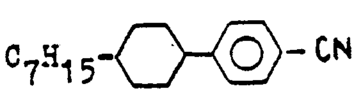 | 18 | |
| | 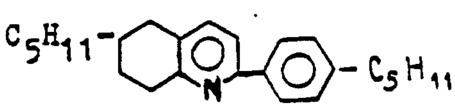 (IIe) | 10 | |

Fortsetzung der Tabelle 5

| 1 | 2 | 3 | 4 |
|---|---|---|---|
| | 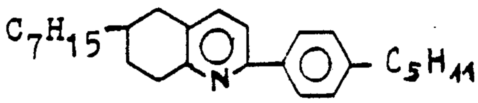 (IIi) | 5 | |
| | 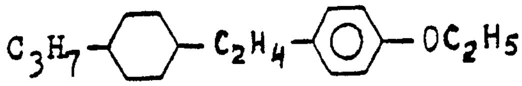 | 8 | |
| | 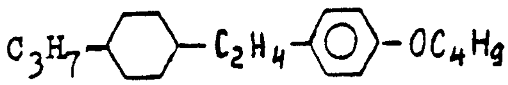 | 8 | |
| | 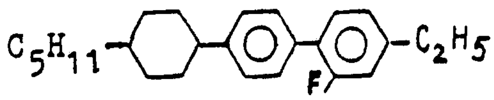 | 15 | |
| | 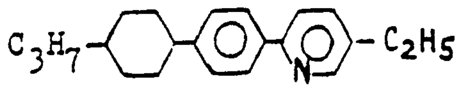 | 10 | |
| | 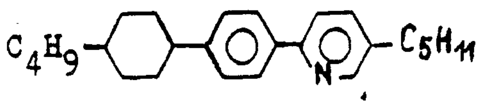 | 10 | |
| | 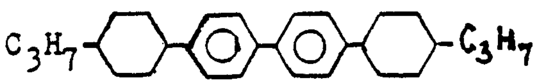 | 4 | |
| 7. | 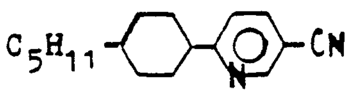 | 8 | -21+88,3 |
| | 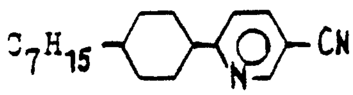 | 12 | |
| | 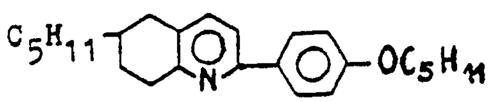 (IIId) | 8 | |
| | 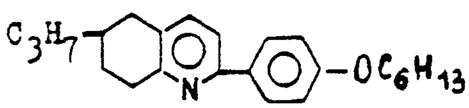 (IIIc) | 8 | |
| | 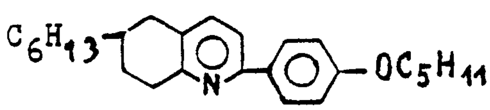 (IIIh) | 9 | |
| | 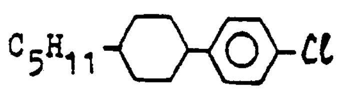 | 20 | |
| | 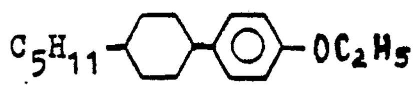 | 10 | |

Fortsetzung der Tabelle 5

| 1 | 2 | 3 | 4 |
|---|---|---|---|
| | 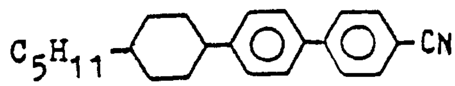 | 5 | |
| | 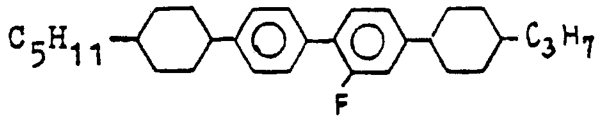 | 10 | |
| | 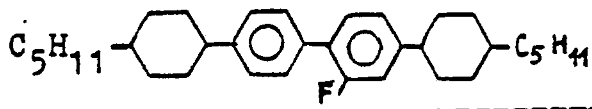 | 10 | |
| 8 | 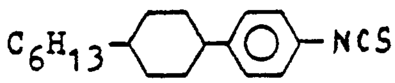 | 8 | -16+94,8 |
| | 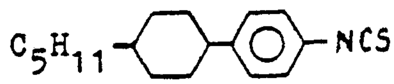 | 12 | |
| | 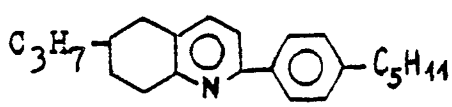 (IIc) | 10 | |
| | 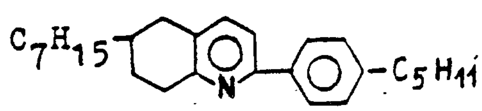 (IIi) | 15 | |
| | 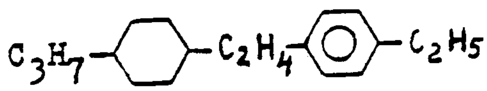 | 15 | |
| | 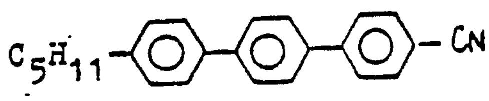 | 8 | |
| | 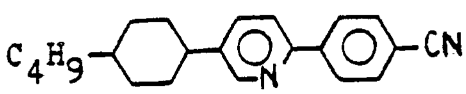 | 7 | |
| | 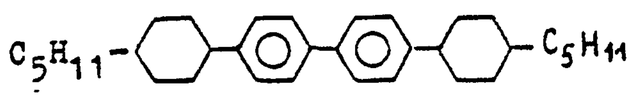 | 10 | |
| | 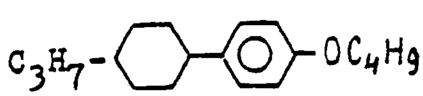 | 10 | |
| | 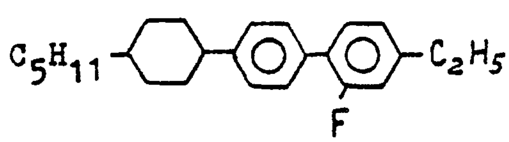 | 5 | |
| 9 | 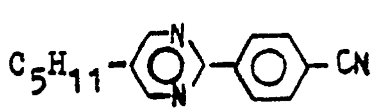 | 8 | -14+100,4 |

Fortsetzung der Tabelle 5

| 1 | 2 | 3 | 4 |
|---|---|---|---|
| | 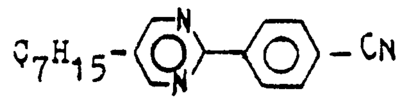 | 12 | |
| | 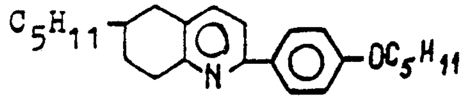 (IIId) | 15 | |
| | 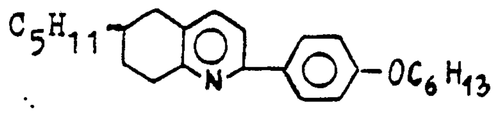 (IIIe) | 15 | |
| | 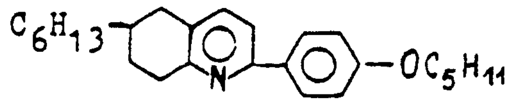 (IIIh) | 10 | |
| | 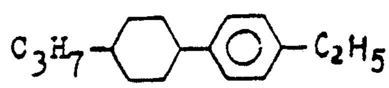 | 10 | |
| | 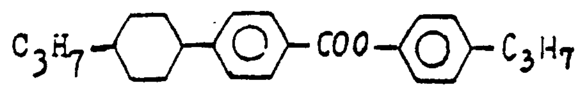 | 8 | |
| | 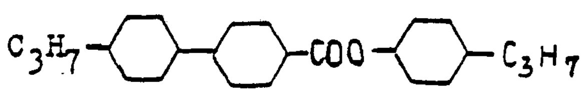 | 7 | |
| | 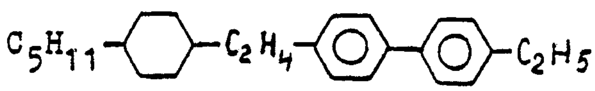 | 10 | |
| | 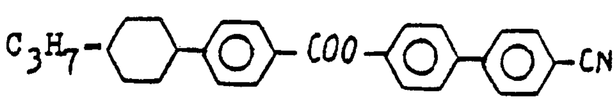 | 5 | |
| 10 | 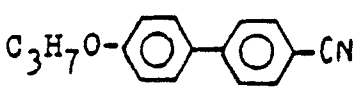 | 8 | -10+96,3 |
| | 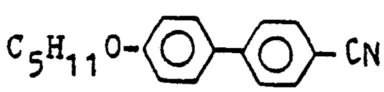 | 12 | |
| | 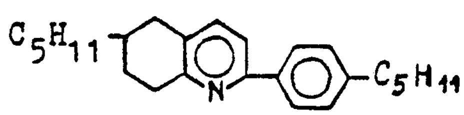 (IIc) | 10 | |
| | 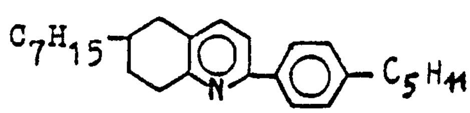 (IIi) | 10 | |
| | 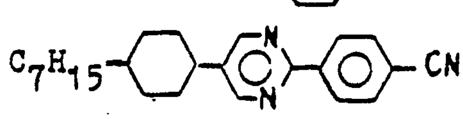 | 8 | |

Fortsetzung der Tabelle 5

| 1 | 2 | 3 | 4 |
|---|---|---|---|
| | 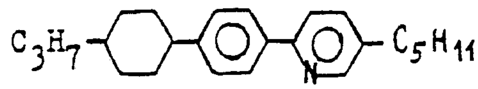 | 7 | |
| | 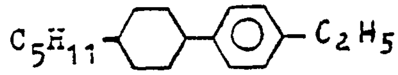 | 15 | |
| | 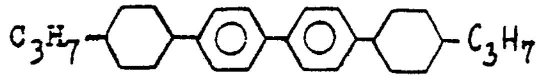 | 10 | |
| | 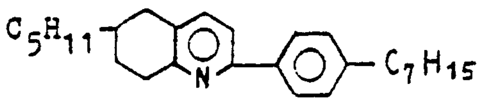 (IIa) | 10 | |
| | 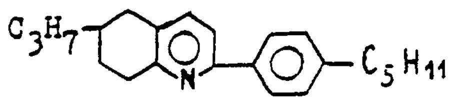 (IIc) | 10 | |
| 11 | 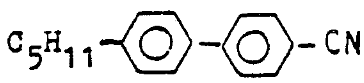 | 5 | -12+105,2 |
| | 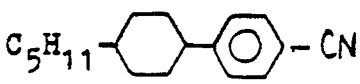 | 5 | |
| | 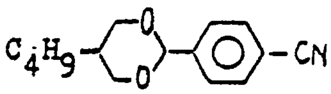 | 5 | |
| | 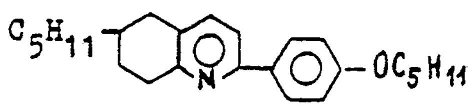 (IIId) | 10 | |
| | 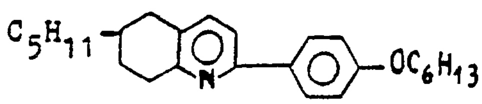 (IIIe) | 15 | |
| | 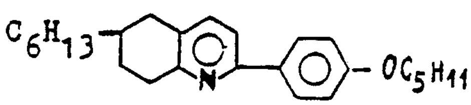 (IIIh) | 15 | |
| | 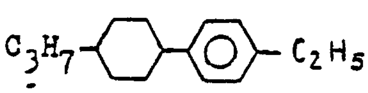 | 10 | |
| | 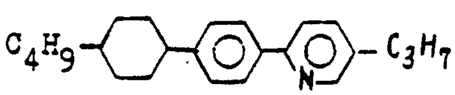 | 5 | |
| | 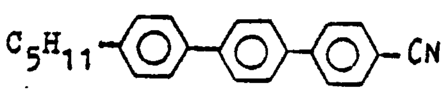 | 5 | |
| | 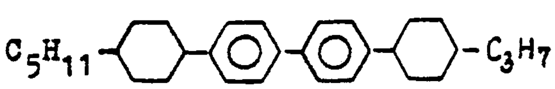 | 10 | |

Fortsetzung der Tabelle 5

| 1 | 2 | 3 | 4 |
|---|---|---|---|
| | 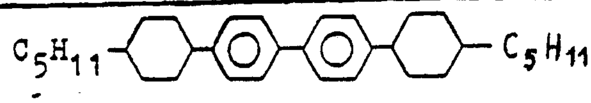 | 5 | |
| | 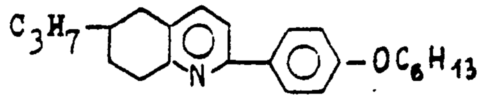 (IIIc) | 10 | |
| 12 | 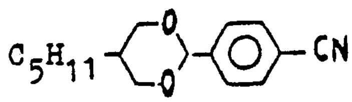 | 10 | -14+108,1 |
| | 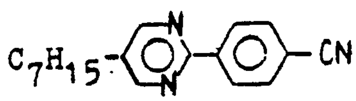 | 3 | |
| | 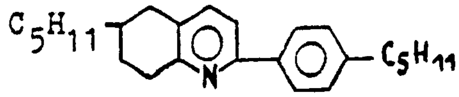 (IIc) | 15 | |
| | 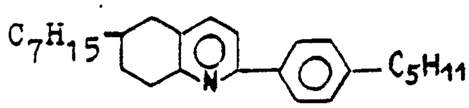 (IIi) | 15 | |
| | 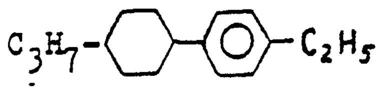 | 10 | |
| | 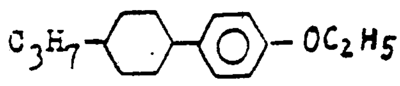 | 5 | |
| | 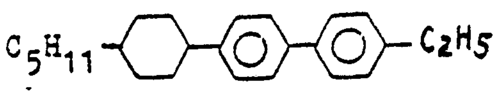 | 3 | |
| | 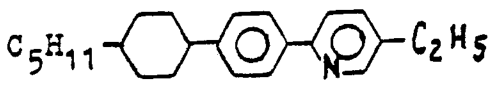 | 4 | |
| | 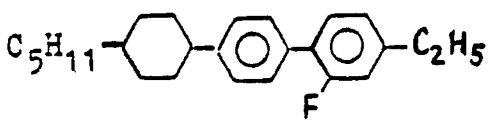 | 5 | |
| | 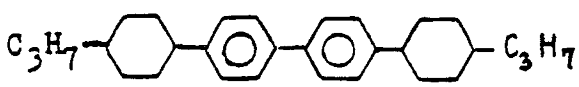 | 10 | |
| | 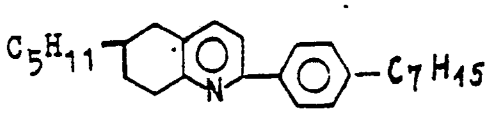 (IIa) | 10 | |
| | 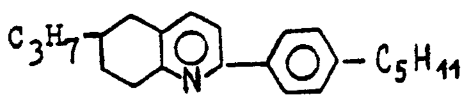 (IIc) | 10 | |
| 13 | 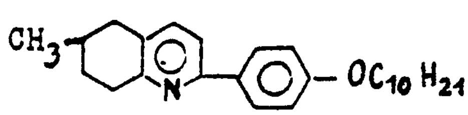 (IIIa) | 6 | -17+104,8 |

Fortsetzung der Tabelle 5

| 1 | 2 | 3 | 4 |
|---|---|---|---|
| | 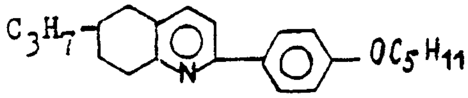 (IIIb) | 6 | |
| | 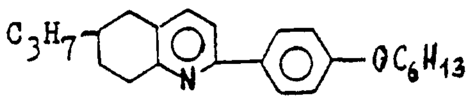 (IIIc) | 6 | |
| | 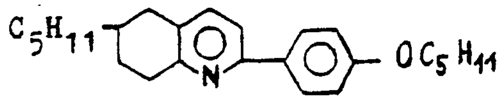 (IIId) | 7 | |
| | 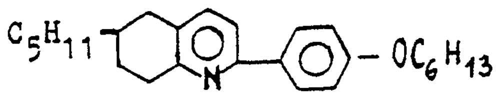 (IIIe) | 7 | |
| | 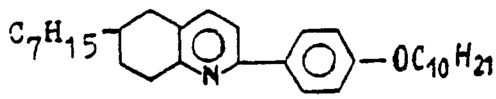 (IIIf) | 6 | |
| | 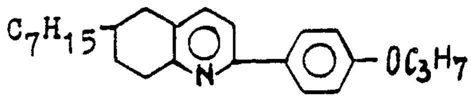 (IIIg) | 6 | |
| | 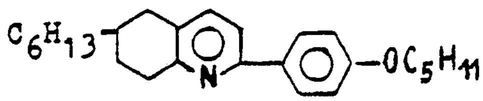 (IIIh) | 6 | |
| | 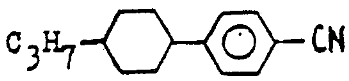 | 3 | |
| | 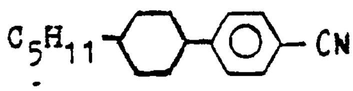 | 12 | |
| | 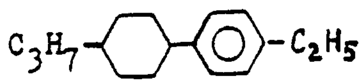 | 7 | |
| | 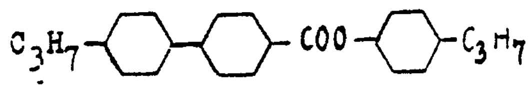 | 10 | |
| | 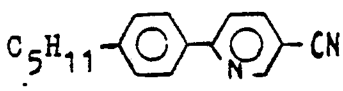 | 3 | |
| | 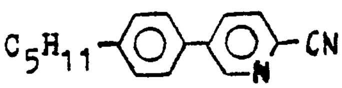 | 5 | |
| | 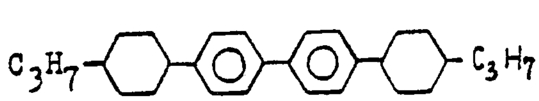 | 10 | |
| 14 | 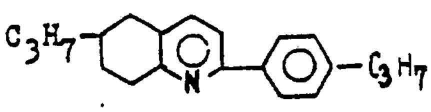 (IIIb) | 6 | -19+105,7 |

Fortsetzung der Tabelle 5

| 1 | 2 | 3 | 4 |
|---|---|---|---|
| | 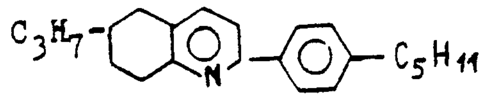 (III c) | 7 | |
| | 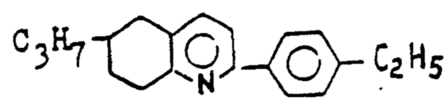 (III d) | 6 | |
| | 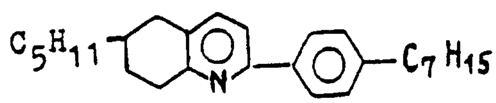 (III a) | 7 | |
| | 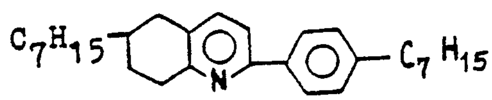 (III f) | 6 | |
| | 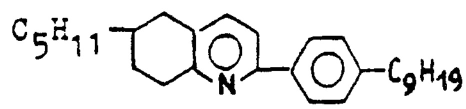 (III g) | 6 | |
| | 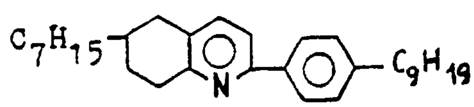 (III h) | 6 | |
| | 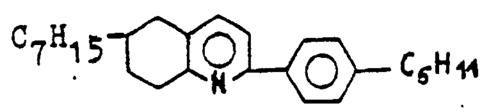 (III i) | 6 | |
| | 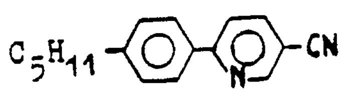 | 3 | |
| | 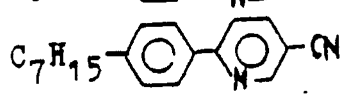 | 12 | |
| | 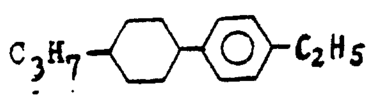 | 10 | |
| | 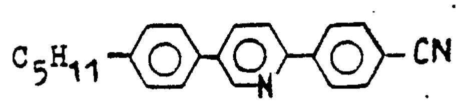 | 8 | |
| | 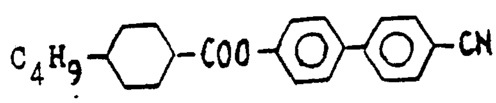 | 8 | |
| | 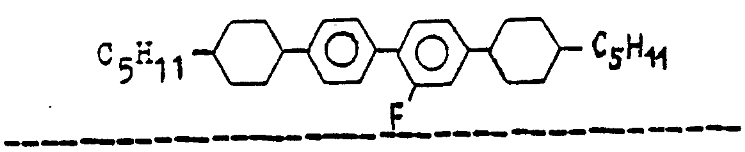 | 9 | |
| 15 | 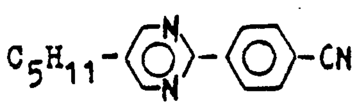 | 5 | -5+111,3 |
| | 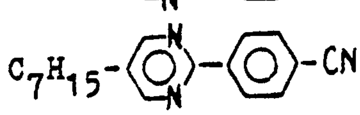 | 7 | |

Fortsetzung der Tabelle 5

| 1 | 2 | 3 | 4 |
|---|---|---|---|
| | 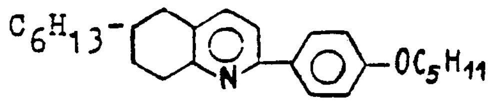 (III h) | 20 | |
| | 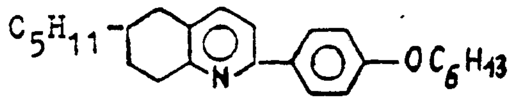 (III c) | 20 | |
| | 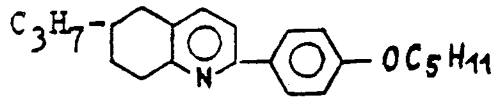 (III l) | 15 | |
| | 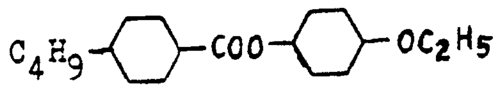 | 10 | |
| | 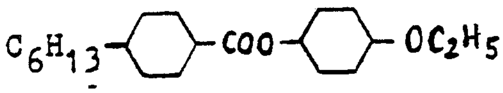 | 8 | |
| | 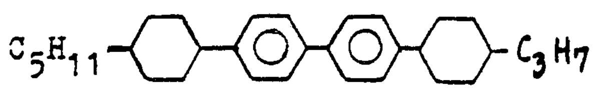 | 10 | |
| | 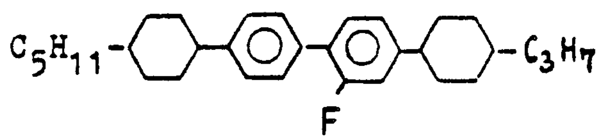 | 5 | |
| 16 | 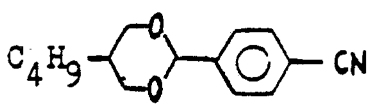 | 7 | -6+113,2 |
| | 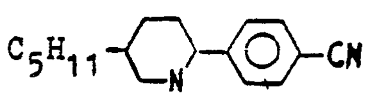 | 10 | |
| | 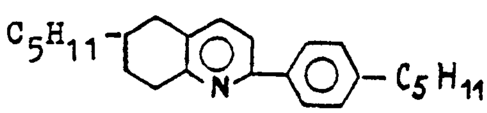 (II i) | 30 | |
| | 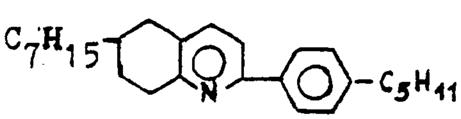 (II i) | 25 | |
| | 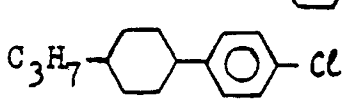 | 15 | |
| | 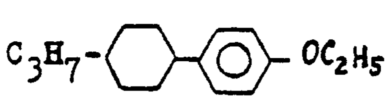 | 5 | |
| | 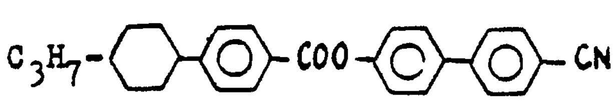 | 8 | |

Fortsetzung der Tabelle 5

| 1 | 2 | | 3 | 4 |
|---|---|---|---|---|
| 17 | 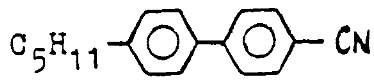 | | 25 | -23+57,8 |
| | 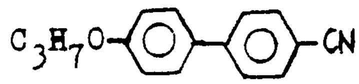 | | 10 | |
| | 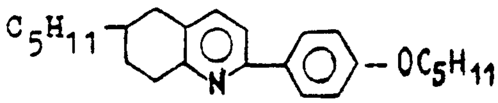 | (IIId) | 3 | |
| | 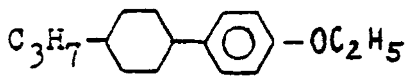 | | 8 | |
| | 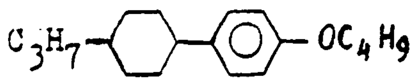 | | 12 | |
| | 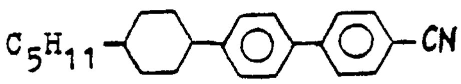 | | 10 | |
| | 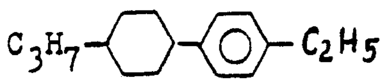 | | 10 | |
| | 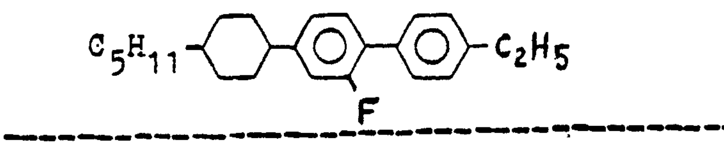 | | 22 | |
| 18 | 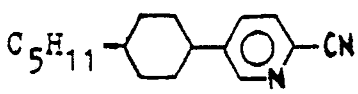 | | 8 | -28+65,1 |
| | 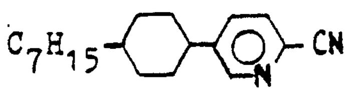 | | 12 | |
| | 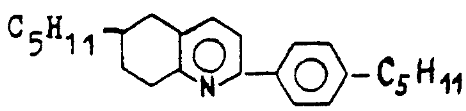 | (IIe) | 3 | |
| | 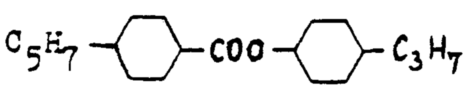 | | 8 | |
| | 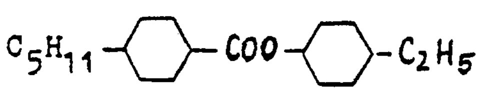 | | 8 | |
| | 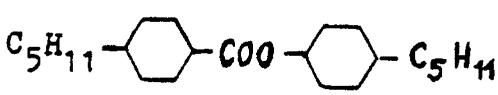 | | 8 | |
| | 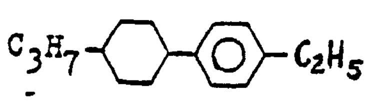 | | 20 | |

Fortsetzung der Tabelle 5

| 1 | 2 | 3 | 4 |
|---|---|---|---|
| | 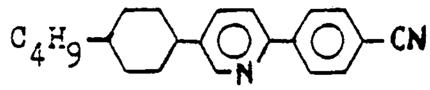 | 3 | |
| | 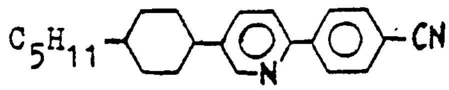 | 2 | |
| | 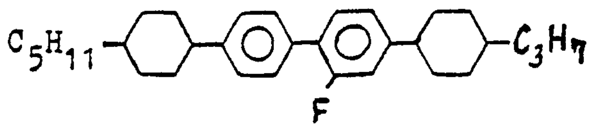 | 5 | |
| | 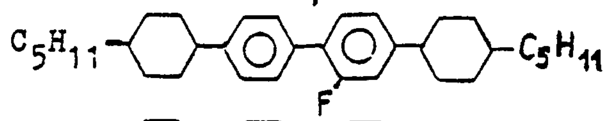 | 5 | |
| | 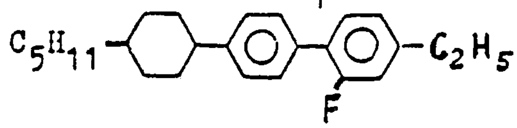 | 18 | |
| 19 | 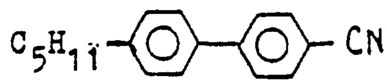 | 30 | -20+55,2 |
| | 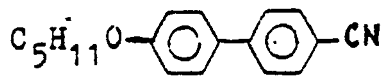 | 8 | |
| | 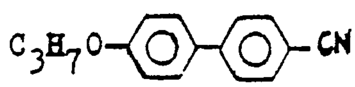 | 10 | |
| | 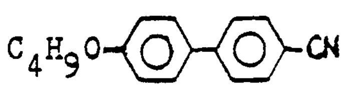 | 7 | |
| | 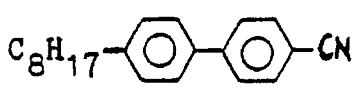 | 15 | |
| | 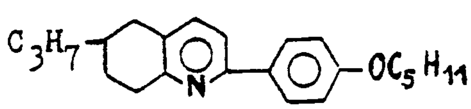 (IIIб) | 1 | |
| | 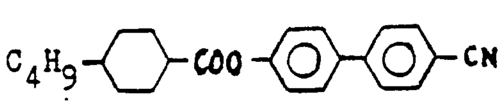 | 10 | |
| | 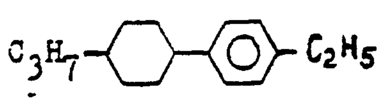 | 10 | |
| | 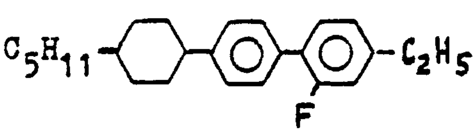 | 9 | |

**Fortsetzung der Tabelle 5**

| 1 | 2 | 3 | 4 |
|---|---|---|---|
| 20 | 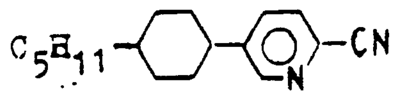 | 8 | -27+60,5 |
| | 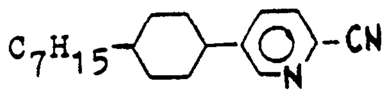 | 15 | |
| | 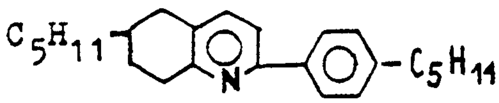 (IIe) | 1 | |
| | 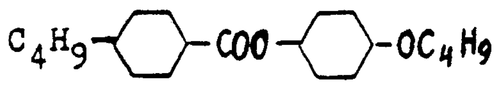 | 10 | |
| | 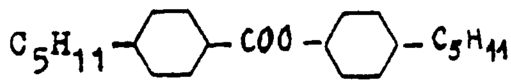 | 10 | |
| | 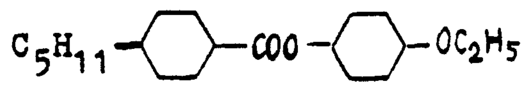 | 10 | |
| | 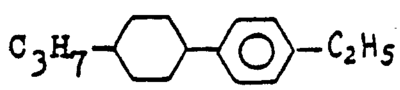 | 20 | |
| | 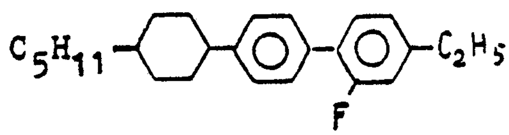 | 5 | |
| | 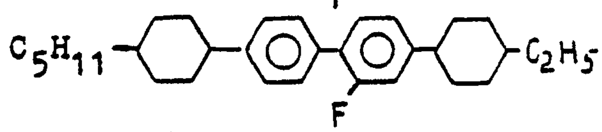 | 10 | |
| | 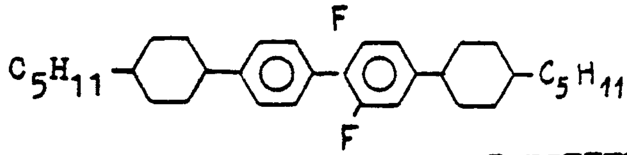 | 11 | |

Die Beispiele 3 bis 20 zeigen, daß die Verbindungen der Formeln II und III den flüssigkristallinen Materialien hohe Klärpunkte und breite Bereiche der nematischen Phase vermitteln. Diese Materialien lassen sich für electrooptische Anzeigeelemente von breiter Zweckbestimmung verwenden.

Beispiel 21

Herstellung von 4- [4-(trans-4-Alkylcyclohexyl)-phenyl] -6-alkyl-5,6,7,8-tetrahydrochinolinen (IV)

21.1 Die Verbindungen der Formel IV werden analog zu den Verbindungen der Formel II durch Umsetzen von 4-Alkylcyclohexanonen mit 1-(3-Dimethylaminopropionyl)-4-(trans-4-alkylcyclohexyl)-benzoh-

ydrochlorid und anschließender Kondensation des 1,5-Dicarbonylderivates mit Hydroxylammoniumchlorid in einer Ausbeute von 25 bis 30% hergestellt.

Die Eigenschaften der Verbindungen IV und der Elementaranalysenbefund sind in der Tabelle 6 angegeben.

Tabelle 6

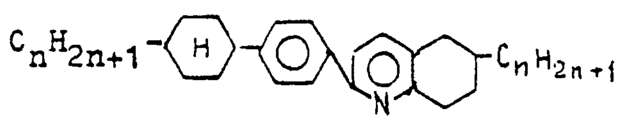

(IV)

| IV | n | m | $T_{C-N}$ °C | $T_{N-I}$ °C | Gefunden in % C | Gefunden in % H | Brutto-formel | Berechnet in % C | Berechnet in % H |
|---|---|---|---|---|---|---|---|---|---|
| a | 5 | 3 | 116 | 209 | 86,33 | 10,21 | $C_{29}H_{41}N$ | 86,29 | 10,24 |
| b | 3 | 5 | 132 | 196 | 86,21 | 10,30 | $C_{29}H_{41}N$ | 86,29 | 10,24 |
| c | 3 | 3 | 108 | 220 | 86,40 | 9,87 | $C_{27}H_{37}N$ | 86,34 | 9,93 |
| d | 4 | 3 | 100 | 208 | 86,36 | 10,11 | $C_{28}H_{39}N$ | 86,32 | 10,09 |
| e | 3 | 2 | 92 | 201 | 86,31 | 9,81 | $C_{26}H_{35}N$ | 86,37 | 9,75 |

Die Tabelle 7 zeigt die Beispiele für die Zusammensetzungen und Eigenschaften von flüssigkristallinen Materialien, welche neue Verbindungen der Formel IV enthalten.

Tabelle 7

| Nr. | Zusammensetzung | Masse% | Bereich der nematischen Phase in °C |
|---|---|---|---|
| 1 | 2 | 3 | 4 |
| 22. | 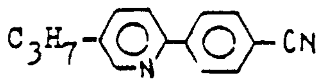 | 20 | -15+68,0 |
| | 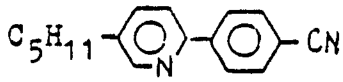 | 30 | |
| | 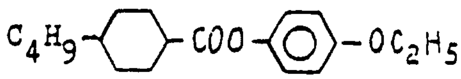 | 25 | |
| | 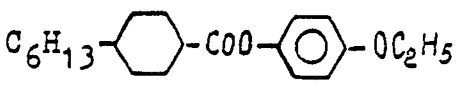 | 15 | |
| | 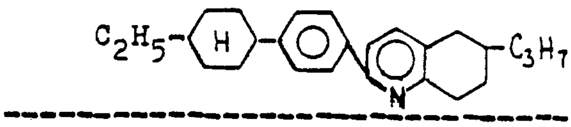 (IVc) | 10 | |
| 23. | 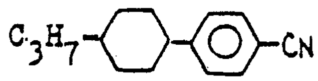 | 17 | -23+93,5 |
| | 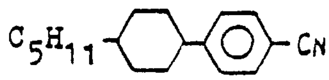 | 23 | |
| | 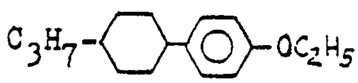 | 16 | |
| | 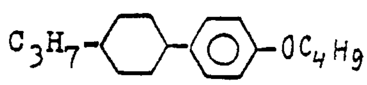 | 12 | |
| | 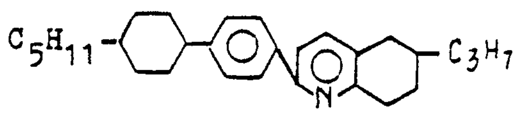 (IVb) | 22 | |
| | 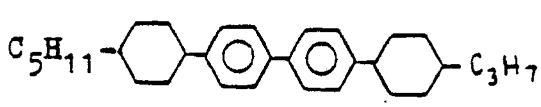 | 10 | |
| 24. | 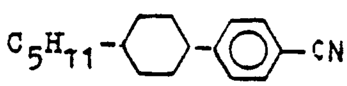 | 10 | -28+83,2 |
| | 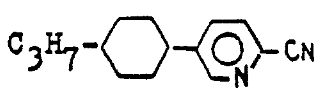 | 15 | |

Fortsetzung der Tabelle 7

| 1 | 2 | 3 | 4 |
|---|---|---|---|
| | 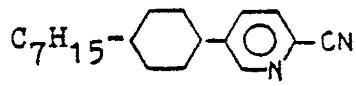 | 23 | |
| | 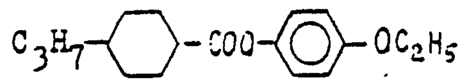 | 8 | |
| | 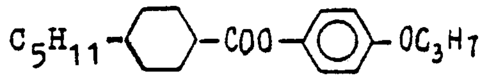 | 6 | |
| | 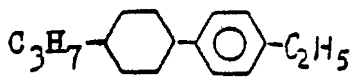 | 5 | |
| | 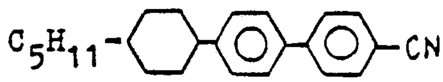 | 5 | |
| | 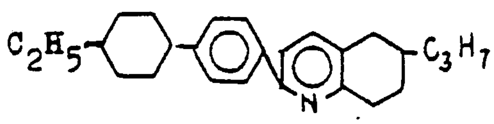 (IVe) | 5 | |
| | 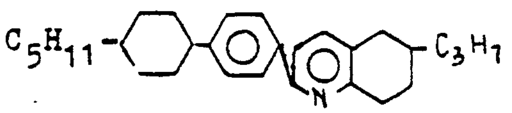 (IVb) | 5 | |
| | 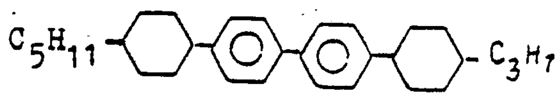 | 8 | |
| 25. | 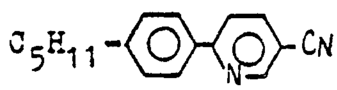 | 8 | -31+98,2 |
| | 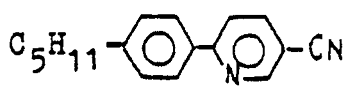 | 12 | |
| | 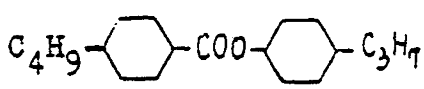 | 5 | |
| | 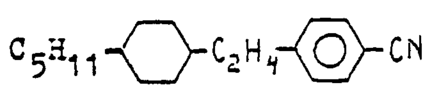 | 5 | |
| | 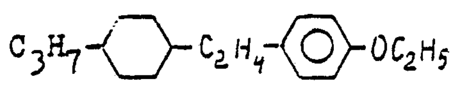 | 8 | |
| | 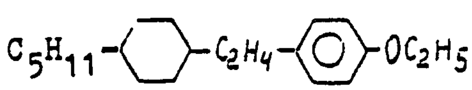 | 7 | |
| | 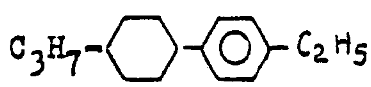 | 15 | |

Fortsetzung der Tabelle 7

| 1 | 2 | 3 | 4 |
|---|---|---|---|
| | 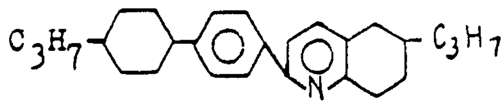 (IVc) | 10 | |
| | 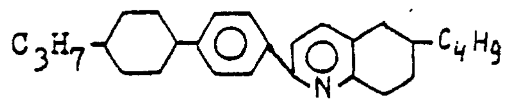 (IVd) | 10 | |
| | 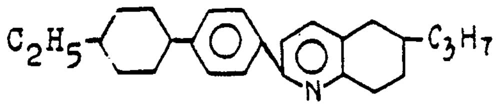 (IVe) | 10 | |
| | 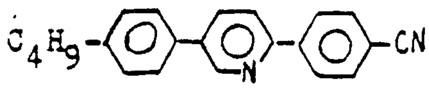 | 5 | |
| | 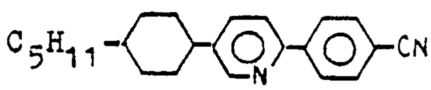 | 5 | |
| 26. | 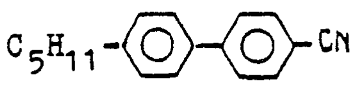 | 5 | -26+106,7 |
| | 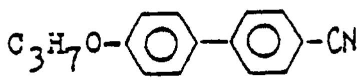 | 7 | |
| | 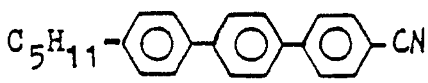 | 3 | |
| | 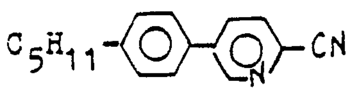 | 8 | |
| | 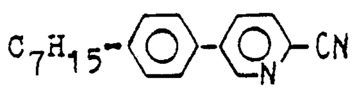 | 12 | |
| | 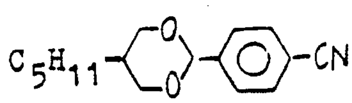 | 5 | |
| | 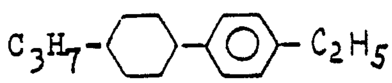 | 20 | |
| | 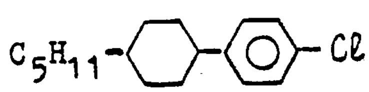 | 5 | |
| | 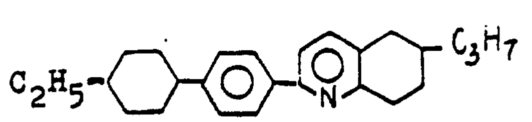 (IVe) | 10 | |

Fortsetzung der Tabelle 7

| 1 | 2 | 3 | 4 |
|---|---|---|---|
| | 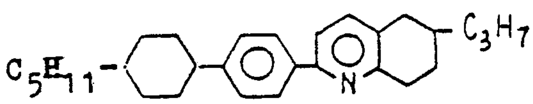 (IV b) | 5 | |
| | 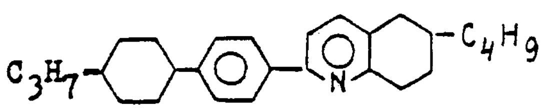 (IV d) | 5 | |
| | 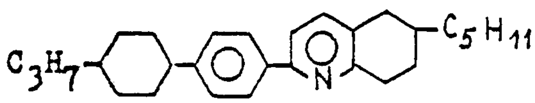 (IV a) | 5 | |
| | 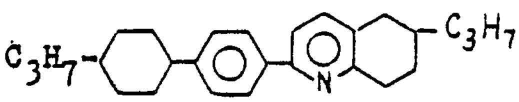 (IV c) | 10 | |
| 27. | 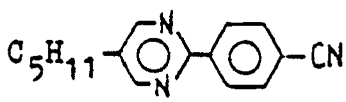 | 7 | -24+115,2 |
| | 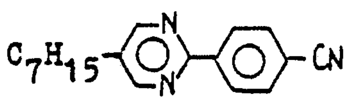 | 10 | |
| | 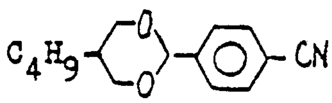 | 7 | |
| | 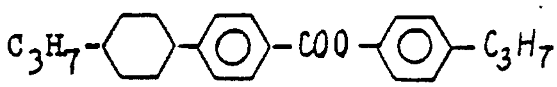 | 8 | |
| | 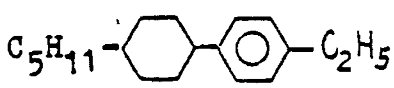 | 20 | |
| | 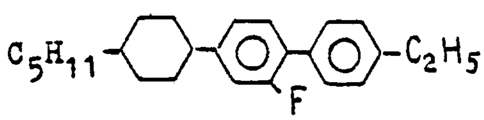 | 3 | |
| | 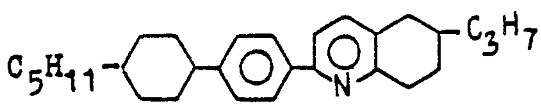 (IV b) | 5 | |
| | 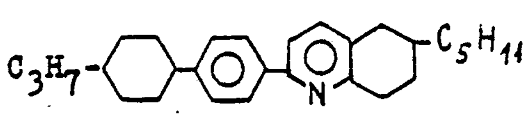 (IV a) | 5 | |

Fortsetzung der Tabelle 7

| 1 | 2 | 3 | 4 |
|---|---|---|---|
| | 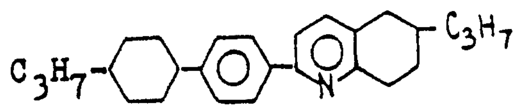 (IV c) | 5 | |
| | 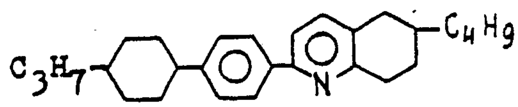 (IV d) | 10 | |
| | 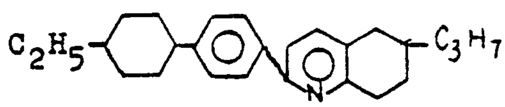 (IV e) | 10 | |
| | 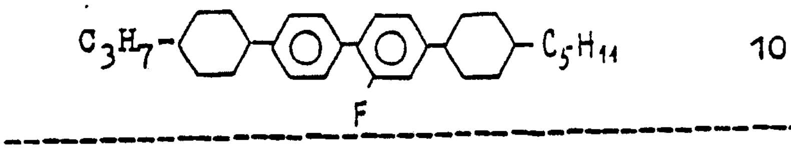 | 10 | |
| 28. | 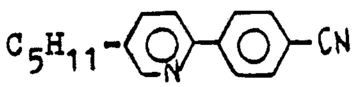 | 7 | -28+115,6 |
| | 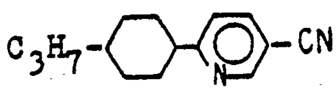 | 8 | |
| | 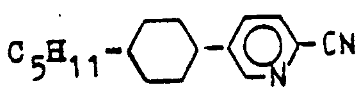 | 9 | |
| | 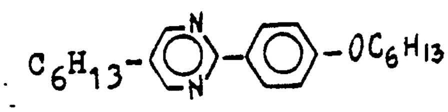 | 3 | |
| | 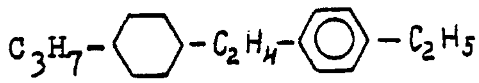 | 25 | |
| | 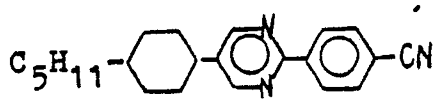 | 8 | |
| | 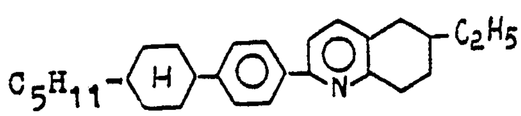 (IV h) | 5 | |
| | 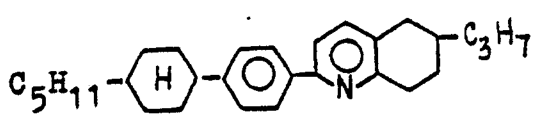 (IV b) | 4 | |
| | 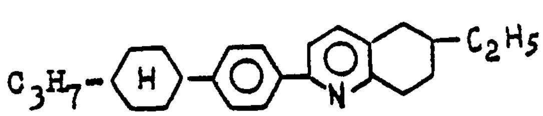 (IV g) | 4 | |

Fortsetzung der Tabelle 7

| 1 | 2 | 3 | 4 |
|---|---|---|---|
| | 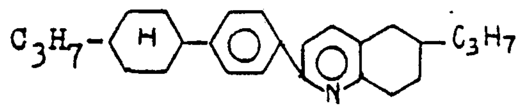 (IV c) | 5 | |
| | 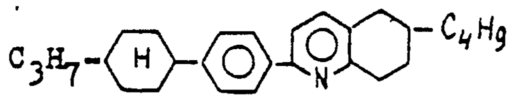 (IV d) | 5 | |
| | 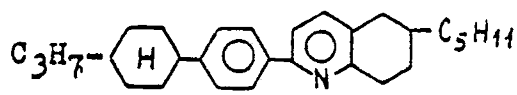 (IV a) | 4 | |
| | 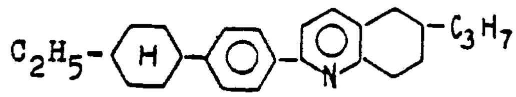 (IV e) | 5 | |
| | 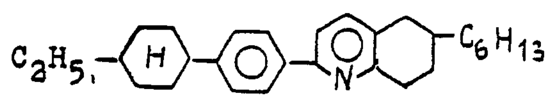 (IV f) | 4 | |
| | 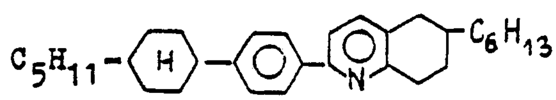 (IV i) | 4 | |
| 29. | 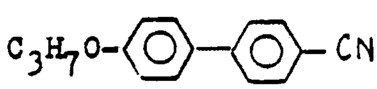 | 8 | -4+119,3° |
| | 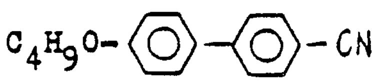 | 12 | |
| | 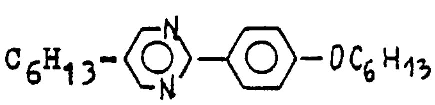 | 15 | |
| | 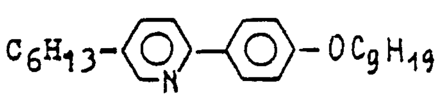 | 11 | |
| | 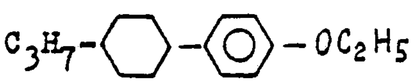 | 5 | |
| | 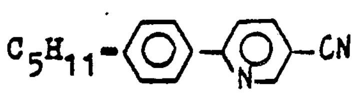 | 4 | |
| | 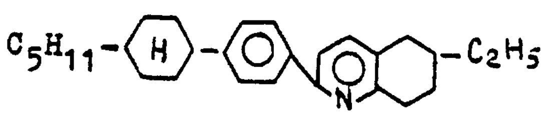 (IV h) | 6 | |

Fortsetzung der Tabelle 7

| 1 | 2 | 3 | 4 |
|---|---|---|---|
| | 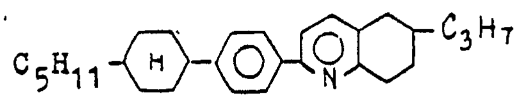 (IV b) | 6 | |
| | 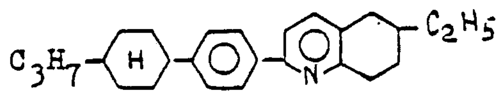 (IV g) | 6 | |
| | 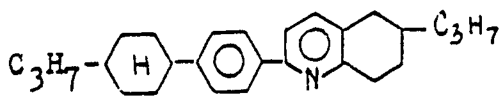 (IV c) | 9 | |
| | 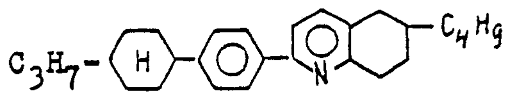 (IV d) | 6 | |
| | 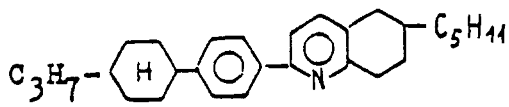 (IV a) | 6 | |
| | 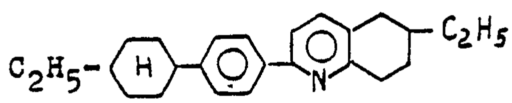 (IV j) | 6 | |
| 30. | 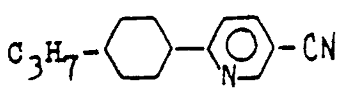 | 13 | -18+63,8 |
| | 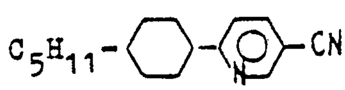 | 17 | |
| | 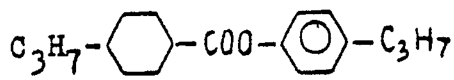 | 10 | |
| | 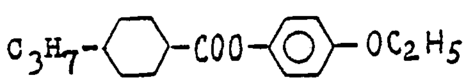 | 15 | |
| | 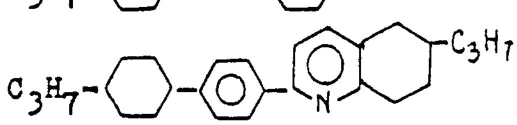 (IV c) | 3 | |
| | 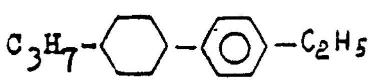 | 25 | |
| | 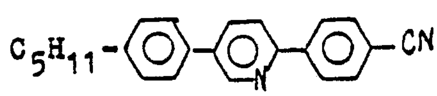 | 10 | |
| | 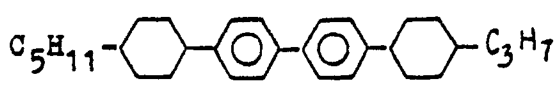 | 7 | |

Fortsetzung der Tabelle 7

| 1 | 2 | 3 | 4 |
|---|---|---|---|
| 31. | 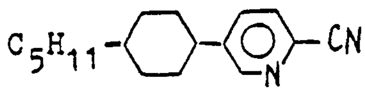 | 13 | -20+60,6 |
| | 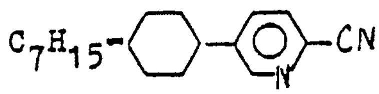 | 17 | |
| | 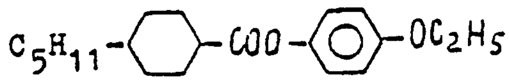 | 10 | |
| | 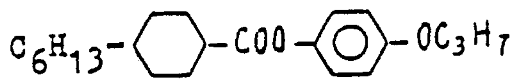 | 10 | |
| | 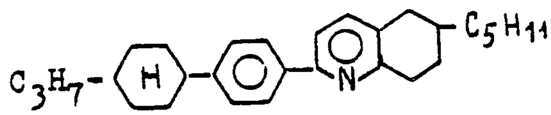 (IVa) | 1 | |
| | 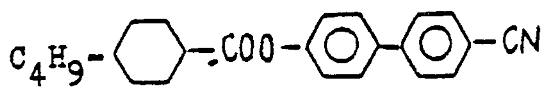 | 10 | |
| | 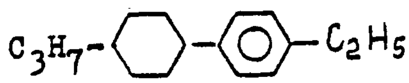 | 25 | |
| | 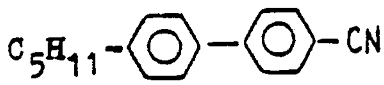 | 5 | |
| | 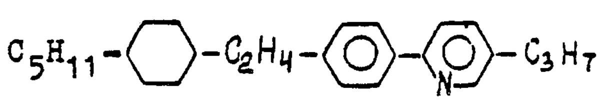 | 9 | |
| 32. | 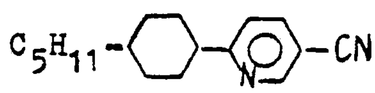 | 10 | -20+116,8 |
| | 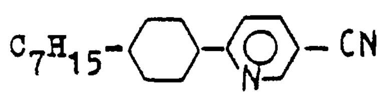 | 14 | |
| | 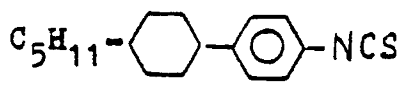 | 3 | |
| | 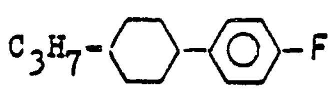 | 8 | |
| | 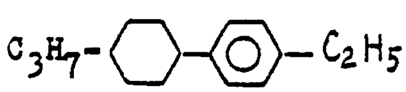 | 25 | |

Fortsetzung der Tabelle 7

| 1 | 2 | 3 | 4 |
|---|---|---|---|
| 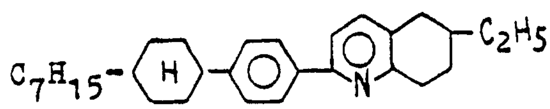 | (IVk) | 5 | |
| 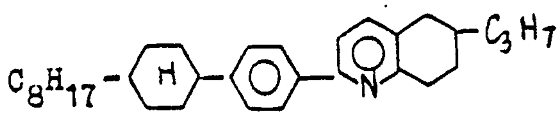 | (IVl) | 4 | |
| 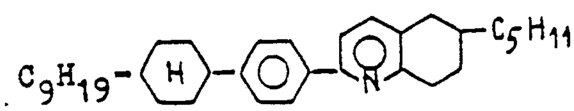 | (IVm) | 4 | |
| 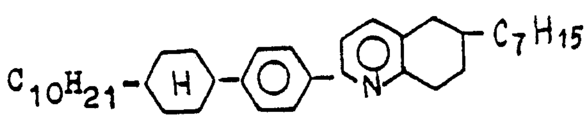 | (IVn) | 5 | |
| 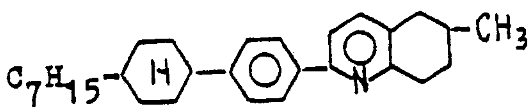 | (IVo) | 5 | |
| 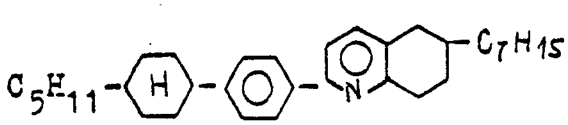 | (IVp) | 4 | |
| 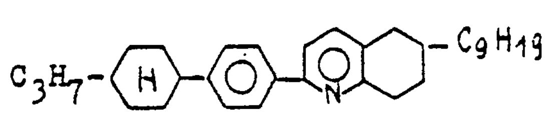 | (IVq) | 5 | |
| 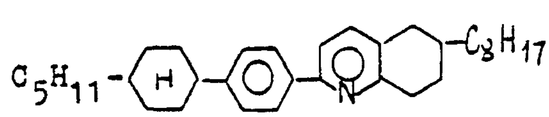 | (IVr) | 4 | |
| 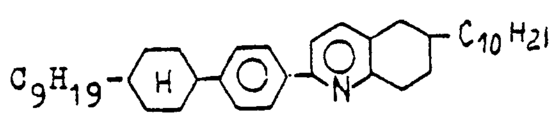 | (IVs) | 4 | |

Die in Tabelle 7 dargestellten Beispiele für flüssigkristalline Materialien (FKM) zeigen, daß die Verwendung der neuen Verbindungen der Formel IV als Komponenten von flüssigkristallinen Materialien den letztgenannten hohe Obergrenzen von Bereichen der nematischen Phase und breite Bereiche der nematischen Phase sichert, durch die die flüssigkristallinen Materialien für elektrooptische Anzeigeelemente von breiter Zweckbestimmung verwendet werden können.

Beispiel 33

Herstellung von 6-Alkyl-2- [4-(trans-4-alkyl-3-methylcyclohexyl)phenyl ] -5,6,7,8-tetrahydrochinolinen (V)

Man erhält analog zu II durch Umsetzung von 4-Alkylcyclohexanonen mit 1-(3-Dimethylaminopropionyl)-4-(4-alkyl-3-methyl-cyclohexyl)-benzolhydrochlorid und anschliepende Kondensation des 1,5-Dicarbonylderivates mit Hydroxylammoniumchlorid in einer Ausbeute von 17 bis 20 %. So hat 6-Propyl-2- [4-(trans-4-amyl-3-methylcyclohexyl)phenyl ] -5, 6,7,8-tetrahydrochinolin $T_{C-N}$ = 59°C, $T_{N-I}$ = 155 °C.

| | | | |
|---|---|---|---|
| Gefunden in %: | C 86,30; | H 10,41; | $C_{30}H_{43}N$ |
| Berechnet in %: | C 86,27; | H 10,38. | |

Beispiel 34

Herstellung von 2-Alkyl-6-(trans-4-alkylcyclohexyl)-5,6,7,8-tetrahydrochinolinen (VII)

34.1 2-Thienyl-6-(trans-4-amylcyclohexyl)-5,6,7,8-tetrahydrochinolin

Man erhält analog zu II durch Umsetzung von 4- (trans-4-Amylcyclohexyl)-cyclohexanon mit 2-(3-Dimethylaminopropionyl)-thiophenhydrochlorid und anschließender Kondensation der 1,5-Dicarbonylverbindung mit Hydroxylammoniumchlorid in einer Ausbeute von 15 %. $T_{Schmelz}$ = 149 °C (aus Alkohol).

34.2. 2-Butyl-6-(trans-4-amyl y lohexyl)-5,6,7,8-tetrahydrochinolin (VII)

Ein Gemisch aus 0,01 Mol 2-Thienyl-6-(trans-4-amyl-y lohexyl)-5,6,7,8-tetrahydrochinolin, 1 g Ni/Re und 30 ml Ethanol wird unter Sieden erhitzt, bis die vollständige Entschwefelung erfolgt. Man filtriert das Reaktionsgut, destilliert den Alkohol ab und reinigt den Rückstand durch Säulenchromatografie über $Al_2O_3$ mit Aktivitätsgrad II (Elutionsmittel Hexan:Benzol im Verhältnis von 1:1). $T_{Schmelz}$ = 33 °C, $T_{I-N}$ = 20 °C (aus Alkohol).

| | | | |
|---|---|---|---|
| Gefunden in %: | C 84,27; | H 11,60; | $C_{24}H_{39}N$. |
| Berechnet in %: | C 84,39; | H 11,51. | |

Beispiel 35

Herstellung von 6-Alkyl-1-(5-alkyl-thien-2-yl)-5,6, 7,8-tetrahydrochinolinen (VIII)

6-Amyl-2-(5-propyl-thien-2-yl)-5,6,7,8-tetrahydrochinolin (VIII)

Man erhält analog zu II durch Umsetzung von 4-Alkylcyclohexanon mit 2-(3-Dimethylaminopropionyl)-5-propylthiophenhydrochlorid und anschließender Kondensation des erhaltenen 1,5-Dicarbonylderivates mit Hydroxylammoniumchlorid in einer Ausbeute von 17 %.

$T_{Schmelz}$ = 34,4 °C (Aus Alkohol)

| | | | |
|---|---|---|---|
| Gefunden in %: | C 75,68; | H 8,21; | $C_{18}H_{23}NS$ |
| Berechnet in %: | C 75,74; | H 8,12 | |

Beispiel 36

Herstellung von 6-Alkyl-2-(4-alkoxy-3-fluorphenyl)-5,6,7,8-tetrahydrochinolinen (IX)

6-Hyptyl-2-(4-decyloxy-3-fluorphenyl)-5,6,7,8-tetrahydrochinolin

Man erhält analog zu Verbindung II durch Umsetzung von 4-Heptylcyclohexanon mit 1-(3-Dimethylaminopropionyl)-3-fluor-4-decyloxybenzolhydrochlorid und anschließender Kondensation des 1,5-Dicarbonylderivates mit Hydroxylammoniumchlorid in einer Ausbeute von 31 % (aus Alkohol).

$T_{C\text{-}S_A}$ = 49 °C, $T_{S_A\text{-}I}$ = 80 °C

| | | | |
|---|---|---|---|
| Gefunden in %: | C 79,81; | H 10,00; | $C_{32}H_{48}NFO$ |
| Berechnet in %: | C 79,78; | H 10,04 | |

Beispiel 37

Herstellung von 6-Alkyl-2-(4-alkoxy-2-fluorphenyl)-5,6,7,8-trahydrochinolinen (X)

6-Amyl-2-(4-methoxy-2-fluorphenyl)-5,6,7,8-tetrahydrochinolin (X)

Man erhält analog zur Verbindung II durch Umsetzung von 4-Amylcyclohexanon mit 1-(3-Dimethylaminopropionyl)-2-fluor-4-methoxybenzolhydrochlorid und anschließender Kondensation des erhaltenen 1,5-Dicarbonylderivates mit Hydroxylammoniumhydrochlorid in einer Ausbeute von 11 %. $T_C$ = 65 °C, $T_{I\text{-}N}$ = 55 °C (aus Alkohol).

| | | | |
|---|---|---|---|
| Gefunden in %: | C 76,94; | H 8,07; | $C_{21}H_{26}NFO$ |
| Berechnet in %: | C 77,03; | H 8,00 | |

Beispiel 38

Herstellung von 6-Alkyl-2-(4-fluorphenyl)-5,6,7,8-tetrahydrochinolin (XI)

6-Heptyl-2-(4-fluorphenyl)-5,6,7,8-tetrahydrochinolin

Man erhält analog zur Verbindung II durch Umsetzung von 4-Heptylcyclohexanon mit 1-(3-Dimethylaminopropionyl)-4-fluorbenzolhydrochlorid und anschließender Kondensation des 1,5-Dicarbonylderivates mit Hydroxylammoniumchlorid in einer Ausbeute von 30 %. $T_C$ = 63 °C, $T_{I\text{-}N}$ = 50 °C (aus Alkohol).

| | | | |
|---|---|---|---|
| Gefunden in %: | C 81,24; | H 8,55; | $C_{22}H_{28}NF$ |
| Berechnet in %: | C 81,19; | H 8,67 | |

Beispiel 39

Herstellung von 6-Alkyl-2-(4-isothiocyanophenyl)-5,6,7,8-tetrahydrochinolin (XII)

39.1. 6-Pentyl-2-(4-nitrophenyl)-5,6,7,8-tetrahydrochinolin

Man erhält analog zur Verbindung II durch Umsetzung von 4-Pentylcyclohexanon mit 1-(3-Dimethylaminopropionyl)-4-nitrobenzolhydrochlorid und anschließender Kondensation des 1,5-Dicarbonylderivates mit Hydroxylammoniumchlorid in einer Ausbeute von 15 % und mit $T_{Schmelz}$ = 87 bis 89 (aus Alkohol).

| | | | |
|---|---|---|---|
| Gefunden in %: | C 73,98, | H 7,51; | $C_{20}H_{24}N_2O_2$ |
| Berechnet in %: | C 74,05; | H 7,46 | |

39.2. 6-Pentyl 2-(4-aminophenyl)-5,6,7,8-tetrahydrochinolin

Ein Gemisch aus 35 ml Ethanol, 0,005 Mol 6-Pentyl-2-(4-nitrophenyl)-5,6,7,8-tetrahydrochinolin, 1 g Ni/Re, 2 ml Hydrazinhydrat und 0,1 g NaOH wird in einem mit Rückflußkühler versehenen Kolben gesiedet, bis die Ausgangsverbindung (39.1) auf dem Chromatogramm verschwindet. Man filtriert das Reaktionsgut, destilliert den Alkohol ab und kristallisiert den Rückstand aus Hexan um. Man isoliert das Aminoderivat in einer Ausbeute von 72 %.
$T_{Schmelz}$ = 82 bis 83,5 °C.

| | | | |
|---|---|---|---|
| Gefinden in %: | C 81,66; | H 9,02; | $C_{20}H_{26}N_2$ |
| Berechnet in %: | C 81,59; | H 8,90 | |

39.3 6-Pentyl-2-(4-isothiocyanophenyl)-5,6,7,8-tetrahydrochinolin (XII)

Man löst 0,01 Mol 6-Pentyl-2-(4-aminophenyl)-5,6,7,8-tetrahydrochinolin (39.2) in 5 ml absolutem Benzol auf, setzt 0,01 Mol $CS_2$, 1,5 ml $Et_3N$ hinzu und läßt 1 Woche stehen. Man verdünnt das Reaktionsgut mit Absolut ether und filtriert den ausgefallenen Niederschlag ab. Der Niederschlag wird in einen Dreihalskolben übertragen, der mit Rührwerk, Thermometer und Tropftrichter mit Gegenstrom versehen ist. Man bringt in den Kolben 20 ml absolutes Chloroform ein und tropft bei 0 °C 1,5 ml $Et_3N$ und dann 1,5 ml Ethylchlorformiat zu. Das Reaktionsgut wird innerhalb von 10 Minuten bei 0 °C und 1 Stunde bei Raumtemperatur umgerührt, die Chloroformschicht mit 1%igem HCl und Wasser gewaschen und nach dem Trocknen über $Na_2SO_4$ durch $SiO_2$ bei L 5/40 (h = 2 cm) filtriert. Die Mutterlauge wird im Vakuum eingedampft. Man kristallisiert den Rückst aus Hexan um. Man isoliert 6-Phenyl-2-(4-isothiocyanophenyl)-5,6,7,8-tetrahydrochinolin in 50%iger Ausbeute mit folgenden Übergangstemperaturen: $T_{C-S}$ = 92,0 °C: $T_{S-N}$ = 104,0 °C: $T_{N-I}$ = 115,3 °C.

| | | | |
|---|---|---|---|
| Gefunden in %: | C 75,09; | H 7,16; | $C_{21}H_{24}N_2S$. |
| Berechnet in %: | C 74,96; | H 7,19. | |

Beispiel 40

Herstellung von 6-Alkyl-2-(4-cyanophenyl)-5,6,7,8-tetrahydrochinolinen (XIII)

40.1. 6-Heptyl-2-(4-bromphenyl)-5,6,7,8-tetrahydrochinolin

Man erhält analog zur Verbindung II aus 4-Heptylcyclohexanon und 1-(3-Dimethylaminopropionyl)-4-brombenzolhydrochlorid und anschließender Kondensation des 1,5-Dicarbonylderivates mit Hydroxylammoniumchlorid in 23%iger Ausbeute. Analog werden Bromderivate hergestellt, deren Eigenschaften, Bruttoformel und Elementaranalysenbefund die Tabelle 8 angibt.

Tabelle 8

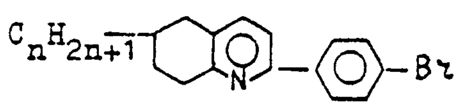

| Verbindung | | n | Ausbeute in % | $T_{Schmelz}$ in °C | Gefunden in % C | Gefunden in % H | Bruttoformel | Berechnet in % C | Berechnet in % H |
|---|---|---|---|---|---|---|---|---|---|
| 40.1 | a | 3 | 35 | 116 | 65,38 | 6,04 | $C_{18}H_{20}BrN$ | 65,46 | 6,18 |
| | b | 4 | 30 | 94 | 66,31 | 6,26 | $C_{19}H_{22}BrN$ | 66,28 | 6,44 |
| | c | 5 | 26 | 108 | 67,18 | 7,01 | $C_{20}H_{24}BrN$ | 67,04 | 6,75 |
| | d | 6 | 18 | 87 | 67,59 | 7,03 | $C_{21}H_{26}BrN$ | 67,74 | 7,04 |
| | e | 7 | 23 | 95 | 68,36 | 7,21 | $C_{22}H_{28}BrN$ | 68,39 | 7,30 |
| | f | 8 | 30 | 88 | 69,70 | 7,46 | $C_{23}H_{30}BrN$ | 69,72 | 7,55 |

40.2. 6-Heptyl-2-(4-cyanophenyl)-5,6,7,8-tetrahydrochinolin (XIIIe)

Ein Gemisch aus 0,01 Mol 6-Heptyl-2-(4-bromphenyl)-5,6,7,8-tetrahydrochinolin, 0,006 Mol $Cu_2CN_2$ und 5 ml N-Methylpyrrolidon wird unter Umrühren innerhalb von 4 Stunden gesiedet, dann abgekühlt, mit 20 ml wäßriger $NH_4OH$-Lösung verdünnt und bei Raumtemperatur innerhalb von 3 Stunden vermischt. Man filtriert den Niederschlag ab, wäscht mit Wasser und löst in Benzol auf. Die Benzollösung wird über $Na_2SO_4$ getrocknet und durch $SiO_2$- Schicht bei h = 2 cm, L 5/40 filtriert. Man destilliert Benzol ab, kristallisiert den Rückstand aus Ethanol um und isoliert 1,88 g (57%). Analog sind andere Verbindungen XIII a-d hergestellt, deren Eigenschaften, Bruttoformeln und Elementaranalysenbefund die Tabelle 9 angibt.

Tabelle 9

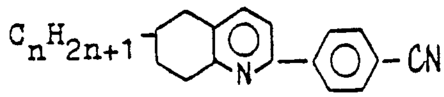

| Verbindung | | n | Ausbeute in % | $T_{C-N}$ in °C | $T_{N-I}$ in °C | Gefunden in % C | Gefunden in % H | Bruttoformel | Berechnet in % C | Berechnet in % H |
|---|---|---|---|---|---|---|---|---|---|---|
| XIII | a | 3 | 64,0 | 102,0 | 97,6$^x$ | 82,40 | 7,11 | $C_{19}H_{20}N_2$ | 82,57 | 7,29 |
| | b | 4 | 59,0 | 84,0 | 89 | 82,76 | 7,83 | $C_{20}H_{22}N_2$ | 82,72 | 7,64 |
| | c | 5 | 61,0 | 96,2 | 98,0 | 82,79 | 8,01 | $C_{21}H_{24}N_2$ | 82,85 | 7,95 |
| | d | 6 | 67,0 | 69,0 | 97,3 | 82,69 | 8,34 | $C_{22}H_{26}N_2$ | 82,97 | 8,23 |
| | e | 7 | 57,0 | 67,0 | 98,1 | 83,08 | 8,56 | $C_{23}H_{28}N_2$ | 83,09 | 8,49 |
| | f | 8 | 64,0 | 65,9 | 93,8 | 83,11 | 8,67 | $C_{24}H_{30}N_2$ | 83,24 | 8,73 |

$^x$der monotrope Übergang

Die vergleichenden Werte von Phasenübergangstemperaturen und Bereichen der nematischen Phase $\Delta T_N$ für die bekannten 5-Alkyl-2-(4-cyanophenyl)-pyridine und neuen 6-Alkyl-2-(4-cyanophenyl)-5,6,7,8-tetrahydrochinoline (XIII) sind in der Tabelle 10 angegeben.

Die Tabelle 10 zeigt, daß die Klärpunkte $T_{N-I}$ der Derivate XIII a-f bedeutend höher als die der Pyridinanaloga liegen und daß der Bereich der nematischen Phase bedeutend breiter ist.

Tabelle 10

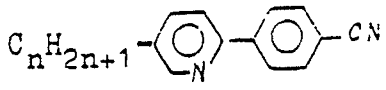 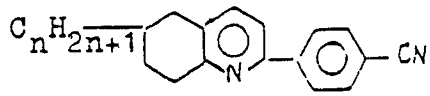 (XIII)

| | n | $T_{C-N}$, °C | $T_{N-I}$, °C | $\Delta T_N$, °C | | n | $T_{C-N}$, °C | $T_{N-I}$, °C | $\Delta T_N$, °C |
|---|---|---|---|---|---|---|---|---|---|
| a | 3 | 43,4 | 43,8 | 0,4 | a | 3 | 102,0 | 97,6 x | -4,4 |
| b | 4 | 32,3 | 26,5 x | -5,8 | b | 4 | 84,0 | 89,0 | 5,0 |
| c | 5 | 33,6 | 43,5 | 9,9 | c | 5 | 96,2 | 98,0 | 1,8 |
| d | 6 | 29,0 | 32,5 | 3,5 | d | 6 | 69,0 | 97,3 | 18,3 |
| e | 7 | 30,9 | 47,0 | 16,1 | e | 7 | 67,0 | 98,1 | 31,1 |
| f | 8 | 39,5 | 43,0 | 13,5 | f | 8 | 65,9 | 93,8 | 17,9 |

x der monotrope Ubergang

Tabelle 11 gibt die Zusammensetzungen und Eigenschaften von flüssigkristallinen Materialien an, welche neue Verbindungen der allgemeinen Formel XIII enthalten.

Tabelle 11

| Nr. | Zusammensetzung | | Masse% | Bereich der nematischen Phase in °C |
|---|---|---|---|---|
| 1 | 2 | | 3 | 4 |
| 41. | 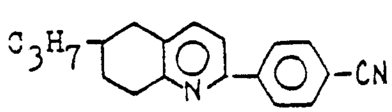 | (XIII a) | 20 | -7+92,5 |
| | 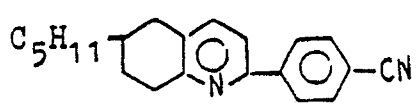 | (XIII c) | 30 | |
| | 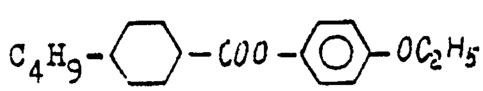 | | 25 | |

Fortsetzung der Tabelle 11

| 1 | 2 | | 3 | 4 |
|---|---|---|---|---|
| | 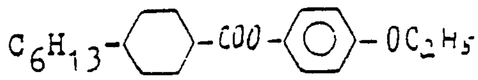 | | 15 | |
| | 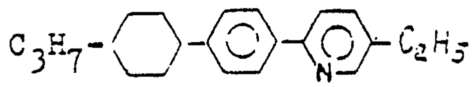 | | 10 | |
| 42. | 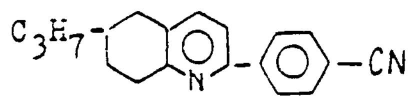 | (XIII a) | 10 | -8+90,1 |
| | 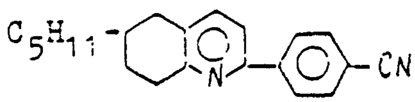 | (XIII c) | 15 | |
| | 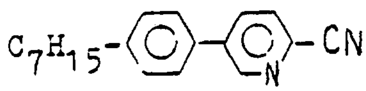 | | 20 | |
| | 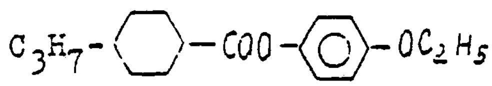 | | 15 | |
| | 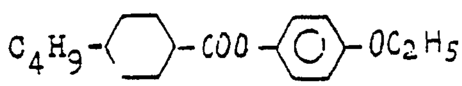 | | 10 | |
| | 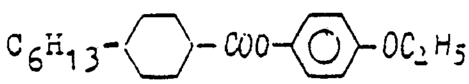 | | 15 | |
| | 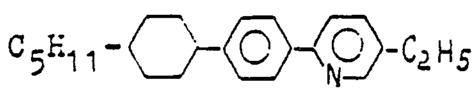 | | 5 | |
| | 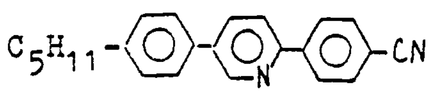 | | 10 | |
| 43 | 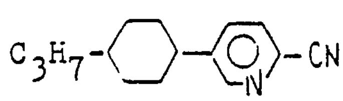 | | 17 | -7+68,2 |
| | 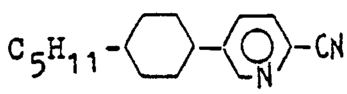 | | 20 | |
| | 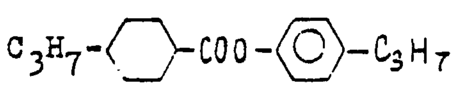 | | 10 | |
| | 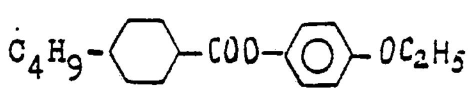 | | 10 | |

Fortsetzung der Tabelle 11

| 1 | 2 | 3 | 4 |
|---|---|---|---|
| | 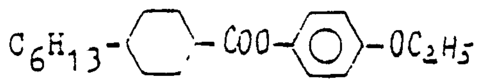 | 10 | |
| | 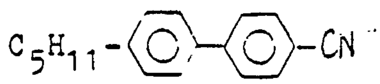 | 10 | |
| | 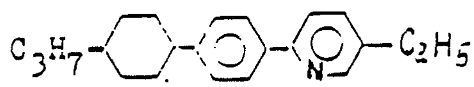 | 3 | |
| | 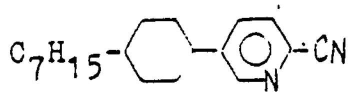 | 10 | |
| | 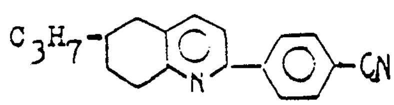 (XIII a) | 10 | |
| 44. | 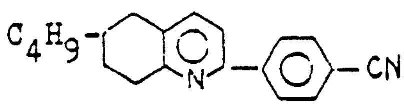 (XIII b) | 5 | -22+86,1 |
| | 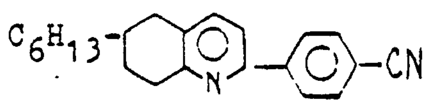 (XIII d) | 5 | |
| | 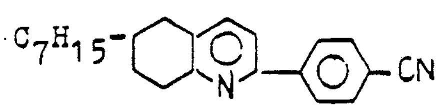 (XIII e) | 5 | |
| | 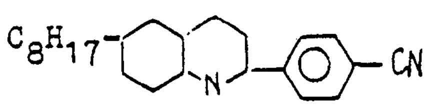 (XIII f) | 5 | |
| | 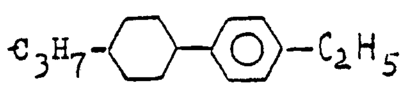 | 20 | |
| | 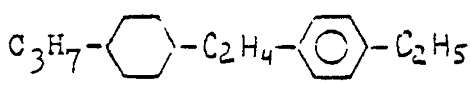 | 10 | |
| | 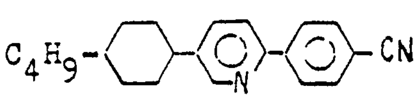 | 10 | |
| | 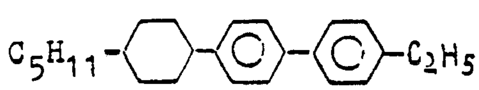 | 8 | |
| | 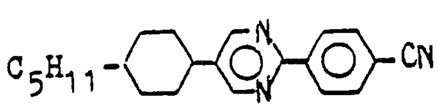 | 7 | |

Fortsetzung der Tabelle 11

| 1 | 2 | | 3 | 4 |
|---|---|---|---|---|
| | 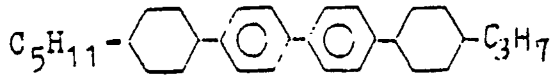 | | 5 | |
| | 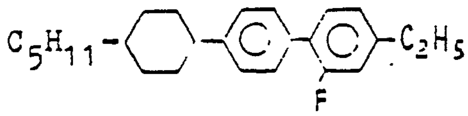 | | 8 | |
| | 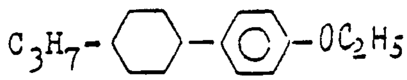 | | 12 | |
| 45. | 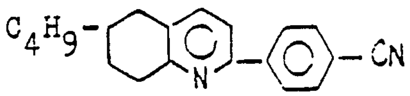 | (XIII b) | 3 | -27+68,2 |
| | 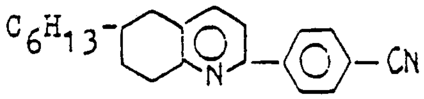 | (XIII d) | 2 | |
| | 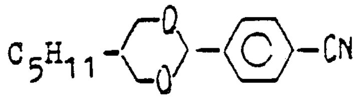 | | 10 | |
| | 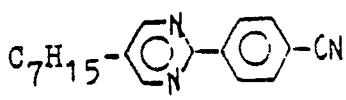 | | 15 | |
| | 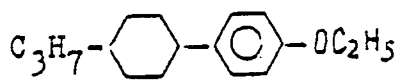 | | 10 | |
| | 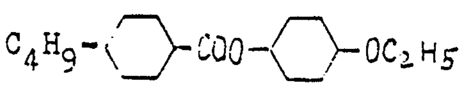 | | 10 | |
| | 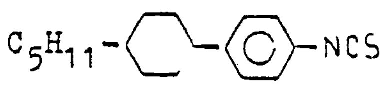 | | 10 | |
| | 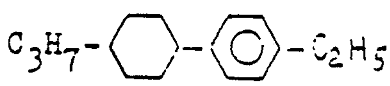 | | 20 | |
| | 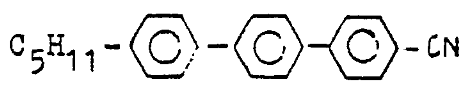 | | 5 | |
| | 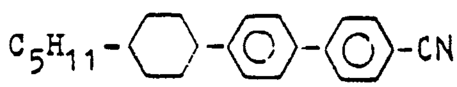 | | 5 | |
| | 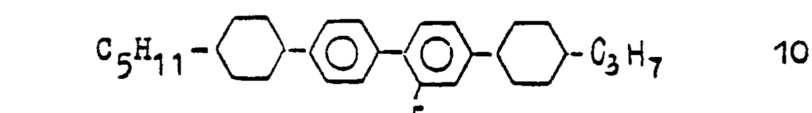 | | 10 | |

Fortsetzung der Tabelle 11

| 1 | 2 | 3 | 4 |
|---|---|---|---|
| 46. | 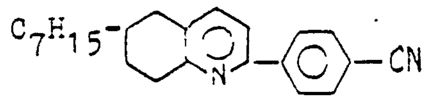 (XIII e) | 3 | -32+65,9 |
| | 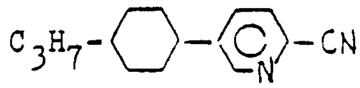 | 7 | |
| | 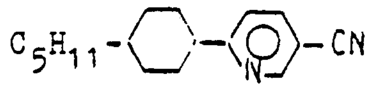 | 8 | |
| | 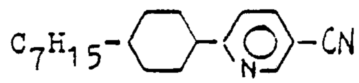 | 2 | |
| | 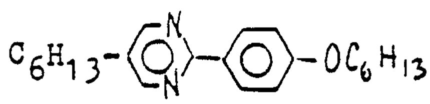 | 5 | |
| | 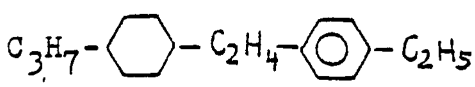 | 20 | |
| | 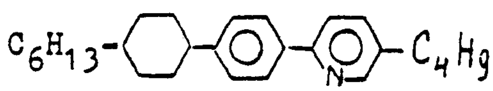 | 8 | |
| | 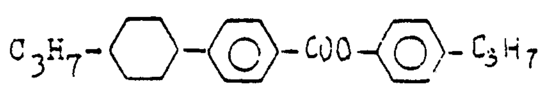 | 3 | |
| | 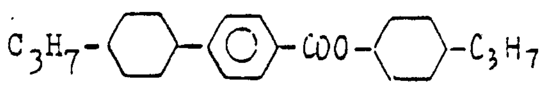 | 3 | |
| | 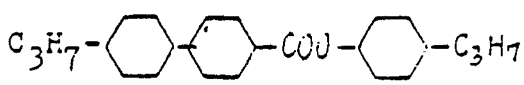 | 12 | |
| | 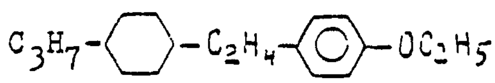 | 10 | |
| | 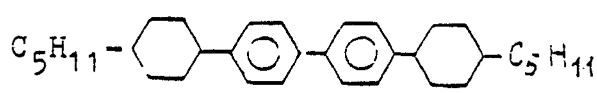 | 9 | |
| | 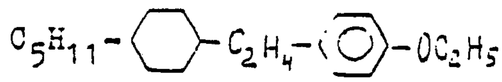 | 10 | |
| 47. | 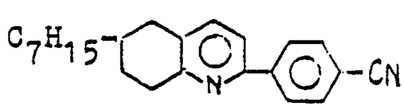 (XIII e) | 1 | -32+62,9 |
| | 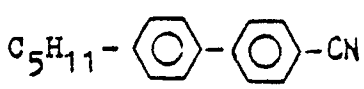 | 30 | |

Fortsetzung der Tabelle 11

| 1 | 2 | 3 | 4 |
|---|---|---|---|
| | 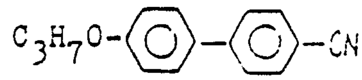 | 7 | |
| | 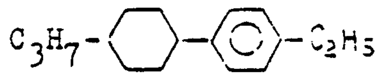 | 15 | |
| | 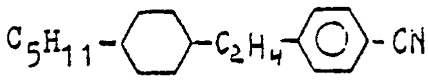 | 10 | |
| | 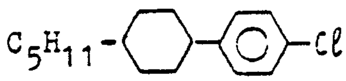 | 5 | |
| | 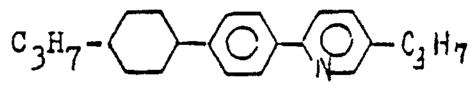 | 8 | |
| | 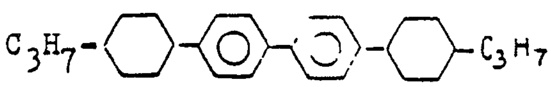 | 10 | |
| | 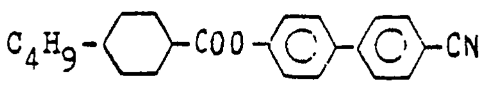 | 10 | |
| | 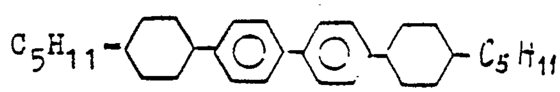 | 4 | |
| 48. | 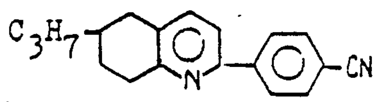 (XIII a) | 10 | -8+92,2 |
| | 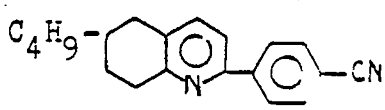 (XIII b) | 10 | |
| | 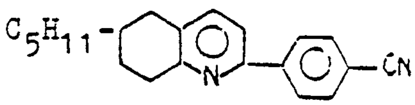 (XIII c) | 10 | |
| | 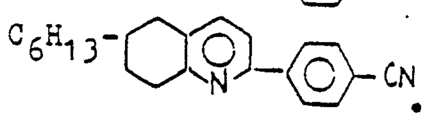 (XIII d) | 10 | |
| | 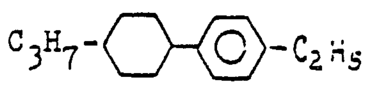 | 30 | |
| | 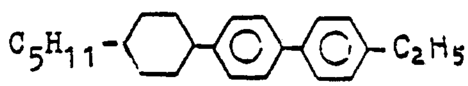 | 10 | |
| | 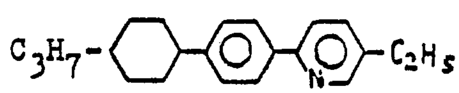 | 10 | |

Fortsetzung der Tabelle 11

| 1 | 2 | 3 | 4 |
|---|---|---|---|
| | 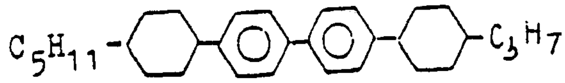 | 10 | |
| 49. | 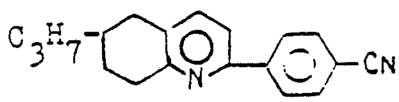 (XIII a) | 8 | -6+101,2 |
| | 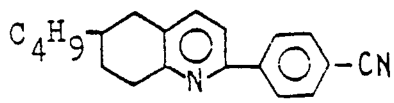 (XIII b) | 9 | |
| | 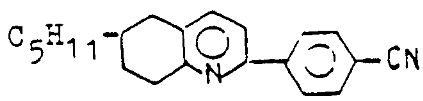 (XIII c) | 10 | |
| | 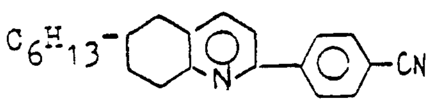 (XIII d) | 8 | |
| | 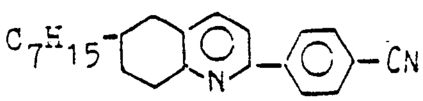 (XIII e) | 8 | |
| | 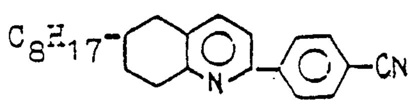 (XIII f) | 7 | |
| | 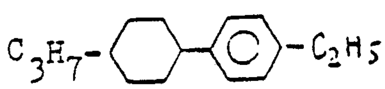 | 20 | |
| | 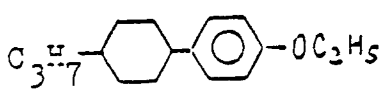 | 5 | |
| | 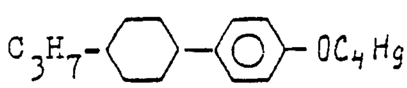 | 5 | |
| | 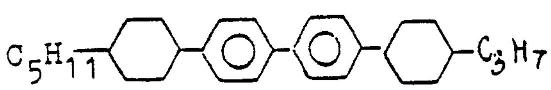 | 10 | |
| | 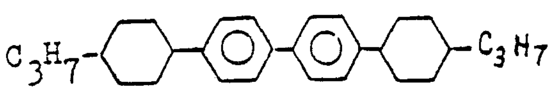 | 10 | |
| 50. | 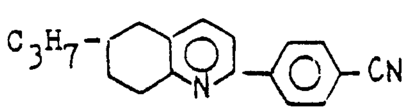 (XIII a) | 11 | -3+106,7 |
| | 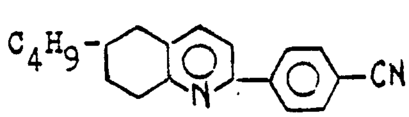 (XIII b) | 11 | |

Fortsetzung der Tabelle 11

| 1 | 2 | 3 | 4 |
|---|---|---|---|
| 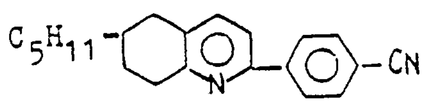 | (XIII c) | 11 | |
| 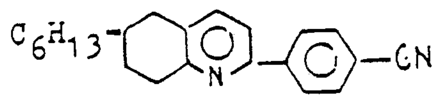 | (XIII d) | 11 | |
| 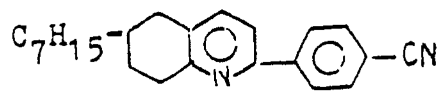 | (XIII e) | 7 | |
| 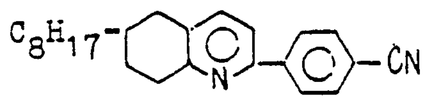 | (XIII f) | 4 | |
| 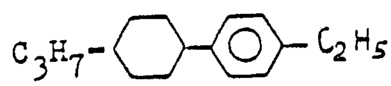 | | 20 | |
| 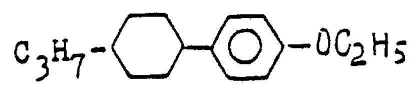 | | 5 | |
| 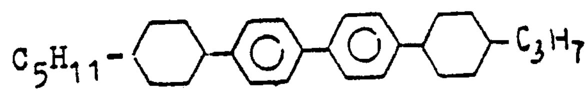 | | 10 | |
| 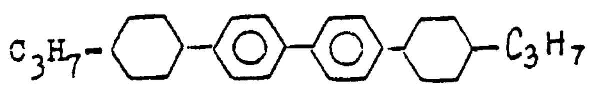 | | 10 | |

Wie aus Tabelle 1 zu ersehen ist, sichert die Verwendung der neuen Verbindungen der Formel XIII als Komponenten von flüssigkristallinen Materialien den letztgenannten die hohen Klärpunkte, die für den Einsatz derselben in elektrooptischen Anzeigeelementen von Mehrzweckbestimmung erforderlich sind.

Beispiel 51

Herstellung von 6-Alkyl-2-(2-hydroxy-4-alkyloxy)-5,6,7,8-tetrahydrochinolin (XIV)

6-Heptyl-2-(2-hydroxy-4-nonyloxy)-5,6,7,8-tetrahydrochinolin

Man erhält analog zur Verbindung II durch Umsetzung von 4-Heptylcyclohexanon mit 1-(3-Dimethylaminopropionyl)- 2-hydroxy-4-nonyloxybenzolhydrochlorid und anschließender Kondensation der 1,5-Dicarbonylverbindung mit Hydroxylammoniumchlorid in einer Ausbeute von 15 % mit $T_{C\text{-}S_B}$ = = 73 °C, $T_{S_B\cdot S_A}$ = 89 °C, $T_{S_{A\text{-}I}}$ = 165 °C (aus Alkohol).

| | | | |
|---|---|---|---|
| Gefunden in %: | C 80,18; | H 10,01; | $C_{31}H_{47}NO_2$. |
| Berechnet in %: | C 79,95; | H 10,17. | |

In der Tabelle 12 sind Zusammensetzung und Eigenschaften des flüssigkristallinen Materials dargestellt, welches die neue Verbindung der Formel XIV enthält.

Tabelle 12

| Nr. | Zusammensetzung | Masse% | Bereich der nematischen Phase in °C |
|---|---|---|---|
| 52. | 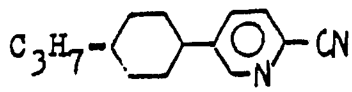 | 20 | -15÷63,8 |
| | 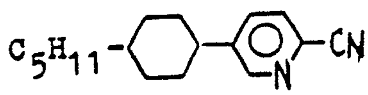 | 32 | |
| | 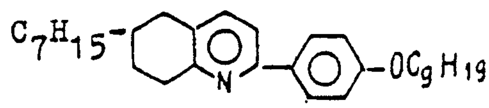 (XIV) | 5 | |
| | 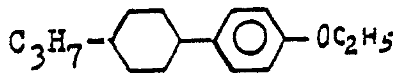 | 16 | |
| | 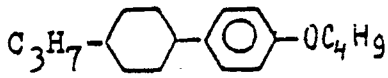 | 12 | |
| | 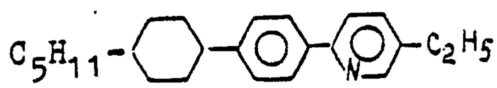 | 10 | |
| | 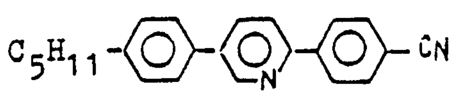 | 5 | |

Beispiel 53

Herstellung von 6-Alkyl-2-(2-hydroxy-4-alkyl)-5,6,7,8-tetrahydrochinolin (XV)

6-Pentyl-2-(2-hydroxy-4-methyl)-5,6,7,8-tetrahydrochinolin

Man erhält analog zur Verbindung II durch Umsetzung von 4-Pentylcyclohexanon mit 1-(3-Dimethylaminopropionyl)-2-hydroxy-4-methylbenzolhydrochlorid und anschließender Kondensation der 1,5-Dicarbonylverbindung mit Hydroxylammoniumchlorid in einer Ausbeute von 16%. $T_{C\text{-}S_B}$ = 88 °C, $T_{S_B\text{-}S_A}$ = 120,1 °C, $T_{S_A\text{-}I}$ = 136,2 °C

| Gefunden in %: | C 81,65; | H 8,81; | $C_{21}H_{27}NO$ |
|---|---|---|---|
| Berechnet in %: | C 81,51; | H 8,79 | |

Die Tabelle 13 zeigt die Zusammensetzung und die Eigenschaften des flüssigkristallinen Materials an, welches die neue Verbindung der Formel XV enthält.

Tabelle 13

| Nr. | Zusammensetzung | | Masse% | Bereich der nematischen Phase in °C |
|---|---|---|---|---|
| 54. | 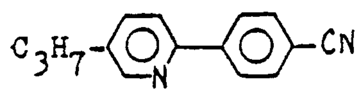 | | 20 | -17÷65,3 |
| | 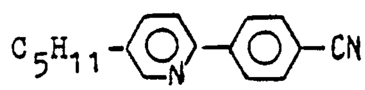 | | 30 | |
| | 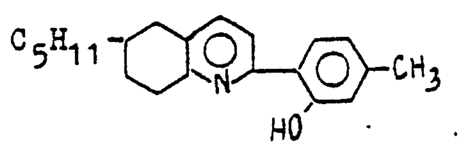 | (XV) | 5 | |
| | 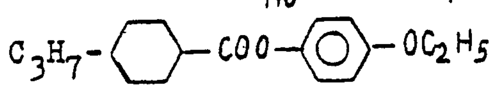 | | 17 | |
| | 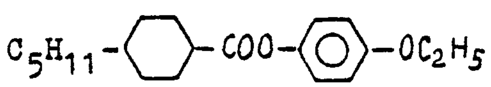 | | 18 | |
| | 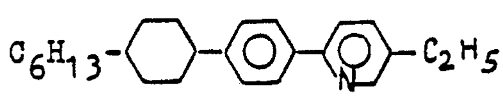 | | 10 | |

Beispiel 55

Herstellung von 6-Alkyl-2-(4-brom-2-hydroxyphenol)-5,6,7,8-tetrahydrochinolinen (XVI)

6-Heptyl-2-(4-brom-2-hydroxyphenyl)-5,6,7,8-tetrahydrochinolin

Man erhält analog zur Verbindung II durch Umsetzung von 4-Heptylcyclohexanon mit 1-(3-Dimethylaminopropionyl)-2-hydroxy-4-brombenzolhydrochlorid und anschließender. Kondensation der 1,5-Dicarbonylverbindung mit Hydroxylammoniumchlorid in 11%iger Ausbeute (aus Alkohol). Auf ähnliche Weise werden andere Verbindungen XVI (b, c) hergestellt.

Die Phasenübergangstemperaturen für die Verbindungen XVI a-c gibt die Tabelle 14 an.

Tabelle 14

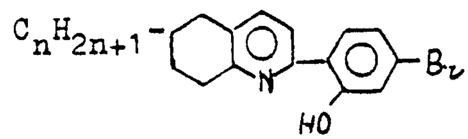

| XVI | n | Ausbeute in % | $T_{C-S}$ in °C | $T_{S-I}$ in °C | Gefunden in % C | Gefunden in % H | Bruttoformel | Berechnet in % C | Berechnet in % H |
|---|---|---|---|---|---|---|---|---|---|
| a | 7 | 11 | 94,0 | 143,0 | 65,51 | 7,12 | $C_{22}H_{28}BrNO$ | 65,67 | 7,01 |
| b | 3 | 10 | 133,0 | 128,4$^{x}$ | 65,71 | 6,05 | $C_{18}H_{20}BrNO$ | 65,46 | 6,10 |
| c | 5 | 15 | 108,0 | 141,0 | 64,20 | 6,17 | $C_{20}H_{24}BrNO$ | 64,17 | 6,46 |

Auf ähnliche Weise werden andere Derivate der Formel XVI hergestellt, wenn Hal J oder F bedeutet.

Beispiel 56

Herstellung von 6-Alkyl-2-(4-cyano-2-hydroxyphenyl)-5,6,7,8-tetrahydrochinolinen (XVII)

6-Heptyl-2-(4-cyano-2-hydroxyphenyl)-5,6,7,8-tetrahydrochinolin (XVII a)

Ein Gemisch aus 0,01 Mol Verbindung XVI, 0,012 Mol CuCN und 10 ml N-Methylpyrrolidon wird gesiedet, bis die Ausgangsverbindung XVI a verschwindet. Man kühlt die Reaktionsmasse ab, gießt auf 50 ml 25%iges Ammoniak aus, rührt innerhalb von 1 Stunde um und extrahiert mit Methylenchlorid. Die organische Schicht wird mit Wasser gewaschen, durch $SiO_2$ bei L 5/40 (h = 3 cm) filtriert und eingedampft. Der Rückstand wird aus Hexan und Alkohol umkristallisiert.

Auf ähnliche Weise werden andere Verbindungen XVI b-c hergestellt, deren Ausbeute, Phasenübergangstemperaturen in Tabelle 15 dargestellt sind.

Tabelle 15

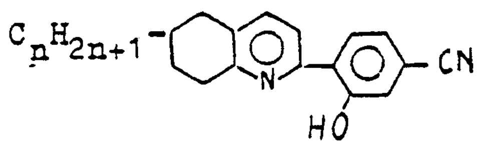

(XVII)

| XVII | n | Ausbeute in % | $T_{C-N}$ | $T_{N-I}$ | Gefunden in % C | Gefunden in % H | Bruttoformel | Berechnet in % C | Berechnet in % H |
|---|---|---|---|---|---|---|---|---|---|
| a | 7 | 77 | 102 | 139,5 | 79,30 | 8,11 | $C_{23}H_{28}N_2O$ | 79,27 | 8,10 |
| b | 3 | 81 | 135 | 145 | 78,11 | 6,75 | $C_{19}H_{20}N_2O$ | 78,05 | 6,89 |
| c | 5 | 79 | 109 | 141 | 78,90 | 7,61 | $C_{21}H_{24}N_2O$ | 78,72 | 7,55 |

Die Tabelle 16 gibt Phasenübergangstemperaturen der Verbindungen XIII und XVII vergleichsweise an.

Tabelle 16

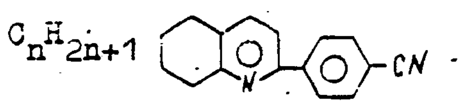

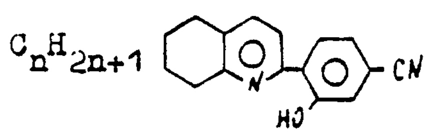

XVII

| XIII | n | $T_{C-N}$, °C | $T_{N-I}$, °C | $\Delta T_N$, °C | XVII | n | $T_{C-N}$, °C | $T_{N-I}$, °C | $\Delta T_N$, °C |
|---|---|---|---|---|---|---|---|---|---|
| a | 3 | 102,0 | 97,6 | -4,4 | b | 3 | 135,0 | 145,0 | 10,0 |
| c | 5 | 96,2 | 98,0 | 1,8 | c | 5 | 109,0 | 141,0 | 32,0 |
| e | 7 | 67,0 | 98,1 | 31,1 | a | 7 | 102,0 | 139,5 | 37,5 |

In der Tabelle 17 sind die Zusammensetzungen und Eigenschaften von flüssigkristallinen Materialien dargestellt, welche neue Verbindungen der Formel XVII a-c enthalten.

Tabelle 17

| Nr. | Zusammensetzung | Masse% | Bereich der nematischen Phase in °C |
|---|---|---|---|
| 1 | 2 | 3 | 4 |
| 57 | 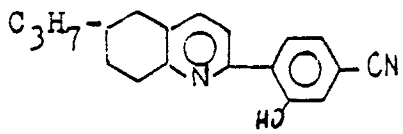 (XVII B) | 20 | -5÷115,1 |
| | 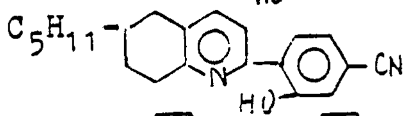 (XVII C) | 30 | |
| | 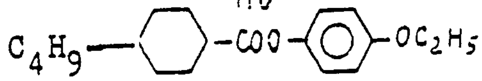 | 25 | |
| | 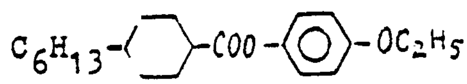 | 15 | |
| | 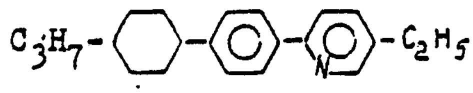 | 10 | |

Fortsetzung der Tabelle 17

| 1 | 2 | 3 | 4 |
|---|---|---|---|
| 58 | 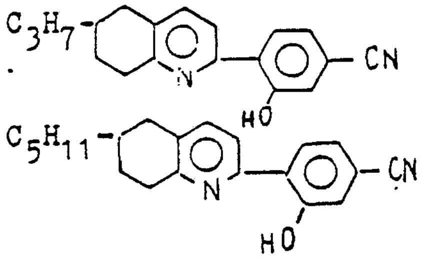 (XVII b) | 10 | -6+101,3 |
| | (XVII c) | 15 | |
| | 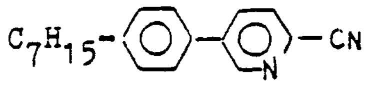 | 20 | |
| | 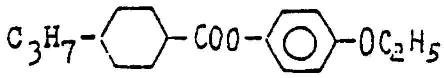 | 15 | |
| | 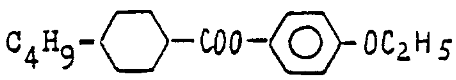 | 10 | |
| | 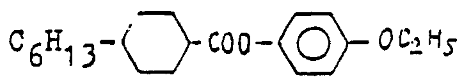 | 15 | |
| | 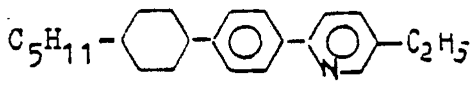 | 5 | |
| | 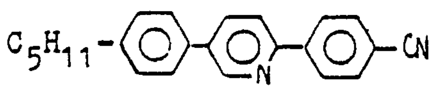 | 10 | |
| 59 | 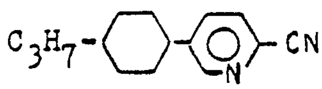 | 17 | -6+73,7 |
| | 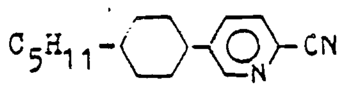 | 20 | |
| | 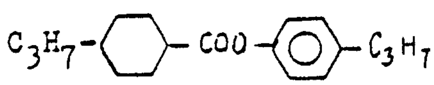 | 10 | |
| | 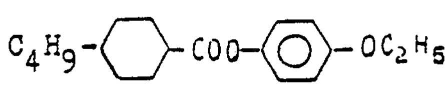 | 10 | |
| | 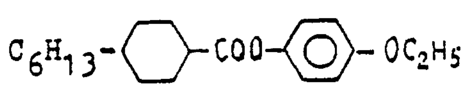 | 10 | |
| | 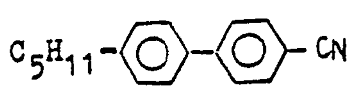 | 10 | |
| | 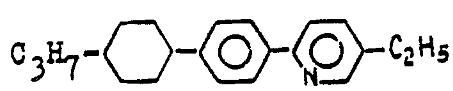 | 3 | |

Fortsetzung der Tabelle 17

| 1 | 2 | 3 | 4 |
|---|---|---|---|
| | 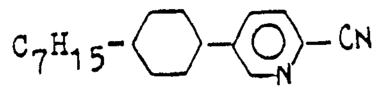 | 10 | |
| | 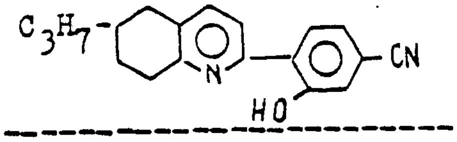 (XVII b) | 10 | |
| 60 | 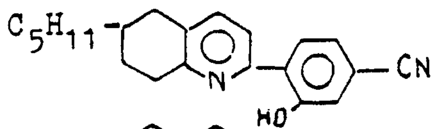 (XVII c) | 7 | -20+94,5 |
| | 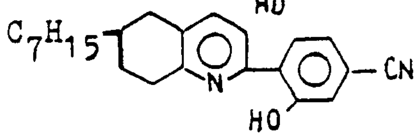 (XVII a) | 7 | |
| | 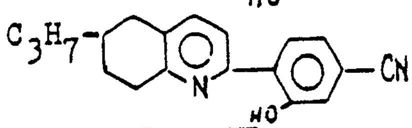 (XVII b) | 6 | |
| | 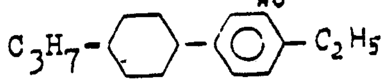 | 20 | |
| | 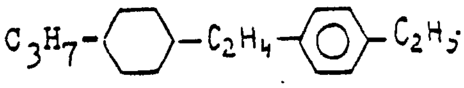 | 10 | |
| | 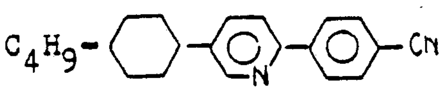 | 10 | |
| | 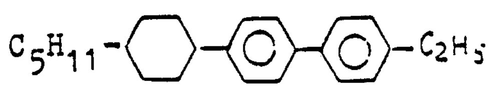 | 8 | |
| | 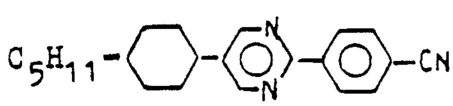 | 7 | |
| | 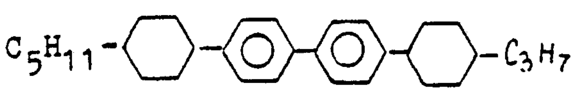 | 5 | |
| | 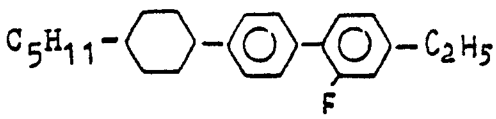 | 8 | |
| | 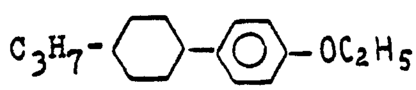 | 12 | |

Fortsetzung der Tabelle 17

| 1 | 2 | | 3 | 4 |
|---|---|---|---|---|
| 61 | 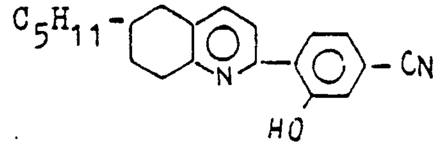 | (XVII c) | 3 | -26÷70,7 |
| | 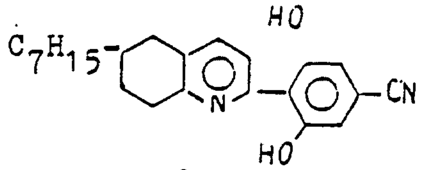 | (XVII a) | 2 | |
| | 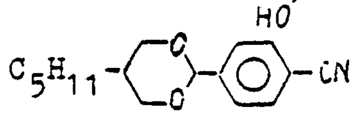 | | 10 | |
| | 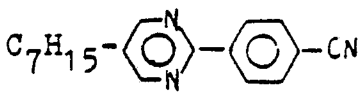 | | 15 | |
| | 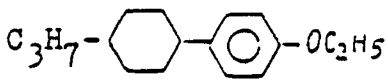 | | 10 | |
| | 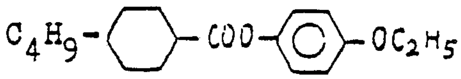 | | 10 | |
| | 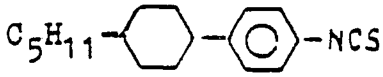 | | 10 | |
| | 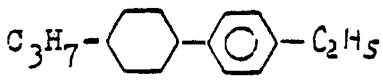 | | 20 | |
| | 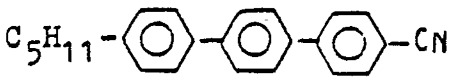 | | 5 | |
| | 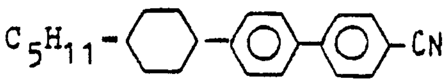 | | 5 | |
| | 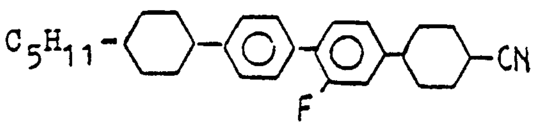 | | 10 | |
| 62 | 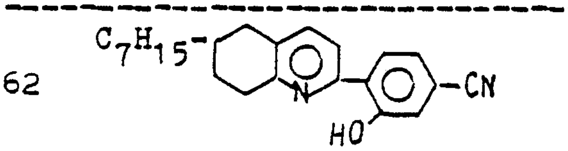 | (XVII a) | 3 | -32÷67,3 |
| | 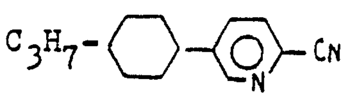 | | 7 | |
| | 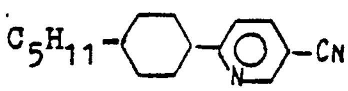 | | 8 | |
| | 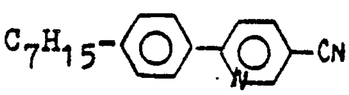 | | 2 | |

Fortsetzung der Tabelle 17

| 1 | 2 | 3 | 4 |
|---|---|---|---|
| | 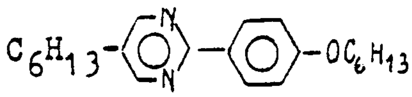 | 5 | |
| | 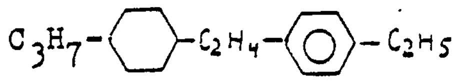 | 20 | |
| | 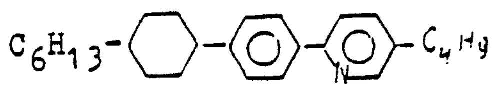 | 8 | |
| | 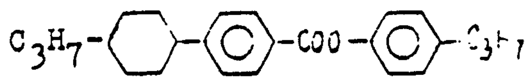 | 3 | |
| | 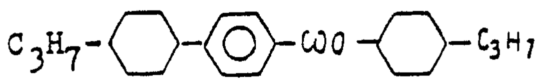 | 3 | |
| | 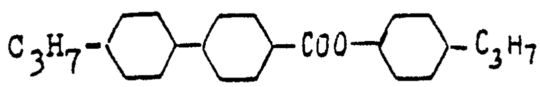 | 12 | |
| | 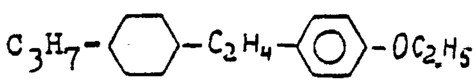 | 10 | |
| | 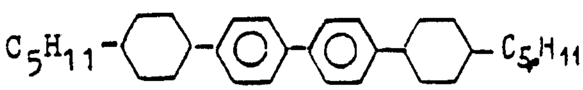 | 9 | |
| | 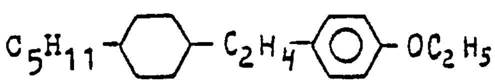 | 10 | |
| 63 | 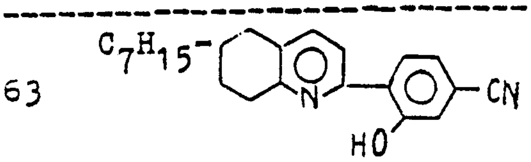 (XVII a) | 1 | -32+63,4 |
| | 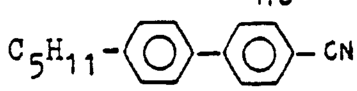 | 30 | |
| | 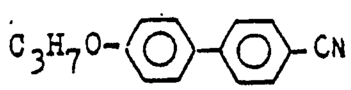 | 7 | |
| | 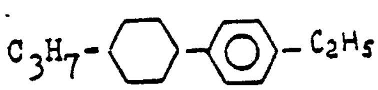 | 15 | |
| | 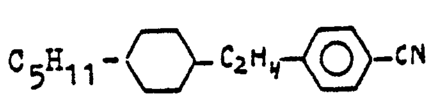 | 10 | |

Fortsetzung der Tabelle 17

| 1 | 2 | 3 | 4 |
|---|---|---|---|
| | 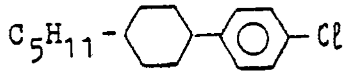 | 5 | |
| | 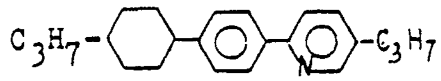 | 8 | |
| | 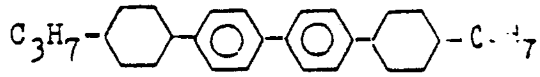 | 10 | |
| | 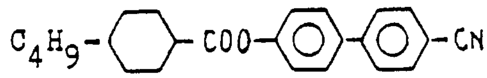 | 10 | |
| | 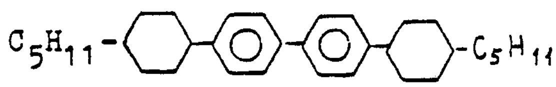 | 4 | |
| 64 | 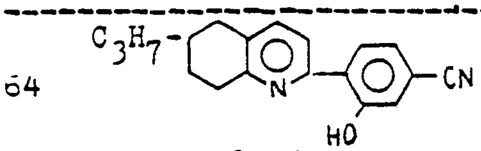 (XVII b) | 10 | -6+113,6 |
| | 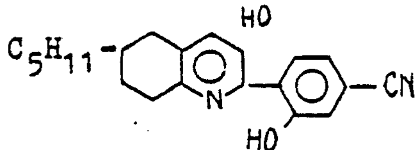 (XVII c) | 10 | |
| | 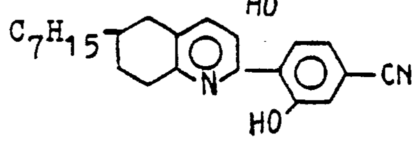 (XVII a) | 20 | |
| | 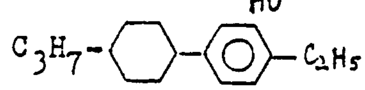 | 30 | |
| | 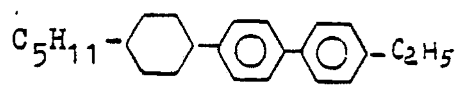 | 10 | |
| | 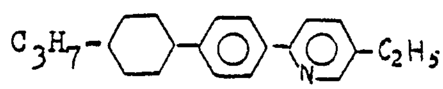 | 10 | |
| | 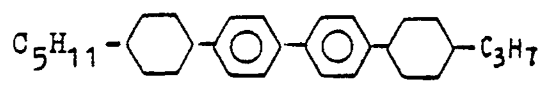 | 10 | |
| 65 | 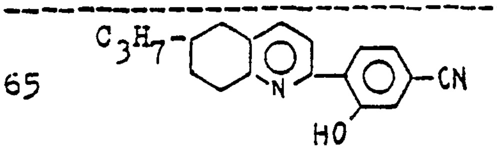 (XVII b) | 16 | -3+123,3 |
| | 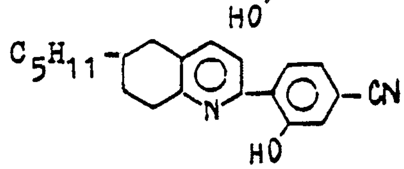 (XVII c) | 17 | |

Fortsetzung der Tabelle 17

| 1 | 2 | 3 | 4 |
|---|---|---|---|
| 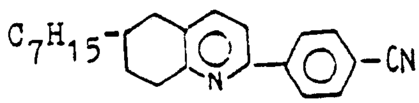 | (XVII a) | 17 | |
| 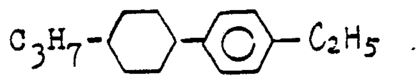 | | 20 | |
| 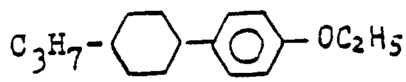 | | 5 | |
| 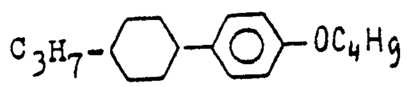 | | 5 | |
| 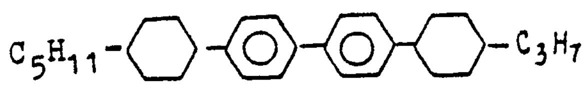 | | 10 | |
| 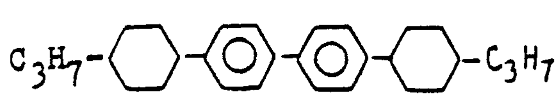 | | 10 | |

Tabelle 17 zeigt, daß die Verwendung der neuen Verbindungen der Formel XVII als Komponenten von flüssigkristallinen Materialien den letztgenannten einen breiten Bereich der nematischen Phase sicherstellt.

Beispiel 66

Herstellung von 6-Alkyl-2-(4-alkyl- oder alkoxystyryl)-5,6,7,8-tetrahydrochinolinen (XVIII)

66.1. 2-Methyl-6-pentyl-5,6,7,8-tetrahydrochinolin

2-Methyl-6-pentyl-5,6,7,8-tetrahydrochinolin wird analog zur Verbindung II hergestellt. Man erhitzt 1 Mol 4-Pentylcyclohexanon und 0,3 Mol 1-Dimethylaminobutanon-3 im Stickstoffstrom bei einer zwischen 120 und 130 °C liegenden Temperatur innerhalb von 8 bis 10 Stunden, destilliert die Ausgangsverbindung ab, löst den Rückstand in Alkohol (150 ml) auf, setzt 0,6 Mol Hydroxylammoniumchlorid zu und siedet innerhalb von 6 Stunden. Die Reaktionsmasse wird mit Wasser verdünnt, alkalisiert und mit Benzol extrahiert. Den Benzolextrakt filtriert man durch $SiO_2$ bei L 5/40 (h = 3 cm) ab, treibt das Lösungsmittel ab und destilliert den Rückstand unter vermindertem Druck ab. $T_{Siede}$ 208/1 Torr.

66.2. 6-Pentyl-2-(4-butoxystyryl)-5,6,7,8-tetrahydrochinolin (XVIII a)

Man siedet 0,01 Mol 2-Methyl-6-pentyl-5,6,7,8-tetrahydrochinolin, 0,014 Mol 4-Butoxybenzaldehyd in 10 ml Essigsäureanhydrid innerhalb von 30 Stunden im Stickstoffstrom. Man treibt das Essigsäureanhydrid ab, alkalisiert und extrahiert den Rückstand mit Benzol. Der Benzolextrakt wird durch $SiO_2$ bei L 5/40 und L = 3 cm filtriert, das Lösungsmittel abdestilliert und der Rückstand aus Alkohol umkristallisiert.
$T_{C\text{-}N}$ = 120 °C, $T_{N\text{-}I}$ = 138,5 °C

| | | | |
|---|---|---|---|
| Gefunden in %: | C 82,88; | H 9,26, | $C_{26}H_{35}NO$ |
| Berechnet in %: | C 82,71; | H 9,34. | |

Analog werden 4-substituierte Styrylderivate hergestellt, die Br, Cl, F, CN, $C_nH_{2n+1}$-Gruppen enthalten.

Beispiel 67

Herstellung von 1-(4-Alkyl- oder 4-Alkoxyphenyl)-2-(6-alkyl-5,6,7,8-tetrahydrochinolyl-2)-ethan (XIX)

67a 1-(4-Butoxyphenyl)-2-(6-pentyl-5,6,7,8-tetrahydrohinolyl-2)-ethan

Die Lösung von 30 ml Ethanol und 2 g 2-(4-Butoxystyrol)-6-pentyl-5,6,7,8-tetrahydrochinolin (XVIII a) bringt man in einen Autoklav ein, setzt 2 g Ni/Re zu und hydriert unter einem $R_2$-Druck von 10 atm bei einer zwischen 25 und 30 °C liegenden Temperatur, bis der Wasserstoff vollständig aufgenommen wird. Der Katalysator wird abfiltriert, mit Ethanol gewaschen, das Lösungsmittel abdestilliert und der Rückstand aus Hexan umkristallisiert. Man isoliert die Derivatverbindung XIX a in einer Ausbeute von 70 %.
$T_C$ = 42,6 °C, $T_{I-N}$ = 29,2 °C

| | | | |
|---|---|---|---|
| Gefunden in %: | C 82,19; | H 9,91. | $C_{26}H_{37}NO$. |
| Berechnet in %: | C 82,27; | H 9,83 | |

Beispiel 68

Herstellung von 2- [2-(4-Alkyl- oder Alkoxyphenyl)ethynyl] -6-alkyl-5,6,7,8-tetrahydrochinolin (XX)

2- [-2-(4-Butoxyphenyl)ethynyl] -6-alkyl-5,6,7,8-tetrahydrochinolin (XX a)

Man löst 0,03 Mol 2-(4-Butoxystyryl)-6-pentyl-5,6,7,8-tetrahydrochinolin in 21 ml Eisessig auf und tropft 0,03 Mol $Br_2$ in 3 ml Essigsäure unter Umrühren bei Raumtemperatur zu. Man siedet das Gemisch innerhalb von 40 Minuten, kühlt ab und gießt in 100 ml Wasser aus. Man extrahiert mit Benzol und chromatografiert auf einer Säule aus $Al_2O_3$ mit einem Aktivitätsgrad II (Eluent Hexan:Benzol im Verhältnis von 2:1). Man treibt das Lösungsmittel ab, bringt den Rückstand von ungereinigtem $\alpha$ -oder $\beta$ -bromsubstituiertem 5,6,7,8-Tetrahydrochinolin in alkoholische KOH-Lösung (0,03 Mol KOH in 25 ml Alkohol) ein und siedet das Reaktionsgut innerhalb von 2 Stunden, verdünnt dann mit Wasser, extrahiert mit Benzol, filtriert den Benzolextrakt durch $SiO_2$ bei L 5/40 (h = 3 cm), dampft ein und kristallisiert aus Alkohol um, bis eine konstante Schmelztemperatur erreicht wird. Man isoliert die Verbindung XXa in 11%iger Ausbeute. $T_{C-N}$ = 110,5 °C, $T_{N-I}$ = = 124,0 °C.

| | | | |
|---|---|---|---|
| Gefunden in %: | C 83,21; | H 8,70; | $C_{26}H_{33}NO$ |
| Berechnet in %: | C 83,15; | H 8,86 | |

Beispiel 69

Herstellung von 5-Alkyl-2-[4-(4-alkyl-3-methylcyclohexyl)phenyl] -pyridin (XXI)

5-Propyl-2- [4-(4-pentyl-3-methylcyclohexyl)phenyl] -pyridin (XXI a)

Man vermischt 0,03 Mol 1-A ryloyl-4-(4-pentyl-3-methylcyclohexyl)benzol und 0,03 Mol 1-(β -Propylethylenyl)piperidin in 10 ml absolutem Ethanol bei einer zwischen 0 und 10 °C liegenden Temperatur, und das Vermischen dauert bei Raumtemperatur noch 2 Stunden lang. Man setzt 90 ml Ethylalkohol, 0,09 Mol Hydroxylammoniumchlorid, 10 ml Wasser und 5 ml konzentrierte Salzsäure zu und siedet innerhalb von 8 Stunden, destilliert 2 Drittel des Lösungsmittelvolumens ab, setzt dem Rückstand 150 ml Wasser zu und alkalisiert mit wäßriger Ätznatronlösung, bis der pH-Wert 8 erreicht wird. Das ausgeschiedene Produkt wird mit Benzol extrahiert, die Benzolschicht mit Wasser gewaschen, über $Na_2SO_4$ getrocknet und durch $SiO_2$ bei L = 5/40 und h = 3 cm filtriert. Man treibt das Lösungsmittel ab, kristallisiert den Rückstand aus Aceton und Hexan um.

Auf ähnliche Weise werden andere Verbindungen XXI b-e hergestellt.

Phasenübergangstemperaturen von Verbindungen XXI a-e sind in der Tabelle 18 dargestellt.

Tabelle 18

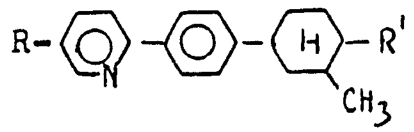

| XXI | R | R' | $T_{C-N}$ C-S, °C | $T_{S-N}$, °C | $T_{N-I}$, °C | Ausbeute in % |
|---|---|---|---|---|---|---|
| a | $C_3H_7$ | $C_5H_{11}$ | 15 | | 111 | 28 |
| b | $C_5H_{11}$ | $C_3H_7$ | 47 | 50,6 | 111,1 | 30 |
| c | $C_2H_5$ | $C_3H_7$ | 62 | | 95,6 | 36 |
| d | $C_2H_5$ | $C_5H_{11}$ | 34 | | 94,7 | 41 |
| e | $CH_2=CH-CH_2$ | $C_5H_{11}$ | 53 | | 83,2 | 40 |

Fortsetzung der Tabelle 18

| XXI | Gefunden in % | | Brutto-formel | Berechnet in % | |
|---|---|---|---|---|---|
| | C | H | | C | H |
| a | 85,91 | 10,23 | $C_{26}H_{37}N$ | 85,89 | 10,26 |
| b | 85,76 | 10,30 | $C_{26}H_{37}N$ | 85,89 | 10,26 |
| c | 85,86 | 9,80 | $C_{23}H_{31}N$ | 85,92 | 9,72 |
| d | 85,91 | 10,07 | $C_{25}H_{35}N$ | 85,90 | 10,09 |
| e | 86,42 | 9,90 | $C_{25}H_{35}N$ | 86,37 | 9,76 |

Tabelle 19 gibt die Zusammensetzung und Eigenschaften von flüssigkristallinen Materialien an, welche neue Verbindungen der Formel XXI enthalten.

Tabelle 19

| Nr. | Zusammensetzung | Masse% | Bereich der nematischen Phase in °C |
|---|---|---|---|
| 1 | 2 | 3 | 4 |
| 70 | 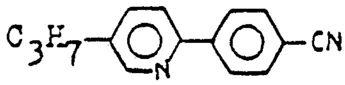 | 20 | -25÷56,3 |
| | 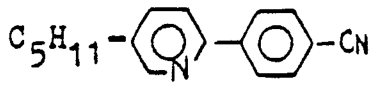 | 30 | |
| | 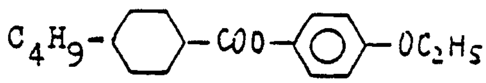 | 25 | |
| | 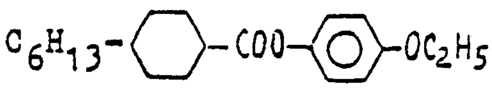 | 15 | |
| | 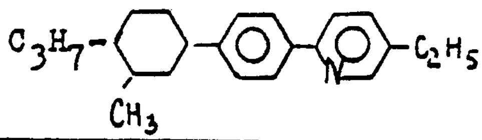 | 10 | |

Die Verwendung von Verbindungen der Formel XXI als Komponenten von flüssigkristallinen Materialien sichert den letztgenannten geringe Untergrenzen von Bereichen der nematischen Phase, welche für die Verwendung von Materialien für elektrooptische Anzeigeelemente erforderlich sind.

Beispiel 71

Herstellung von 2- [4-(4-Alkyl-3-methylcyclohexyl)phenyl] -5-(trans-4-alkylcyclohexyl)-pyridinen (XXII)

2- [4-(4-Pentyl-3-methylcyclohexyl)phenyl] -5-(trans-4-ethylzyklohexyl)-pyridin (XXII a)

erhält man analog zur Verbindung XXI (Beispiel 70) durch Umsetzung von 1-Acryloyl-4-(4-pentyl-3-methylcyclohexyl)benzol mit 1-[β-(trans-4-ethylcyclohexyl)ethylenyl] -pyperidin und anschließende Behandlung von 3-(trans-4-Ethylcyclohexyl)-6- [4-(4-pentyl-3-methylcyclohexyl)phenyl] 3,4-dihydro-2-N-piperidino-2H-pyran mit alkoholischer Lösung von Hydroxylammoniumchlorid.
Die Phasenübergangstemperaturen von Verbindungen XXII a-c sind in der Tabelle 20 angegeben.

Tabelle 20 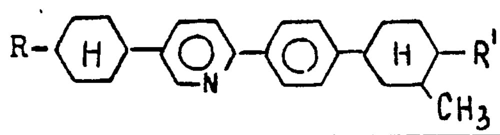

| XXII | R | R' | $T_{C-S}$, °C | $T_{S-N}$, °C | $T_{N-I}$, °C | Ausbeute in % |
|---|---|---|---|---|---|---|
| a | $C_2H_5$ | $C_5H_{11}$ | 70 | 145 | 248 | 21 |
| b | $C_4H_9$ | $C_3H_7$ | 47 | 181 | 259 | 30 |
| c | $C_4H_9$ | $C_5H_{11}$ | 54 | 200 | 257 | 33 |

Fortsetzung der Tabelle 20

| | Gefunden in % | | Bruttoformel | Berechnet in % | |
|---|---|---|---|---|---|
| | C | H | | C | H |
| a | 86,31 | 10,49 | $C_{31}H_{45}N$ | 86,25 | 10,51 |
| b | 86,30 | 10,34 | $C_{31}H_{45}N$ | 86,25 | 10,51 |
| c | 86,40 | 10,56 | $C_{33}H_{49}N$ | 86,21 | 10,74 |

Tabelle 21 gibt die Zusammensetzung und Eigenschaften von flüssigkristallinen Materialien an, welche neue Verbindungen der Formel XXII enthalten.

Tabelle 21

| Nr. | Zusammensetzung | Masse% | Bereich der nematischen Phase in °C |
|---|---|---|---|
| 72 | 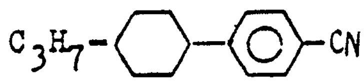 | 20 | -25÷79,3 |
| | 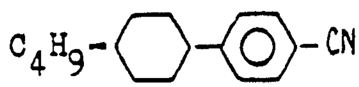 | 16 | |
| | 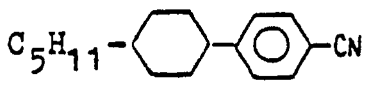 | 22 | |
| | 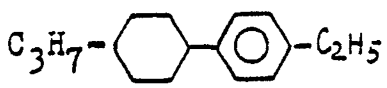 | 7 | |
| | 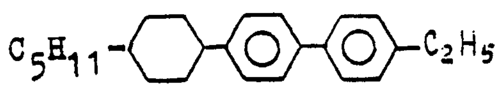 | 25 | |
| | 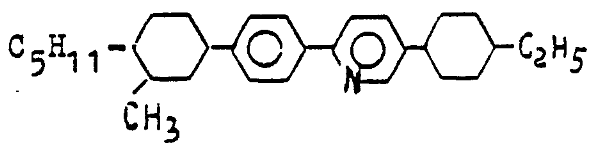 | 10 | |

Die Verwendung der Verbindungen XXII als Komponenten von flüssigkristallinen Materialien sichert den letztgenannten geringe Untergrenzen der Bereiche der nematischen Phase, welche zur Verwendung von Materialien für elektrooptische Anzeigeelemente erforderlich sind.

Beispiel 73

2- [4-(trans-4-Alkylcyclohexyl)phenyl] -5-alkenylpyridine XXIII

2- [4-(trans-4-Heptylcyclohexyl)phenyl] -5-(but-3-en)pyridin

Eine Lösung von 0,08 Mol 4-(trans-4-Heptylcyclohexyl)brombenzol in 30 ml Benzol wird im Argonstrom zu 0,08 Mol 15%iger Lösung von Butyllithium in Hexan zugetropft und innerhalb von 2 bis 3 Stunden umgerührt. Man filtriert den Niederschlag ab, löst in 100 ml Diethylether auf, tropft 0,06 Mol Pyridin bei 0 °C zu und rührt das Reaktionsgut bei Raumtemperatur innerhalb von 3 Stunden um, kühlt dann auf eine Temperatur von -5 °C ab und tropft 0,06 Mol Lösung von 1-Brombuten-3 in 100 ml Tetrahydrofuran zu.

Die Reaktionsmasse wird innerhalb von 2 Stunden bei Raumtemperatur umgerührt, mit 300 ml Wasser zersetzt, die organische Schicht abgetrennt, durch $SiO_2$ bei L = 5/40 (h = 3 cm) filtriert, das Lösungsmittel entfernt und der Rückstand aus Aceton umkristallisiert. Man isoliert die Verbindung XXIII a.

Auf ähnliche Weise werden andere Analóga XXIII hergestellt. Phasenübergangstemperaturen und Verbindungen XXIII a-g sind in der Tabelle 22 dargestellt.

Tabelle 22

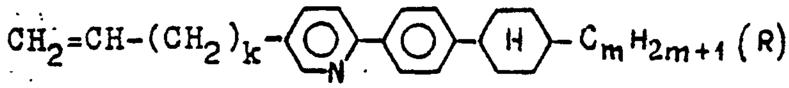

(XXIII)

| Nr. XXIII | K | $C_mH_{2m+1}$ (R) | $T_{C-S}$, °C | $T_{S-N}$, °C | $T_{N-I}$ °C |
|---|---|---|---|---|---|
| a | 1 | $C_3H_7$ | 50,0 | 80,0 | 162,1 |
| b | 1 | $C_5H_{11}$ | 68,7 | 107,8 | 163,6 |
| c | 1 | $C_7H_{15}$ | 47,4 | 114,3 | 151,2 |
| d | 1 | $C_9H_{19}$ | 68,8 | 117,8 | 144,6 |
| e | 2 | $C_2H_5$ | 51,0 | 87,4 | 163,2 |
| f | 2 | $C_7H_{15}$ | 38,0 | 143,0 | 173,3 |
| g | 3 | $C_2H_5$ | 20,0 | 79,8 | 134,1 |

Fortsetzung der Tabelle 22

| Nr. | Gefunden in % | | Bruttoformel | Berechnet in % | |
|---|---|---|---|---|---|
| | C | H | | C | H |
| a | 86,36 | 9,21 | $C_{23}H_{29}N$ | 86,47 | 9,15 |
| b | 86,43 | 9,49 | $C_{25}H_{33}N$ | 86,40 | 9,57 |
| c | 86,38 | 10,00 | $C_{27}H_{37}N$ | 86,34 | 9,93 |
| d | 86,15 | 10,22 | $C_{29}H_{41}N$ | 86,29 | 10,24 |
| e | 86,50 | 9,30 | $C_{23}H_{29}N$ | 86,47 | 9,15 |
| f | 86,18 | 10,11 | $C_{28}H_{39}N$ | 86,32 | 10,09 |
| g | 86,51 | 9,31 | $C_{24}H_{31}N$ | 86,43 | 9,37 |

Beispiel 74

Herstellung von 2-(4-Alkyl- oder Alkoxyphenyl)-5-alkenylpyridinen (XXIV)

5-(Prop-2-en)-2-(4-pentylphenyl)pyridin

Man erhält analog zur Verbindung XXIII durch Umsetzung von 4-Pentylphenyllithium mit Pyridin und anschließender Behandlung mit 1-Brompropen-2.

| | | | |
|---|---|---|---|
| Gefunden in %: | C 86,06; | H 8,87; | $C_{19}H_{23}N$ |
| Berechnet in %: | C 85,99; | H 8,74. | |

Auf ähnliche Weise wird 5-(But-3-en)-2-(4-decyloxy-3-fluorphenyl)pyridin hergestellt, $T_{C-N}$ = 40 °C, $T_{N-I}$ = 42 °C.

Beispiel 75

Herstellung von 5-Alkyl-2-(4-alkenylphenyl)-pyridinen (XXV)

75.1. 2-(4-Vinylphenyl)-5-pentylpyridin (XXV a)

In einen Kolben, versehen mit magnetischem Rührwerk, Tropftrichter und Rückflußkühler, werden im Argon strom 10 mMol 2-(4-Jodphenyl)-5-pentylpyridin, 0,1 mMol $PdCl_2(PPh_3)_2$ in 5 ml Tetrahydrofuran eingebracht. Aus dem Tropftrichter tropft man allmähliche die Lösung von 15 mMol $CH_2$ = CH-MgBr in 15 ml Tetrahydrofuran zu, rührt bei Raumtemperatur innerhalb von 4 Stunden um, zersetzt das überschüssige Grignards Reagens mit Methanol, setzt Wasser und Hydrochinon zu und extrahiert mit Ether. Die ätherische Lösung wird über $MgSO_4$ getrocknet, durch $Al_2O_3$ (h = 3 cm) filtriert und das Lösungsmittel abdestilliert. Die Substanz wird durch Umkristallisation aus Äthanol isoliert.

$T_{C-S}$ = 6 °C, $T_{S-I}$ = 54,7 °C (Ausbeute 38 %)

| | | | |
|---|---|---|---|
| Gefunden in %: | C 86,22; | H 8,39; | $C_{18}H_{21}N$ |
| Berechnet in %: | C 86,01; | H 8,42. | |

Auf ähnliche Weise ist 5-Pentyl-2- [4-(pent-4-en)phenyl ] -pyridin in 40%iger Ausbeute mit $T_{S-I}$ = 17°C hergestellt.

| | | | |
|---|---|---|---|
| Gefunden in %: | C 86,12; | H 9,19; | $C_{21}H_{27}N$ |
| Berechnet in %: | C 85,95; | H 9,27. | |

Beispiel 76

Herstellung von 5-Alkenyl-2- [4-(alkenylphenyl)] pyridin (XXVI)

In einen Kolben, versehen mit magnetischem Rührwerk, Tropftrichter und Rückflußkühler, bringt man im

Argonstrom 10 mMol 2-(4-Jodphenyl)-5-(prop-2-en)pyridin ($T_{C-S}$ = 62 °C, $T_{S-I}$ = 85,1 °C), 0,1 mMol $PdCl_2$ - $(PPh_3)_2$ in 5 ml Tetrahydrofuran ein und dropft man allmählich eine Lösung von 15 mMol $CH_2=CH-CH_2MgBr$ in 15 ml Tetrahydrofuran zu. Das Zersetzen und Isolieren verwirklicht man analog zur Herstellung der Verbindung XXV.

| | | | |
|---|---|---|---|
| Gefunden in %: | C 86,90; | H 7,19; | $C_{17}H_{17}N$ |
| Berechnet in %: | C 86,77; | H 7,28. | |

Beispiel 77

Herstellung von 2-(4-Fluorphenyl)-5-alkenylpyridinen (XXVII)

2-(4-Fluorphenyl)5-(prop-2-en)pyridin

77.1. Hergestellt analog zur Verbindung XXIII

$T_C$ = 17 °C

| | | | |
|---|---|---|---|
| Gefunden in %: | C 78,96; | H 5,60; | $C_{14}H_{12}FN$ |
| Berechnet in %: | C 78,85; | H 5,67 | |

Auf ähnliche Weise ist 2- (4-Fluorphenyl)-5-(but-3-en)pyridin mit $T_C$ = 18 °C (aus Alkohol) hergestellt.

| | | | |
|---|---|---|---|
| Gefunden in %: | C 79,38; | H 6,31; | $C_{15}H_{14}FN$ |
| Berechnet in %: | C 79,27; | H 6,21 | |

Beispiel 78

Herstellung von 2-(4-Ethinylphenyl)-5-alkylpyridinen (XXVIII)

78.1. 2- [(4-Hept-1-inyl)-phenyl)] -5-pentylpyridin XXVIIIa

In einen Kolben, versehen mit Rückflußkühler, werden 10 mMol 2-(4-Jodphenyl)-5-pentylpyridin, 30 ml Chloroform, 20 mMol $Et_3N$, 0,1 mMol CuJ, 0,1 mMol $PdCl_2$ $(PPh_3)_2$, 15 mMol $CH{=}CC_5H_{11}$ eingebracht und innerhalb von 4 Stunden gesiedet. Man filtriert, verdünnt mit Wasser, trocknet die organische Schicht über $Na_2SO_4$ und dampft ein. Der Rückstand wird aus Alkohol umkristallisiert. Man isoliert die Verbindung XXVIIIa in 40%iger Ausbeute.

$T_{C-S}$ = 35 °C, $T_{S-I}$ = 44,6 °C.

| | | | |
|---|---|---|---|
| Gefunden in %:<br>Berechnet in %: | C 86,60;<br>C 86,47; | H 9,01;<br>H 9,15 | $C_{23}H_{29}N$ |

Auf ähnliche Weise wird 2-/(4-Hept-1-inyl)phenyl)-5-heptylpyridin (XXVIII b) mit $T_{C-S}$ = 27 °C, $T_{S-I}$ = 66,5°C (aus Alkohol) hergestellt.

| | | | |
|---|---|---|---|
| Gefunden in %:<br>Berechnet in %: | C 86,55:<br>C 86,40: | H 9,41:<br>H 9,57 | $C_{25}H_{33}N$ |

Beispiel 79

Herstellung von 1-(5-Alkylpyrid-2-yl)-2-(4-alkyl-oder alkoxyphenyl)-acethylenen (XXIX)

1-(5-Pentylpyrid-2-yl)-2-(4-butoxyphenyl)acetylen XXIX a

79.1. 1-(5-Brompyrid-2-yl)-2-(4-butoxyphenyl)acetylen

wird analog zur Verbindung XXVIII a durch Umsetzung von 2-Jod-5-brompyridin und 1-Ethinyl -4-butoxybenzol in Gegenwart von $Et_3N$, $PdCl_2(PPh_3)_2$ in 50%iger Ausbeute hergestellt.

Auf ähnliche Weise ist 1-(5-Brompyrid-2-yl)-2-(4-pentylphenyl)acetylen hergestellt.worden.

$T_C$ = 103 °C (aus Alkohol); 1-(5-Brompyrid-2-yl)-2-(4-propylphenyl)acetylen, $T_C$ = 102-104 °C (aus Alkohol).

79.2. 1-(5-Pentylpyrid-2-yl)-2-(4-butoxyphenyl)acetylen (XXIX a)

erhält man durch Umsetzen von 1-(5-Brompyrid-2-yl)-2-(4-butoxyphenyl)acetylen mit $C_5H_{11}MgBr$ in Tetrahydrofuran TGPh in 20%iger Ausbeute. $T_{C-N}$ = 75 °C, $T_{N-I}$ = 78 °C

| | | | |
|---|---|---|---|
| Gefunden in %:<br>Berechnet in %: | C 82,36:<br>C 82,20: | H 8,49.<br>H 8,47 | $C_{22}H_{27}NO$ |

Auf ähnliche Weise wurden Verbindungen der Formel XXIX hergestellt:
1-(5-Heptylpyrid-2-yl)-2-(4-ethoxyphenyl)acetylen (XXIX b)
$T_C$ = 84,5 °C, $T^{*}_{I-N}$ = 76,5 °C

| | | | |
|---|---|---|---|
| Gefunden in %:<br>Berechnet in %: | C 82,30:<br>C 82,20: | H 8,44;<br>H 8,47 | $C_{22}H_{27}NO$ |

1-(5-Propylpyrid-2-yl)-2-(4-ethoxyphenyl)-acetylen (XXIX c)
$T_C$ = 101 °C; $T^{*}_{I-N}$ = 89 °C (aus Alkohol)

| | | | |
|---|---|---|---|
| Gefunden in %: | C 81,34; | H 8,44; | $C_{18}H_{19}NO$ |
| Berechnet in %: | C 81,48; | H 8,47 | |

1-(5-Pentylpyrid-2-yl)-2-(4-methoxyphenyl)acetylen (XXIX d)
$T_C$ = 51,5 °C; $T^{*}_{I\text{-}N}$ = 50 °C

| | | | |
|---|---|---|---|
| Gefunden in %: | C 81,60; | H 7,88; | $C_{19}H_{21}NO$ |
| Berechnet in %: | C 81,68; | H 7,58 | |

1-[5-(trans-4-Ethylcyclohexyl)pyrid-2-yl] -2-(4-butoxyphenyl)a etylen (XXIX e) mit $T_{C\text{-}N}$ = 130 °C; $T_{N\text{-}I}$ = 207 °C

| | | | |
|---|---|---|---|
| Gefunden in %: | C 82,91; | H 8,52; | $C_{25}H_{31}NO$ |
| Berechnet in %: | C 83,06; | H 8,64 | |

Beispiel 80

Herstellung von 2,5-Bis-(4-R-phenylethinyl)pyridinen (XXX)

2,5-Bis-(4-propylphenylethinyl)pyridin (XXX a)

erhält man analog zu XXIX durch Reaktion von 2,5-Di brompyridin mit

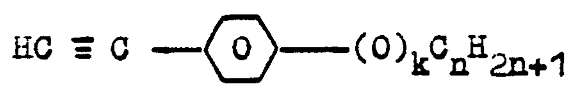

in Gegenwart von CuJ und $PdCl_2(PPh_3)_2$ in 40%iger Ausbeute.
$T_{C\text{-}S}$ = 139 °C; $T_{S\text{-}N}$ = 144 °C; $T_{N\text{-}I}$ = 237 °C (aus Aceton)

| | | | |
|---|---|---|---|
| Gefunden in %: | C 89,34; | H 7,02; | $C_{27}H_{25}N$ |
| Berechnet in %: | C 89,21; | H 6,93 | |

Beispiel 81

Herstellung von 1-(5-cyanopyrid-2-yl)-2-(4-alkyl-oder alkoxyphenyl)acetylen (XXXI)

81.1. 1-(5-cyanopyrid-2-yl)-2-(4-amylphenyl)-acetylen (XXXI a)

Ein Gemisch aus 0,01 Mol 1-(5-Brompyrid-2-yl)-2-(4-amylphenyl)acetylen, 0,011 Mol CuCN, 15 ml N-Methylpyrrolidon erhitzte man unter Sieden, bis die Ausgangsverbindung verschwand. Das Zersetzen und

Isolieren führte man analog zur Verbindung XIII durch.
$T_{C-N}$ = 95,0 °C; $T_{N-I}$ = 101 °C

| | | | |
|---|---|---|---|
| Gefunden in %: | C 83,16; | H 6,75; | $C_{19}H_{18}N_2$ |
| Berechnet in %: | C 83,18; | H 6,61 | |

Beispiel 82

Herstellung von 2-(4-Alkoxy-2-hydroxyphenyl)-5-alkylpyridinen (XXXII)

2-(4-Nonyloxy-2-hydroxyphenyl)-5-nonylpyridin (XXXII a)

Man vermischt bei 0 °C 0,01 Mol 1-Akryloyl-2,4-dinonyloxybenzol mit 0,1 Mol 1- [β -Nonylethylenyl] piperidin und läßt für 4 Stunden stehen.

Die Reaktionsmasse verdünnt man mit 100 ml Ethylalkohol und 10 ml Wasser, setzt 0,03 Mol Hydroxylammoniumchlorid zu und siedet innerhalb von 8 Stunden. Dann treibt man 2 Drittel des Lösungsmittelvolumens ab, verdünnt den Rückstand mit 100 ml Wasser, alkalisiert bis zum Erreichen eines pH-Wertes von etwa 8 und extrahiert mit Benzol.

Die Benzollösung filtriert man durch $SiO_2$-Schicht mit L = 5/40 (h = 3 cm), setzt 0,005 Mol $AlCl_3$ zu und läßt für 24 Stunden stehen. Man gießt die Reaktionsmasse auf Eiswasser aus, trennt die organische Schicht ab, wäscht mit Wasser, 10%iger Lösung von Natriumhydrogencarbonat, Wasser, trocknet über $Na_2SO_4$, filtriert und destilliert das Lösungsmittel ab. Der Rückstand wird aus Alkohol umkristallisiert. Man isoliert die Verbindung XXXII a in 15%iger Ausbeute mit $T_{C-S}$ = 44 °C; $T_{S_A-I}$ = 104 °C.

| | | | |
|---|---|---|---|
| Gefunden in %: | C 79,18; | H 10,22; | $C_{29}H_{45}NO_2$ |
| Berechnet in %: | C 79,22; | H 10,32. | |

Auf ähnliche Weise wurde 2-(4-Butyloxy-2-hydroxyphenyl)-5-heptylpyridin XXXII b mit $T_{C-S_A}$ = 53 °C und $T_{S_A-I}$ = = 100 °C hergestellt.

| | | | |
|---|---|---|---|
| Gefunden in %: | C 77,41; | H 9,30; | $C_{22}H_{31}NO_2$ |
| Berechnet in %: | C 77,38; | H 9,10. | |

Auf ähnliche Weise wurde 2-(4-Hexyloxy-2-hydroxyphenyl)-5-heptylpyridin XXXII c mit $T_{C-S_A}$ = 46 °C und $T_{S_A-I}$ = 91 °C hergestellt.

| | | | |
|---|---|---|---|
| Gefunden in %: | C 78,25; | H 9,43; | $C_{24}H_{35}NO_2$ |
| Berechnet in %: | C 78,00; | H 9,55 | |

Beispiel 83

Herstellung von 2-(4-Alkyl-2-hydroxyphenyl)-5-alkylpyridinen (XXXIII)

2-(4-Methyl-2-hydroxyphenyl)-5-pentylpyridin (XXXIII a)

erhält man analog zu Beispiel 82 durch Umsetzung von 1-/β -Pentylethylenyl/ piperidin mit 1-Acryloyl-2-methoxy-4-methylbenzol und anschließender Kondensation mit Hydroxylammoniumchlorid und Dealkylierung der Methoxygruppe im hergestellten 2-(4-Methyl-2-Methoxyphenyl)-5-pentylpyridin.
$T_C$ = 67,4 °C (aus Alkohol).

| | | | |
|---|---|---|---|
| Gefunden in %: | C 79,76; | H 8,35; | $C_{17}H_{21}NO$ |
| Berechnet in %: | C 79,96; | H 8,29 | |

Beispiel 84

Herstellung von 2-(4-Alkyl- oder Alkoxy-2-hydroxyphenyl)-5-(trans-4-alkylcyclohexyl)-pyridin (XXXIV)

2-(4-Ethoxy-2-hydroxyphenyl)-5-(trans-4-butylcyclohexyl)pyridin (XXXIV a)

erhält man analog zu XXXII a durch Umsetzung von 1-Acryloyl-2,4-diethoxybenzol mit 1-[β-(trans-4-alkylcyclohexyl)ethylenyl] piperidin. $T_{C\text{-}S}$ = 119 °C; $T_{S\text{-}N}$ = 219 °C; $T_{N\text{-}I}$ = 231 °C (aus Alkohol.

| | | | |
|---|---|---|---|
| Gefunden in %: | C 78,22; | H 8,7; | $C_{23}H_{31}NO_2$ |
| Berechnet in %: | C 78,15; | H 8,83 | |

Auf ähnliche Weise wurde 2-(4-Methyl-2-hydroxyphenyl)-5-(trans-4-ethylcyclohexyl)pyridin (XXXIV b) hergestellt. $T_{C\text{-}S}$ = 125,6 °C: $T_{S\text{-}N}$ = 145 °C:$T_{N\text{-}I}$ = 183,1 °C (aus Alkohol).

| | | | |
|---|---|---|---|
| Gefunden in %: | C 80,32; | H 9,88; | $C_{20}H_{29}NO$ |
| Berechnet in %: | C 80,21; | H 9,76 | |

Beispiel 85

Herstellung von 2-(4-Brom-2-hydroxyphenyl)-5-alkylpyridinen (XXXV)

2-(4-Brom-2-hydroxyphenyl)-5-heptylpyridin (XXXV a)

erhält man analog zur Verbindung XXXII durch Umsetzung von 1-[β-Heptylethylenyl/piperidin mit 1-Acryloyl-4-brom-2-methoxybenzol. $T_{C\text{-}S}$ = 66 °C: $T_{S\text{-}I}$ = 78 °C

| | | | |
|---|---|---|---|
| Gefunden in %: | C 61,95; | H 6,42; | $C_{18}H_{22}BrNO$ |
| Berechnet in %: | C 62,07; | H 6,37. | |

Analog lassen sich 2-(4-Fluor- oder Jod-2-hydroxyphenyl)-5-alkylpyridine herstellen.

Beispiel 86

Herstellung von 2-(4-Cyano-2-hydroxyphenyl)-5-alkylpyridinen (XXXVI)

2-(4-Cyano-2-hydroxyphenyl)-5-heptylpyridin (XXXVI a)

erhält man analog zur Verbindung XVII durch Umsetzung von 2-(4-Brom-2-hydroxyphenyl)-5-heptylpyridin mit CuCN in N-Methylpyrrolidon. $T_C$ = 83 °C (aus Alkohol).

| | | | |
|---|---|---|---|
| Gefunden in %: | C 77,35; | H 7,65; | $C_{19}H_{22}N_2O$ |
| Berechnet in %: | C 77,52; | H 7,53 | |

Beispiel 87

Herstellung von 1- [5-(trans-4-Alkylcyclohexyl)-pyrid-2-yl] -2- 4-alkyl- oder alkoxyphenyl)ethan (XXXVII)

1- [5-(trans-4-Ethylcyclohexyl)pyrid-2-yl] -2-(4-but-oxyphenyl)ethan

87.1. 1- [5-(trans-4-Ethylcyclohexyl)pyrid-2-yl]-2-(4-butoxyphenyl)ethylen

Ein Gemisch aus 0,1 Mol 2-Methyl-5-(4-ethylcyclohexyl)-pyridin, 50 ml Essigsäureanhydrid und 0,12 Mol 4-Butoxybenzaldehyd wird innerhalb von 48 Stunden gesiedet. Man destilliert Essigsäureanhydrid ab, verdünnt den Rückstand mit 250 ml Wasser, alkalisiert bis zum Erreichen eines pH-Wertes von etwa 8 und extrahiert bis zum Erreichen Benzolextrakt wird durch $SiO_2$ bei L = 5/40 (h = 3 cm) filtriert und eingedampft. Der Niederschlag wird aus Aceton umkristallisiert.

Man isoliert die Verbindung in 25%iger Ausbeute mit $T_{C-S}$ = 90 °C, $T_{S-N}$ = 114 °C und $T_{N-I}$ = 230 °C.

Auf ähnliche Weise erhält man 1- [5-(trans-4-Butylcyclohexyl)pyrid-2-yl] -2-(4-butoxyphenyl)ethylen mit $T_{C-S}$ = 78 °C, $T_{S-N}$ = 171 °C: $T_{N-I}$ = 243 °C.

87.2 1- [5-(trans-4-Ethylcyclohexyl)pyrid-2-yl]-2-(4-butoxyphenyl)ethan (XXXVII a)

0,01 Mol Verbindung 87,1 in 50 ml Ethylalkohol, 2 g Ni/Re hydriert man unter einem Wasserstoffdruck von 10 atm bei einer Temperatur von 30 °C, bis der Wasserstoff völlig aufgenommen wird. Man filtriert, dampft das Filtrat ein, kristallisiert den Rückstand aus Alkohol um, isoliert die Verbindung XXXVII a in 60%iger Ausbeute mit $T_{C-S}$ = 48 °C, $T_{S-N}$ = 61 °C: $T_{N-I}$ = 93 °C.

| | | | |
|---|---|---|---|
| Gefunden in %: | C 82,26; | H 9,58; | $C_{25}H_{35}NO$ |
| Berechnet in %: | C 82,14; | H 9,65 | |

Auf ähnliche Weise erhält man 1- [5-(trans-4-Butylcyclohexyl)pyrid-2-yl] -2-(4-butoxyphenyl)ethan (XXXVII b). $T_{C-S}$ = 41 °C; $T_{S-I}$ = 119 °C

| | | | |
|---|---|---|---|
| Gefunden in %: | C 82,51; | H 9,97; | $C_{27}H_{39}NO$ |
| Berechnet in %: | C 82,39; | H 9,98 | |

Beispiel 88

1- [5-(trans-4-Alkylcyclohexyl)pyrid-2-yl]-2- [4-(trans-4-alkylcyclohexyl)phenyl] ethan (XXXVIII)

1- [5-(trans-4-Ethylcyclohexyl)pyrid-2-yl]-2-[4-(trans-4-propylcyclohexyl)phenyl]ethan (XXXVIII)

88.1. 1-[5-(trans-4-Ethylcyclohexyl)pyrid-2-yl]-2-[4-(trans-4-propylcyclohexyl)phenyl] ethylen

Ein Gemisch aus 0,1 Mol 2-Methyl-5-(4-ethylcyclohexyl)-pyridin, 0,01 Mol 4-(4-Propylcyclohexyl)-benzaldehyd und 50 ml Acetaldehyd wird innerhalb von 30 Stunden gesiedet. Man treibt das Essigsäureanhydrid unter vermindertem Druck ab, alkalisiert den Rückstand und extrahiert mit Benzol, destilliert das Lösungsmittel ab und kristallisiert den Rückstand aus Aceton um. Man isoliert die Verbindung 88.1 in 25%iger Ausbeute mit $T_{Schmelz}$ > 250 °C.

| | | | |
|---|---|---|---|
| Gefunden in %: | C 86,71; | H 10,07; | $C_{30}H_{41}N$ |
| Berechnet in %: | C 86,68; | H 9,94 | |

88.2. 1- [5-(trans-4-Ethylcyclohexyl)pyrid-2-yl] -2- [4-(trans-4-propylcyclohexyl)phenyl] ethan (XXXVIII)

erhält man analog zu Beispiel 87.2 durch Hydrieren des Ethylenderivates.
$T_{C-N}$ = 140 °C, $T_{N-I}$ = 196 °C.

| | | | |
|---|---|---|---|
| Gefunden in %: | C 86,31; | H 10,27; | $C_{30}H_{43}N$ |
| Berechnet in %: | C 86,27; | H 10,38 | |

Beispiel 89

2- {4- [2-(trans-4-Propylcyclohexyl)ethyl] phenyl}-5-(trans-ethylcyclohexyl)pyridin (XXXIX a)

erhält man analog zu Beispiel 70 durch Umsetzung von 1- [$\beta$-(trans-4-Ethylcyclohexyl)ethylenyl] piperidin mit dem jeweiligem Vinylketon und anschließende Cyklisierung mit Hydroxylammoniumchlorid.
$T_{C-S}$ = 99 °C, $T_{S-N}$ = 161 °C, $T_{N-I}$ = 262 °C.

| | | | |
|---|---|---|---|
| Gefunden in %: | C 86,51; | H 10,24; | $C_{30}H_{43}N$ |
| Berechnet in %: | C 86,27; | H 10,38 | |

Auf ähnliche Weise wird 2-{4-[2-(trans-4-Propylcyclohexyl)ethyl] phenyl}-5-(trans-4-butylcyclohexyl)-pyridin (XXXIX b) hergestellt.
$T_{C-S}$ = 74 °C, $T_{S-N}$ = 212 °C, $T_{N-I}$ = 268 °C.

| | | | |
|---|---|---|---|
| Gefunden in %: | C 86,40; | H 10,45; | $C_{32}H_{47}N$ |
| Berechnet in %: | C 86,23; | H 10,63. | |

Industrielle Anwendbarkeit

Die vorliegende Erfindung findet in der Elektronik zur Wiedergabe der alphanumerischen Information, zum Beispiel bei elektronischen Uhren und Mikrorechenmaschinen breite Anwendung.

## Ansprüche

1. Flüssigkristalline Derivate von stickstoffhaltigen heterozyklischen Systemen der allgemeinen Formel $R^1 - A^1 - Z - A^2 - R^2$,
worin $R^1$ und $R^2$ gleichzeitig oder unabhängig $-C_nH_{2n+1}$, $-C_mH_{2n+1}$, $C_nH_{2n+1}O-$, $C_mH_{2m+1}O-$, $CH_2=CH(CH_2)_k-$, -CN, -N=C=S, -Hal, ·

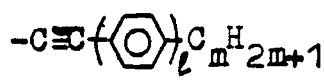

bei k = 0-3; 1 = 0-2; m, n = 1-15, Z $-CH_2-CH_2-$, -CH=CH-, -C≡C-, -COO- mit Einfachbindung sind, wobei falls

A1 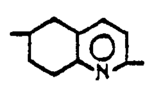 , 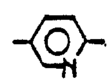 ;

ist, so A2 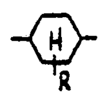 , 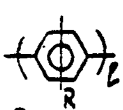 , 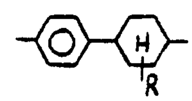 , 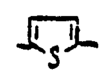

darstellt; falls A2 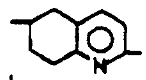 , 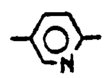 , 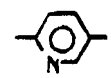

ist, so A1 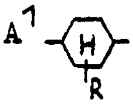 , 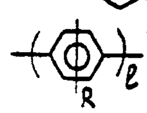 , 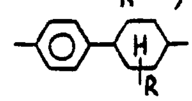 darstellt,

R für $-CH_3$, -Cl, -CN, -F steht, ℓ/0 bis 2 beträgt.

2. Verbindung nach Anspruch 1 der Formel

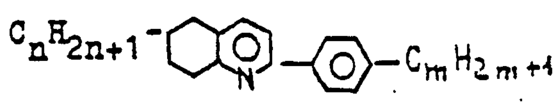

3. Verbindung nach Anspruch 1 der Formel

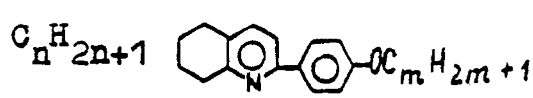

4. Verbindung nach Anspruch 1 der Formel

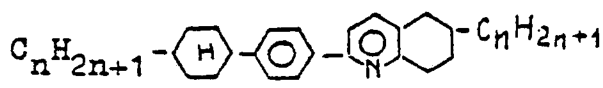

5. Verbindung nach Anspruch 1 der Formel

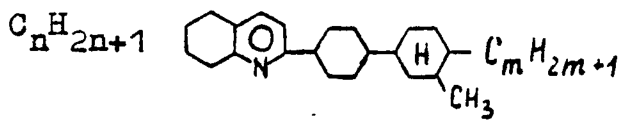

6. Verbindung nach Anspruch 1 der Formel

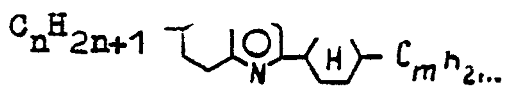

7. Verbindung nach Anspruch 1 der Formel

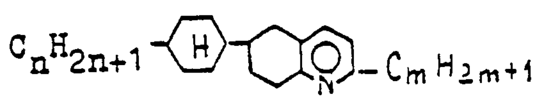

8. Verbindung nach Anspruch 1 der Formel

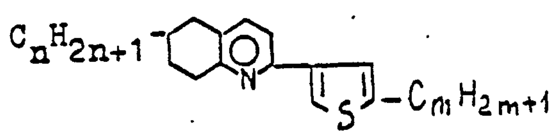

9. Verbindung nach Anspruch 1 der Formel

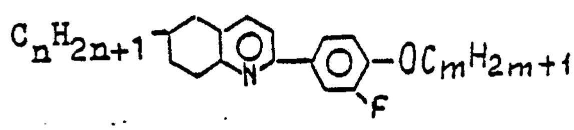

10. Verbindung nach Anspruch 1 der Formel

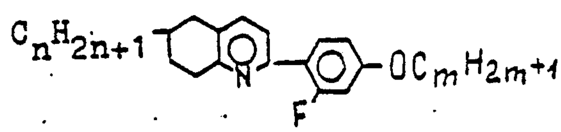

11. Verbindung nach Anspruch 1 der Formel

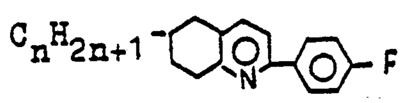

12. Verbindung nach Anspruch 1 der Formel

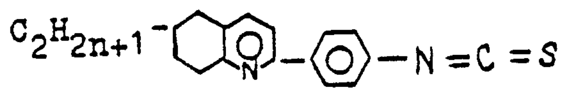

13. Verbindung nach Anspruch 1 der Formel

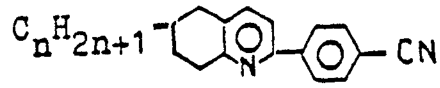

14. Verbindung nach Anspruch 1 der Formel

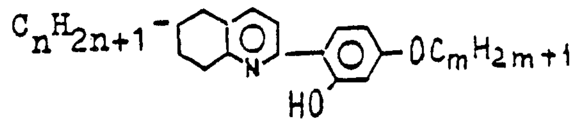

15. Verbindung nach Anspruch 1 der Formel

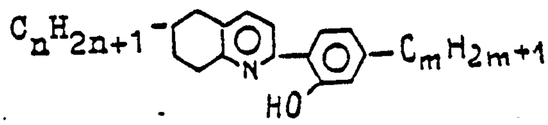

16. Verbindung nach Anspruch 1 der Formel

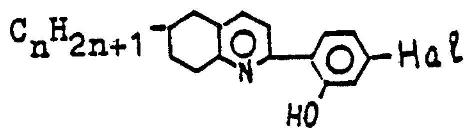

17. Verbindung nach Anspruch 1 der Formel

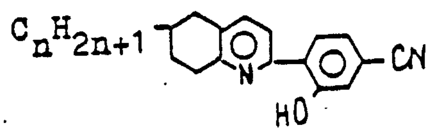

18. Verbindung nach Anspruch 1 der Formel

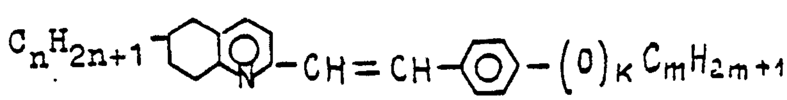

19. Verbindung nach Anspruch 1 der Formel

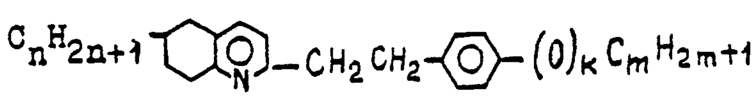

20. Verbindung nach Anspruch 1 der Formel

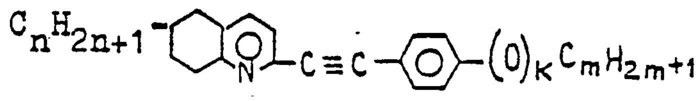

21. Verbindung nach Anspruch 1 der Formel

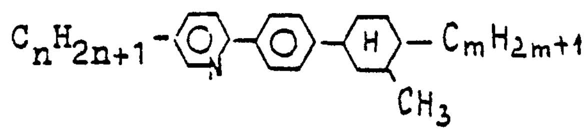

22. Verbindung nach Anspruch 1 der Formel

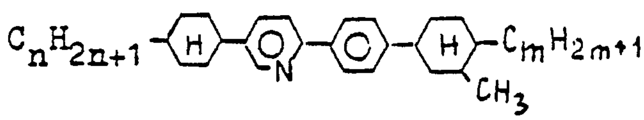

23. Verbindung nach Anspruch 1 der Formel

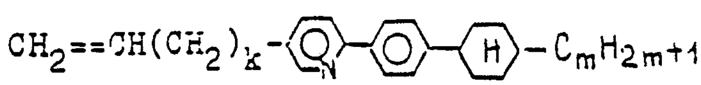

24. Verbindung nach Anspruch 1 der Formel

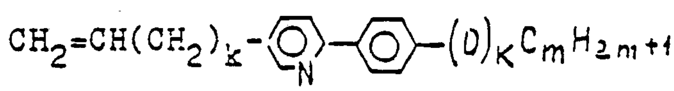

25. Verbindung nach Anspruch 1 der Formel

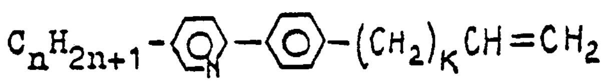

26. Verbindung nach Anspruch 1 der Formel

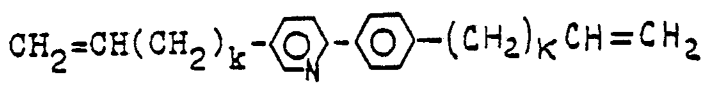

27. Verbindung nach Anspruch 1 der Formel

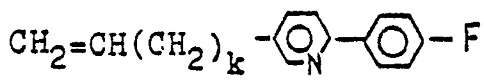

28. Verbindung nach Anspruch 1 der Formel

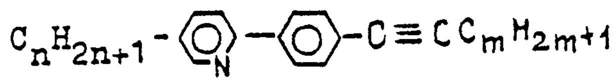

29. Verbindung nach Anspruch 1 der Formel

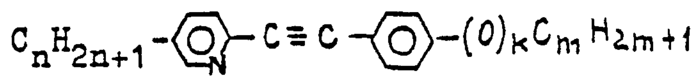

30. Verbindung nach Anspruch 1 der Formel

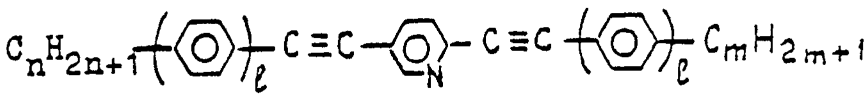

31. Verbindung nach Anspruch 1 der Formel

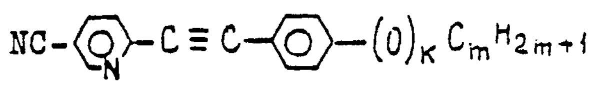

32. Verbindung nach Anspruch 1 der Formel

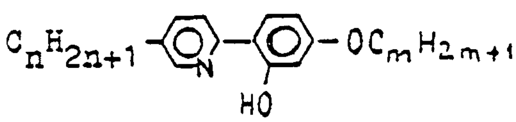

33. Verbindung nach Anspruch 1 der Formel

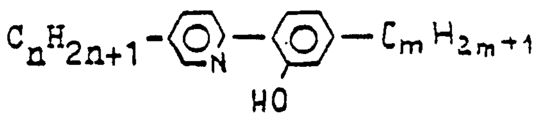

34. Verbindung nach Anspruch 1 der Formel

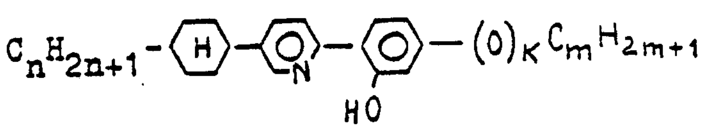

35. Verbindung nach Anspruch 1 der Formel

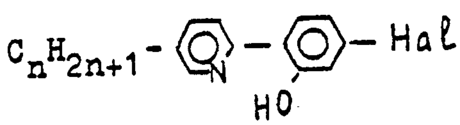

36. Verbindung nach Anspruch 1 der Formel

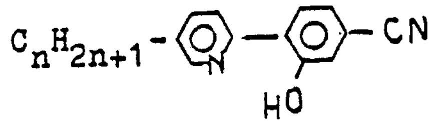

37. Verbindung nach Anspruch 1 der Formel

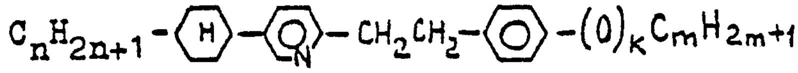

38. Verbindung nach Anspruch 1 der Formel

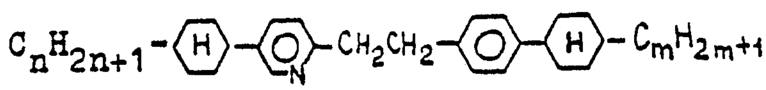

39. Verbindung nach Anspruch 1 der Formel

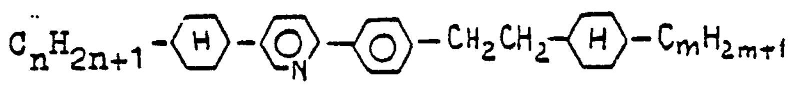

40. Verbindung nach Anspruch 1 der Formel

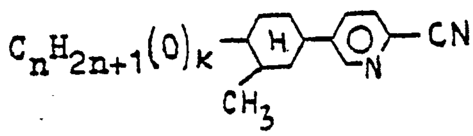

41. Verbindung nach Anspruch 1 der Formel

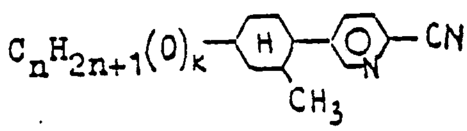

42. Verbindung nach Anspruch 1 der Formel

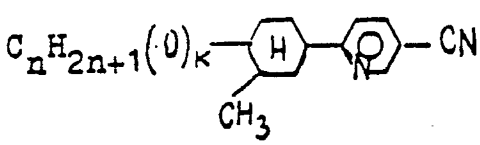

43. Verbindung nach Anspruch 1 der Formel

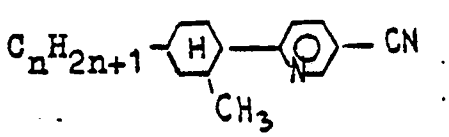

44. Verbindung nach Anspruch 1 der Formel

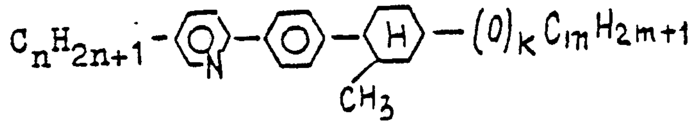

45. Verbindung nach Anspruch 1 der Formel

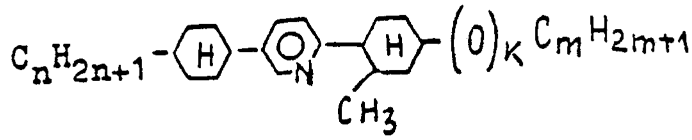

46. Verbindung nach Anspruch 1 der Formel

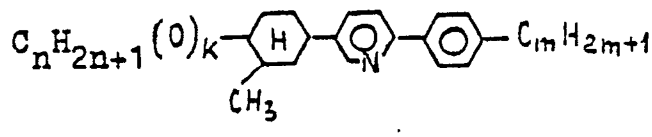

47. Verbindung nach Anspruch 1 der Formel

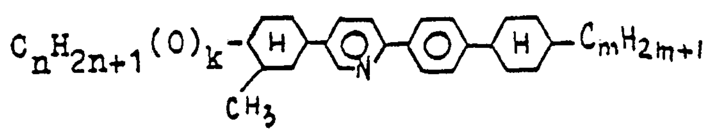

48. Verbindung nach Anspruch 1 der Formel

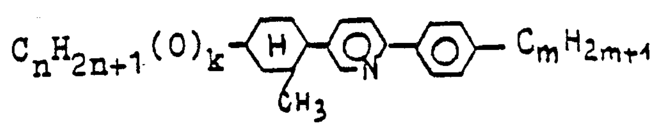

49. Verbindung nach Anspruch 1 der Formel

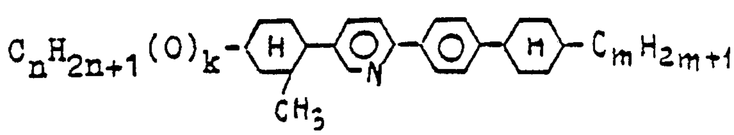

50. Verbindung nach Anspruch 1 der Formel

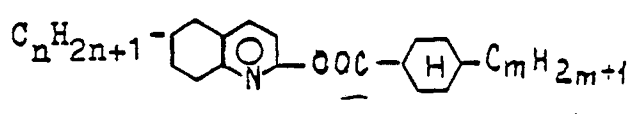

51. Verbindung nach Anspruch 1 der Formel

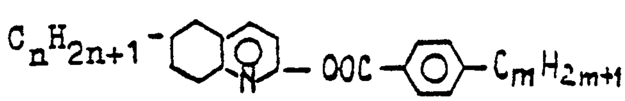

52. Verbindung nach Anspruch 1 der Formel

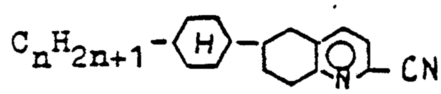

53. Verbindung nach Anspruch 1 der Formel

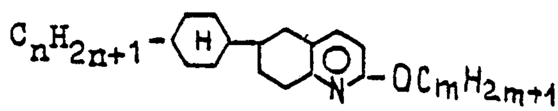

54. Verbindung nach Anspruch 1 der Formel

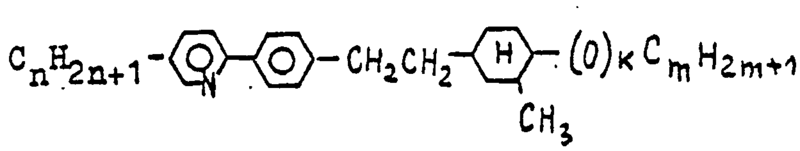

55. Verbindung nach Anspruch 1 der Formel

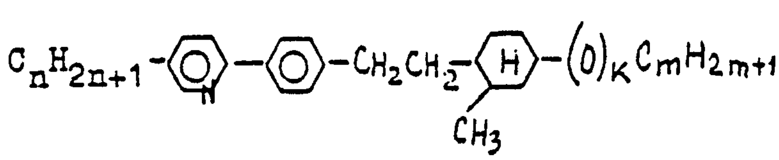

56. Verbindung nach Anspruch 1 der Formel

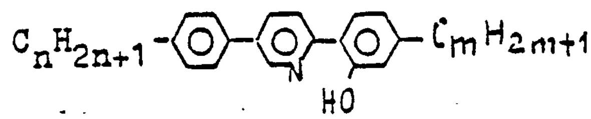

57. Verbindung nach Anspruch 1 der Formel

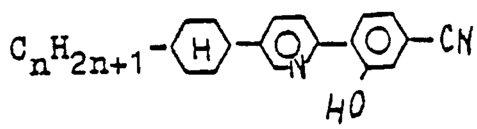

58. Verbindung nach Anspruch 1 der Formel

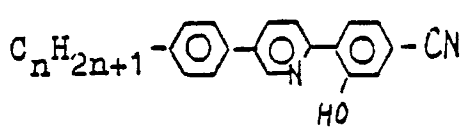

59. Flüssigkristallines Material, welches wenigstens zwei Komponenten enthält, dadurch **gekennzeichnet**, daß wenigstens eine der genannten Komponenten ein flüssigkristallines Derivat des stickstoffhaltigen heterozyklischen Systems nach Anspruch 1 ist.

60. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 2

dient.

61. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 3 dient.

62. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 4 dient.

63. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 5 dient.

64. Flüssigkristallines Material nach Anspruch 59, dadurch **gekennzeichnet**, daß als genanntes flüssigkristallines Systems die Verbindung nach Anspruch 6 dient.

65. Flüssigkristallines Material nach Anspruch 59, dadurch **gekennzeichnet**, daß als genannten flüssigkristallines Derivat des stickstoffhaltigen heterozyklischen Systems die Verbindung nach Anspruch 7 dient.

66. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 8 dient.

67. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 9 dient.

68. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 10 dient.

69. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 11 dient.

70. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 12 dient.

71. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 13 dient.

72. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 14 dient.

73. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 15 dient.

74. Flüssigkristallines Material nach Anspruch 59, dadurch **gekennzeichnet**, daß als genanntes flüssigkristallines Derivat des stickstoffhaltigen he terozyklischen Systems die Verbindung nach Anspruch 16 dient.

75. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 17 dient.

76. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 18 dient.

77. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 19 dient.

78. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 20 dient.

79. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 21 dient.

80. Flüssigkristallines Material nach Anspruch 59, dadurch **gekennzeichnet**, daß als genanntes

flüssigkristallines Derivat des stickstoffhaltigen heterozyklischen Systems die Verbindung nach Anspruch 22 dient.

81. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 23 dient.

82. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 24 dient.

83. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 25 dient.

84. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 26 dient.

85. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 27 dient.

86. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 28 dient.

87. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 29 dient.

88. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 30 dient.

89. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 31 dient.

90. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 32 dient.

91. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 33 dient.

92. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 34 dient.

93. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 35 dient.

94. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 36 dient.

95. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 37 dient.

96. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 38 dient.

97. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 39 dient.

98. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 40 dient.

99. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 41

dient.

100. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 42 dient.

101. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 43 dient.

102. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 44 dient.

103. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 45 dient.

104. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 46 dient.

105. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 47 dient.

106. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 48 dient.

107. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 49 dient.

108. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 50 dient.

109. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 51 dient.

110. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 52 dient.

111. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 53 dient.

112. Flüssigkristallines Material nach Anspruch 59, dadurch **gekennzeichnet**, daß als genanntes flüssigkristallines Derivat des stickstoffhaltigen he terozyklischen Systems die Verbindung nach Anspruch 54 dient.

113. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 55 dient.

114. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 56 dient.

115. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 57 dient.

116. Flüssigkristallines Material nach Anspruch 59, dadurch **gekennzeichnet**, daß als genanntes flüssigkristallines Derivat des stickstoffhaltigen heterozyklischen Systems die Verbindung nach Anspruch 58 dient.

117. Flüssigkristallines Material nach Anspruch 59, dadurch **gekennzeichnet**, daß der Gehalt an dem genannten flüssigkristalline Derivat des stickstoffhaltigen flüssigkristallinen Systems 1 bis 50 Masse% beträgt.